# EUROPÄISCHE PATENTANMELDUNG

(11) **EP 4 260 909 A2**
(43) Veröffentlichungstag der Anmeldung: **18.10.2023**
(21) Anmeldenummer: 23189515.2
(22) Anmeldetag: 25.11.2015
(51) Int. Cl.: A61P 35/00

(54) **SUBSTITUIERTE INDAZOLE, VERFAHREN ZU IHRER HERSTELLUNG, PHARMAZEUTISCHE PRÄPARATE, DIE DIESE ENTHALTEN, SOWIE DEREN VERWENDUNG ZUR HERSTELLUNG VON ARZNEIMITTELN**

(30) Priorität: 26.11.2014 EP 14195032
(62) Teilanmeldung aus: 19191936.4
(71) Anmelder: Bayer Pharma Aktiengesellschaft, 13353 Berlin (DE)
(72) Erfinder: Die Erfindernennung liegt noch nicht vor
(74) Vertreter: BIP Patents

(57) **Zusammenfassung**

Die vorliegende Anmeldung betrifft neue substituierte Indazole, Verfahren zu ihrer Herstellung, ihre Verwendung allein oder in Kombinationen zur Behandlung und/oder Prophylaxe von Krankheiten sowie ihre Verwendung zur Herstellung von Arzneimitteln zur Behandlung und/oder Prophylaxe von Krankheiten, insbesondere zur Behandlung und/oder Prophylaxe von Endometriose sowie Endometriose-assoziierter Schmerzen und anderer Endometriose-assoziierter Symptome wie Dysmenorrhoe, Dyspareunie, Dysurie und Dyschezie, von Lymphomen, Rheumatoider Arthritis, Spondyloarthritiden (insbesondere Spondyloarthritis psoriatica und Morbus Bechterew), Lupus erythematodes, Multipler Sklerosis, Makuladegeneration, COPD, Gicht, Fettlebererkrankungen, Insulinresistenz, Tumorerkrankungen und Psoriasis.

## Beschreibung

Die vorliegende Anmeldung betrifft neue substituierte Indazole, Verfahren zu ihrer Herstellung, Zwischenstufen zur Verwendung bei der Herstellung der neuen Verbindungen, die Verwendung der neuen substituierten Indazole zur Behandlung und/oder Prophylaxe von Krankheiten sowie ihre Verwendung zur Herstellung von Arzneimitteln zur Behandlung und/oder Prophylaxe von Krankheiten, insbesondere von proliferativen Erkrankungen, von Autoimmunerkrankungen, von metabolischen und von inflammatorischen Erkrankungen wie z.B. Rheumatoider Arthritis, Spondyloarthritiden (insbesondere Spondyloarthritis psoriatica und Morbus Bechterew), chronisch obstruktiver Lungenerkrankung (englisch chronic obstructive pulmonary disease, Abkürzung: COPD), Multipler Sklerose, Systemischem Lupus Erythematodes, Gicht, des metabolischen Syndroms, Fettleberhepatitis, Insulinresistenz, Endometriose und entzündungsinduziertem oder chronischem Schmerz sowie von Lymphomen.

Die vorliegende Erfindung betrifft neue substituierte Indazole der allgemeinen Formel (I), die die Interleukin-1 Receptor-Associated Kinase 4 (IRAK4) hemmen.

Humanes IRAK4 (Interleukin-1 receptor-associated kinase 4) spielt eine Schlüsselrolle bei der Aktivierung des Immunsystems. Deshalb ist diese Kinase ein wichtiges therapeutisches Zielmolekül für die Entwicklung von entzündungshemmenden Substanzen. IRAK4 wird von einer Vielzahl von Zellen exprimiert und vermittelt die Signaltransduktion von Toll-like Rezeptoren (TLR), außer TLR3, sowie Rezeptoren der Interleukin (IL)-1β Familie, bestehend aus dem IL-1R (Rezeptor), IL-18R, IL-33R und IL-36R (Janeway und Medzhitov, Annu. Rev. Immunol., 2002; Dinarello, Annu. Rev. Immunol., 2009; Flannery and Bowie, Biochemical Pharmacology, 2010). Weder IRAK4 Knockout Mäuse noch humane Zellen von Patienten, denen IRAK4 fehlt, reagieren auf die Stimulation von TLRs (außer TLR3) und der IL-1β Familie (Suzuki, Suzuki, et al., Nature, 2002; Davidson, Currie, et al., The Journal of Immunology, 2006; Ku, von Bernuth, et al., JEM, 2007; Kim, Staschke, et al., JEM, 2007).

Die Bindung der TLR Liganden bzw. der Liganden der IL-1β Familie an den jeweiligen Rezeptor führt zur Rekrutierung und Bindung von MyD88 [Myeloid differentiation primary response gene (88)] an den Rezeptor. Infolgedessen tritt MyD88 mit IRAK4 in Interaktion und es kommt zur Bildung eines aktiven Komplexes, welcher mit den Kinasen IRAK1 oder IRAK2 interagiert und diese aktiviert (Kollewe, Mackensen, et al., Journal of Biological Chemistry, 2004; Precious et al., J. Biol. Chem., 2009). Infolgedessen wird der NF (nuclear factor)-κB Signalweg und der MAPK (Mitogen-activated protein kinase) Signalweg aktiviert (Wang, Deng, et al., Nature, 2001). Die Aktivierung des NF-κB Signalweges als auch des MAPK Signalweges führen zu Prozessen, die mit verschiedenen Immunprozessen assoziiert sind. So kommt es beispielsweise zu einer erhöhten Expression von unterschiedlichen inflammatorischen Signalmolekülen und Enzymen, wie z.B. Zytokinen, Chemokinen und COX-2 (Cyclooxygenase-2), und zu einer erhöhten mRNA Stabilität von inflammationsassoziierten Genen wie beispielsweise COX-2, IL-6 (Interleukin-6)-, IL-8 (Holtmann, Enninga, et al., Journal of Biological Chemistry, 2001; Datta, Novotny, et al., The Journal of Immunology, 2004). Des Weiteren können diese Prozesse mit der Proliferation und der Differenzierung von bestimmten Zelltypen, wie z.B. Monozyten, Makrophagen, Dendritischen Zellen, T-Zellen und B-Zellen einhergehen (Wan, Chi, et al., Nat Immunol, 2006; McGettrick and J. O'Neill, British Journal of Haematology, 2007).

Die zentrale Rolle von IRAK4 in der Pathologie von unterschiedlichen inflammatorischen Erkrankungen konnte bereits durch den direkten Vergleich von Wildtyp (WT) Mäusen mit genetisch veränderten Tieren mit einer Kinase-inaktiven Form des IRAK4 (IRAK4 KDKI) gezeigt werden. IRAK4 KDKI Tiere weisen ein verbessertes Krankheitsbild im Tiermodell für Multiple Sklerose, Atherosklerose, Herzinfarkt und Alzheimer auf (Rekhter, Staschke, et al., Biochemical and Biophysical Research Communication, 2008; Maekawa, Mizue, et al., Circulation, 2009; Staschke, Dong, et al., The Journal of Immunology, 2009; Kim, Febbraio, et al., The Journal of Immunology, 2011; Cameron, Tse, et al., The Journal of Neuroscience, 2012). Des Weiteren zeigte sich, dass die Deletion von IRAK4 im Tiermodell vor einer viral-induzierten Myokarditis infolge einer verbesserten anti-viralen Reaktion bei gleichzeitig verringerter systemischer Inflammation schützt (Valaperti, Nishii, et al., Circulation, 2013). Außerdem wurde gezeigt, dass die Expression von IRAK4 mit dem Ausmaß des Vogt-Koyanagi-Harada-Syndroms korreliert (Sun, Yang, et al., PLoS ONE, 2014). Zudem konnte die hohe Relevanz von IRAK4 für die Immunkomplexvermittelte IFNα (Interferon-alpha) Produktion durch plasmazytoide Dendritische Zellen, ein Schlüsselprozess bei der Pathogenese des Systemischen Lupus Erythematodes (SLE), gezeigt werden (Chiang et al., The Journal of Immunology, 2010). Des Weiteren ist der Signalweg mit Fettleibigkeit (Adipositas) assoziiert (Ahmad, R., P. Shihab, et al., Diabetology & Metabolic Syndrome, 2015)

Neben der essentiellen Rolle von IRAK4 bei der angeborenen Immunität gibt es auch Hinweise, dass IRAK4 die Differenzierung der sogenannten Th17 T-Zellen, Komponenten der adaptiven Immunität, beeinflusst. In Abwesenheit der IRAK4 Kinaseaktivität werden weniger IL-17 produzierende T-Zellen (Th17 T-Zellen) im Vergleich zu WT Mäusen generiert. Durch die Inhibition von IRAK4 ist die Prophylaxe und/oder Behandlung von Atherosklerose, Diabetes mellitus Typ 1, Rheumatoide Arthritis, Spondyloarthritiden (insbesondere Spondyloarthritis psoriatica und Morbus Bechterew), Lupus erythematodes, Psoriasis, Vitiligo, Riesenzellarteriitis, chronisch entzündlicher Darmerkrankung und Viruserkrankungen, wie z.B. HIV (Humane Immundefizienz-Virus), Hepatitis Virus möglich (Staschke, et al., The Journal of Immunology, 2009; Marquez, et al., Ann Rheum Dis, 2014; Zambrano-Zaragoza, et al., International Journal of Inflammation, 2014; Wang, et al., Experimental and Therapeutic Medicine, 2015; Ciccia, et al., Rheumatology, 2015).

Durch die zentrale Rolle von IRAK4 in der MyD88-vermittelten Signalkaskade von TLRs (außer TLR3) und der IL-1 Rezeptorfamilie kann die Inhibition von IRAK4 zur Prophylaxe und/oder Behandlung von durch die genannten Rezeptoren vermittelte Erkrankungen genutzt werden. TLRs als auch Komponenten der IL-1 Rezeptorfamilie sind in der Pathogenese der Rheumatoiden Arthritis, der Psoriasis Arthritis, Myasthenia gravis, der Vaskulitis wie beispielsweise Morbus Behcet, Granulomatose mit Polyangiitis und Riesenzellarteriitis, Pankreatitis, des Systemischen Lupus Erythematodes, der Dermamyositis und Polymyositis, des Metabolischen Syndroms inklusive z.B. Insulinresistenz, Hypertonie, Dyslipoproteinämie und Adipositas, der Diabetes mellitus (Typ 1 und Typ 2), der diabetischen Nephropathie, der Osteoarthritis, des Sjögren-Syndroms, und der Sepsis involviert (Yang, Tuzun, et al., J Immunol, 2005; Candia, Marquez et al., The Journal of Rheumatology, 2007; Scanzello, Plaas, et al. Curr Opin Rheumatol, 2008; Deng, Ma-Krupa, et al., Circ Res, 2009; Roger, Froidevaux, et al, PNAS, 2009; Devaraj, Tobias, et al., Arterioscler Thromb Vasc Biol, 2011; Kim, Cho, et al., Clin Rheumatol, 2010; Carrasco et al., Clinical and Experimental Rheumatology, 2011; Gambuzza, Licata, et al., Journal of Neuroimmunology, 2011; Fresno, Archives Of Physiology And Biochemistry, 2011; Volin and Koch, J Interferon Cytokine Res, 2011; Akash, Shen, et al., Journal of Pharmaceutical Sciences, 2012; Goh and Midwood, Rheumatology, 2012; Dasu, Ramirez, et al., Clinical Science, 2012; Ouziel, Gustot, et al., Am J Patho, 2012; Ramirez and Dasu, Curr Diabetes Rev, 2012, Okiyama et al., Arthritis Rheum, 2012; Chen et al., Arthritis Research & Therapy, 2013; Holle, Windmoller, et al., Rheumatology (Oxford), 2013; Li, Wang, et al., Pharmacology & Therapeutics, 2013; Sedimbi, Hagglof, et al., Cell Mol Life Sci, 2013; Caso, Costa, et al., Mediators of Inflammation, 2014; Cordiglieri, Marolda, et al., J Autoimmun, 2014; Jialal, Major, et al., J Diabetes Complications, 2014; Kaplan, Yazgan, et al., Scand J Gastroenterol, 2014; Talabot-Aye, et al., Cytokine, 2014; Zong, Dorph, et al., Ann Rheum Di, 2014; Ballak, Stienstra, et al., Cytokine, 2015; Timper, Seelig, et al., J Diabetes Complications, 2015). Hauterkrankungen wie Psoriasis, atopische Dermatitis, Kindler Syndrom, bullösen Pemphigoid, allergische Kontaktdermatitis, Alopecia areata, Acne inversa und Acne vulgaris sind mit dem IRAK4-vermittelten TLR-Signalweg bzw. der IL-1R Familie assoziiert (Schmidt, Mittnacht, et al., J Dermatol Sci, 1996; Hoffmann, J Investig Dermatol Symp Proc, 1999; Gilliet, Conrad, et al., Archives of Dermatology, 2004; Niebuhr, Langnickel, et al., Allergy, 2008; Miller, Adv Dermatol, 2008; Terhorst, Kalali, et al., Am J Clin Dermatol, 2010; Viguier, Guigue, et al., Annals of Internal Medicine, 2010; Cevikbas, Steinhoff, J Invest Dermatol, 2012; Minkis, Aksentijevich, et al., Archives of Dermatology, 2012; Dispenza, Wolpert, et al., J Invest Dermatol, 2012; Minkis, Aksentijevich, et al., Archives of Dermatology, 2012; Gresnigt and van de Veerdonk, Seminars in Immunology, 2013; Selway, Kurczab, et al., BMC Dermatology, 2013; Sedimbi, Hagglof, et al., Cell Mol Life Sci, 2013; Wollina, Koch, et al. Indian Dermatol Online, 2013; Foster, Baliwag, et al., The Journal of Immunology, 2014).

Auch bei pulmonalen Erkrankungen wie Lungenfibrose, obstruktiver Lungenerkrankung (COPD), akutem Atemnot-Syndrom (ARDS), akuter Lungenschädigung (ALI), interstitieller Lungenerkrankung (ILD), Sarkoidose und pulmonaler Hypertonie zeigt sich eine Assoziation mit verschiedenen TLR-vermittelten Signalwegen. Bei der Pathogenese der pulmonalen Erkrankungen kann es sich sowohl um infektiös vermittelte als auch um nicht-infektiös vermittelte Prozesse handeln (Ramirez Cruz, Maldonado Bernal, et al., Rev Alerg Mex, 2004; Jeyaseelan, Chu, et al., Infection and Immunity, 2005; Seki, Tasaka, et al., Inflammation Research, 2010; Xiang, Fan, et al., Mediators of Inflammation, 2010; Margaritopoulos, Antoniou, et al., Fibrogenesis & Tissue Repair, 2010; Hilberath, Carlo, et al., The FASEB Journal, 2011; Nadigel, Prefontaine, et al., Respiratory Research, 2011; Kovach and Standiford, International Immunopharmacology, 2011; Bauer, Shapiro, et al., Mol Med, 2012; Deng, Yang, et al., PLoS One, 2013; Freeman, Martinez, et al., Respiratory Research, 2013; Dubaniewicz, A., Human Immunology, 2013). TLRs als auch IL-1R Familienmitglieder sind auch in die Pathogenese anderer inflammatorischer Erkrankungen wie Allergie, Behcet-Krankheit, Gicht, Lupus erythematodes, Adult Morbus Still-Krankheit, Perikarditis und chronisch entzündliche Darmerkrankungen, wie Kolitis ulcerosa und Morbus Crohn, Transplantatabstoßung und Graft-versus-Host-Reaktion involviert, so dass die Inhibition von IRAK4 hier ein geeigneter prophylaktischer und/oder therapeutischer Ansatz ist (Liu-Bryan, Scott, et al., Arthritis & Rheumatism, 2005; Piggott, Eisenbarth, et al., J Clin Inves, 2005; Christensen, Shupe, et al., Immunity, 2006; Cario, Inflammatory Bowel Diseases, 2010; Nickerson, Christensen, et al., The Journal of Immunology, 2010; Rakoff-Nahoum, Hao, et al., Immunity, 2006; Heimesaat, Fischer, et al., PLoS ONE, 2007; Heimesaat, Nogai, et al., Gut, 2010; Kobori, Yagi, et al., J Gastroenterol, 2010; Schmidt, Raghavan, et al., Nat Immunol, 2010; Shi, Mucsi, et al., Immunological Reviews, 2010; Leventhal and Schroppel, Kidney Int, 2012; Chen, Lin, et al., Arthritis Res Ther, 2013; Hao, Liu, et al., Curr Opin Gastroenterol, 2013; Kreisel and Goldstein, Transplant International, 2013; Li, Wang, et al., Pharmacology & Therapeutics, 2013; Walsh, Carthy, et al., Cytokine & Growth Factor Reviews, 2013; Zhu, Jiang, et al., Autoimmunity, 2013; Yap and Lai, Nephrology, 2013; Vennegaard, Dyring-Andersen, et al., Contact Dermatitis, 2014; D'Elia, Brucato, et al., Clin Exp Rheumatol, 2015; Jain, Thongprayoon, et al., Am J Cardiol., 2015; Li, Zhang, et al., Oncol Rep., 2015).

Durch TLR- und IL-1R Familie-vermittelte gynäkologische Erkrankungen wie Adenomyosis, Dysmenorrhoe, Dyspareunie und Endometriose, insbesondere Endometriose-assoziierte Schmerzen und andere Endometriose-assoziierte Symptome wie Dysmenorrhoe, Dyspareunie, Dysurie und Dyschezie, können durch den prophylaktischen und/oder therapeutischen Einsatz von IRAK4 Inhibitoren positiv beeinflusst werden (Akoum, Lawson, et al., Human Reproduction, 2007; Allhorn, Boing, et al., Reproductive Biology and Endocrinology, 2008; Lawson, Bourcier, et al., Journal of Reproductive Immunology, 2008; Sikora, Mielczarek-Palacz, et al., American Journal of Reproductive Immunology, 2012; Khan, Kitajima, et al., Journal of Obstetrics and Gynaecology Research, 2013; Santulli, Borghese, et al., Human Reproduction, 2013). Der prophylaktische und/oder therapeutische Einsatz von IRAK4 Inhibitoren kann außerdem Atherosklerose positiv beeinflussen (Seneviratne, Sivagurunathan, et al., Clinica Chimica Acta, 2012; Falck-Hansen, Kassiteridi, et al., International Journal of Molecular Sciences, 2013; Sedimbi, Hagglof, et al., Cell Mol Life Sci, 2013).

Neben den bereits aufgeführten Erkrankungen werden IRAK4-vermittelte TLR-Prozesse in der Pathogenese von Augenerkrankungen wie retinale Ischämie, Keratitis, allergisch bedingte Konjunktivitis, Keratoconjunctivitis sicca, Makuladegeneration und Uveitis beschrieben (Kaarniranta and Salminen, J Mol Med (Berl), 2009; Sun and Pearlman, Investigative Ophthalmology & Visual Science, 2009; Redfern and McDermott, Experimental Eye Research, 2010; Kezic, Taylor, et al., J Leukoc Biol, 2011; Chang, McCluskey, et al., Clinical & Experimental Ophthalmology, 2012; Guo, Gao, et al., Immunol Cell Biol, 2012; Lee, Hattori, et al., Investigative Ophthalmology & Visual Science, 2012; Qi, Zhao, et al., Investigative Ophthalmology & Visual Science, 2014).

Die Inhibition von IRAK4 ist außerdem ein geeigneter therapeutischer Ansatz für fibrotische Erkrankungen, wie beispielsweise Leberfibrose, Myokarditis, primär biliäre Zirrhose, zystische Fibrose (Zhao, Zhao, et al., Scand J Gastroenterol, 2011; Benias, Gopal, et al., Clin Res Hepatol Gastroenterol, 2012; Yang, L. and E. Seki, Front Physiol, 2012; Liu, Hu, et al., Biochim Biophys Acta., 2015).

Durch die Schlüsselstellung, die IRAK4 in TLR- und IL-1R Familie-vermittelten Erkrankungen hat, können chronische Lebererkrankungen wie beispielsweise Fettleberhepatitis und insbesondere nichtalkoholischen Fettlebererkrankungen (NAFLD - non-alcoholic fatty liver disease) und/oder nichtalkoholischer Fettleberhepatitis (NASH - non-alcoholic steatohepatitis), alkoholtoxische Hepatitis (ASH - alkoholische Steatohepatitis) präventiv und/oder therapeutisch mit IRAK4 Inhibitoren behandelt werden (Nozaki, Saibara, et al., Alcohol Clin Exp Res, 2004; Csak, T., A. Velayudham, et al., Am J Physiol Gastrointest Liver Physiol, 2011; Miura, Kodama, et al., Gastroenterology, 2010; Kamari, Shaish, et al., J Hepatol, 2011; Ye, Li, et al., Gut, 2012; Roh, Seki, J Gastroenterol Hepatol, 2013; Ceccarelli, S., V. Nobili, et al., World J Gastroenterol, 2014; Miura, Ohnishi, World J Gastroenterol, 2014; Stojsavljevic, Palcic, et al., World J Gastroenterol, 2014).

Aufgrund der zentralen Rolle von IRAK4 in TLR-vermittelten Prozessen ist durch die Inhibition von IRAK4 auch die Behandlung /und/oder Prävention von kardiovaskulären und neurologischen Erkrankungen wie z.B. myokardialem Reperfusionsschaden, Myokardinfarkt, Hypertonie, Bluthochdruck (Oyama, Blais, et al., Circulation, 2004; Timmers, Sluijter, et al., Circulation Research, 2008; Fang and Hu, Med Sci Monit, 2011; Bijani, International Reviews of Immunology, 2012; Bomfim, Dos Santos, et al., Clin Sci (Lond), 2012; Christia and Frangogiannis, European Journal of Clinical Investigation, 2013; Thompson and Webb, Clin Sci (Lond), 2013; Hernanz, Martínez-Revelles, et al., British Journal of Pharmacology, 2015; Frangogiannis, Curr Opin Cardiol, 2015; Bomfim, Echem, et al., Life Sciences, 2015) sowie Alzheimer, Schlaganfall, Hirnschlag, Schädel-Hirn-Trauma, Amyotrophe Lateralsklerose (ALS) und Parkinson möglich (Brough, Tyrrell, et al., Trends in Pharmacological Sciences, 2011; Carty and Bowie, Biochemical Pharmacology, 2011; Denes, Kitazawa, Cheng, et al., The Journal of Immunology, 2011; Lim, Kou, et al., The American Journal of Pathology, 2011; Béraud and Maguire-Zeiss, Parkinsonism & Related Disorders, 2012; Denes, Wilkinson, et al., Disease Models & Mechanisms, 2013; Noelker, Morel, et al., Sci. Rep., 2013; Wang, Wang, et al., Stroke, 2013; Xiang, Chao, et al., Rev Neurosci, 2015; Lee, Lee, et al., J Neuroinflammation, 2015).

Aufgrund der Involvierung von TLR-vermittelten Signalen und IL-1 Rezeptorfamilie-vermittelten Signalen über IRAK4 bei Juckreiz und Schmerz inklusive akutem, chronischem, entzündlichem und neuropathischem Schmerz ist von einer therapeutischen Wirkung in den genannten Indikationen durch die Inhibierung von IRAK4 auszugehen. Für Schmerz seien beispielhaft Hyperalgesie, Allodynie, prämenstrueller Schmerz, Endometriose-assoziierter Schmerz, postoperativer Schmerz, interstitielle Zystitis, CRPS (komplexes regionales Schmerzsyndrom), Trigeminusneuralgie, Prostatitis, Schmerz verursacht durch Rückenmarksverletzungen, entzündungsinduzierter Schmerz, Kreuzschmerzen, Krebsschmerzen, Chemotherapie-assoziierter Schmerz, HIV behandlungs-induzierte Neuropathie, Verbrennungs-induzierter Schmerz und chronischer Schmerz zu nennen (Wolf, Livshits, et al., Brain, Behavior, and Immunity, 2008; Kim, Lee, et al., Toll-like Receptors: Roles in Infection and Neuropathology, 2009; del Rey, Apkarian, et al., Annals of the New York Academy of Sciences, 2012; Guerrero, Cunha, et al., European Journal of Pharmacology, 2012; Kwok, Hutchinson, et al., PLoS ONE, 2012; Nicotra, Loram, et al., Experimental Neurology, 2012; Chopra and Cooper, J Neuroimmune Pharmacol, 2013; David, Ratnayake, et al., Neurobiology of Disease, 2013; Han, Zhao, et al., Neuroscience, 2013; Liu and Ji, Pflugers Arch., 2013; Stokes, Cheung, et al., Journal of Neuroinflammation, 2013; Zhao, Zhang, et al., Neuroscience, 2013; Liu, Zhang, et al., Cell Research, 2014; Park, Stokes, et al., Cancer Chemother Pharmacol, 2014; Van der Watt, Wilkinson, et al., BMC Infect Dis, 2014; Won, K. A., M. J. Kim, et al., J Pain, 2014; Min, Ahmad, et al., Photochem Photobiol., 2015; Schrepf, Bradley, et al., Brain Behav Immun, 2015; Wong, L., J. D. Done, et al., Prostate, 2015).

Dies gilt auch für einige onkologische Erkrankungen. Bestimmte Lymphome, wie beispielsweise ABC-DLBCL (Aktivierte B Zellen-Diffuses großzelliges B-Zell-Lymphom), Mantelzelllymphon und Morbus Waldenström als auch chronisch lymphatische Leukämie, Melanoma, Pankreastumor und Leberzellkarzinom sind durch Mutationen in MyD88 oder Veränderungen in der MyD88-Aktivität charakterisiert, die durch einen IRAK4 Inhibitor behandelt werden können (Ngo, Young, et al., Nature, 2011; Puente, Pinyol, et al., Nature, 2011; Ochi, Nguyen, et al., J Exp Med, 2012; Srivastava, Geng, et al., Cancer Research, 2012; Treon, Xu, et al., New England Journal of Medicine, 2012; Choi, Kim, et al., Human Pathology, 2013; (Liang, Chen, et al., Clinical Cancer Research, 2013). Des Weiteren spielt MyD88 eine wichtige Rolle in Ras-abhängigen Tumoren, so dass IRAK4 Inhibitoren auch zu deren Behandlung geeignet sind (Kfoury, A., K. L. Corf, et al., Journal of the National Cancer Institute, 2013). Es ist außerdem von einer therapeutischen Wirkung bei Brustkrebs, Ovarialkarzinom, Kolorektales Karzinom, Kopf-Hals-Karzinom, Lungenkrebs, Prostatakrebs durch die Inhibierung von IRAK4 auszugehen, da die genannten Indikationen mit dem Signalweg assoziiert sind (Szczepanski, Czystowska, et al., Cancer Res, 2009; Zhang, He, et al., Mol Biol Rep, 2009; Wang, Qian, et al., Br J Cancer Kim, 2010; Jo, et al., World J Surg Oncol, 2012; Zhao, Zhang, et al.; Front Immunol, 2014; Chen, Zhao, et al., Int J Clin Exp Pathol, 2015). Inflammatorische Erkrankungen wie CAPS (Cryopyrin-assoziierte periodische Syndrome), inklusive FCAS (familiäre Kälteurtikaria), MWS (Muckle-Wells-Syndrom), NOMID- (neonatal-onset multisystem inflammatory disease) und CONCA- (chronic infantile, neurological, cutaneous, and articular) Syndrom; FMF (familiäres Mittelmeerfieber), HIDS (Hyper-IgD-Syndrom), TRAPS (Tumornekrosefaktor-Rezeptor 1-assoziiertes periodisches Syndrom), juvenile idiopathische Arthritis, Adult Morbus Still-Krankheit, Morbus Adamantiades-Beh 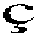 et, rheumatoide Arthritis, Osteoarthritis, Keratoconjunctivitis sicca, PAPA-Syndrom (Pyogene Arthritis, Pyoderma gangraenosum und Akne), Schnitzler Syndrom und Sjögren Syndrom werden durch die Blockierung des IL-1 Signalweges behandelt, so dass auch hier ein IRAK4 Inhibitor zur Behandlung der genannten Krankheiten geeignet ist (Narayanan, Corrales, et al., Cornea, 2008; Brenner, Ruzicka, et al., British Journal of Dermatology, 2009; Henderson and Goldbach-Mansky, Clinical Immunology, 2010; Dinarello, European Journal of Immunology, 2011; Gul, Tugal-Tutkun, et al., Ann Rheum Dis, 2012; Pettersson, Annals of MedicinePetterson, 2012; Ruperto, Brunner, et al., New England Journal of Medicine, 2012; Nordström, Knight, et al., The Journal of Rheumatology, 2012; Vijmasi, Chen, et al., Mol Vis, 2013; Yamada, Arakaki, et al., Opinion on Therapeutic Targets, 2013; de Koning, Clin Transl Allergy, 2014). Der Ligand des IL-33R, IL-33, ist insbesondere in der Pathogenese von akutem Nierenversagen involviert, so dass die Inhibition von IRAK4 zur Prophylaxe und/oder Behandlung ein geeigneter Therapieansatz ist (Akcay, Nguyen, et al., Journal of the American Society of Nephrology, 2011). Komponenten der IL-1 Rezeptorfamilie sind mit Myokardinfarkt, unterschiedlichen pulmonalen Erkrankungen wie Asthma, COPD, idiopathischer interstitieller Pneumonie, allergische Rhinitis, Lungenfibrose und akutem Atemnot-Syndrom (ARDS) assoziiert, so dass eine prophylaktische und/oder therapeutische Wirkung in den genannten Indikationen durch die Inhibierung von IRAK4 zu erwarten ist (Kang, Homer, et al., The Journal of Immunology, 2007; Imaoka, Hoshino, et al., European Respiratory Journal, 2008; Couillin, Vasseur, et al., The Journal of Immunology, 2009; Abbate, Kontos, et al., The American Journal of Cardiology, 2010; Lloyd, Current Opinion in Immunology, 2010; Pauwels, Bracke, et al., European Respiratory Journal, 2011; Haenuki, Matsushita, et al., Journal of Allergy and Clinical Immunology, 2012; Yin, Li, et al., Clinical & Experimental Immunology, 2012; Abbate, Van Tassell, et al., The American Journal of Cardiology, 2013; Alexander-Brett, et al., The Journal of Clinical Investigation, 2013; Bunting, Shadie, et al., BioMed Research International, 2013; Byers, Alexander-Brett, et al., The Journal of Clinical Investigation, 2013; Kawayama, Okamoto, et al., J Interferon Cytokine Res, 2013; Martinez-González, Roca, et al., American Journal of Respiratory Cell and Molecular Biology, 2013; Nakanishi, Yamaguchi, et al., PLoS ONE, 2013; Qiu, Li, et al., Immunology, 2013; Li, Guabiraba, et al., Journal of Allergy and Clinical Immunology, 2014; Saluja, Ketelaar, et al., Molecular Immunology, 2014; Lugrin, Parapanov, et al., The Journal of Immunology, 2015).

Aus dem Stand der Technik sind eine Vielzahl von IRAK4 Inhibitoren bekannt (siehe beispielsweise Annual Reports in Medicinal Chemistry (2014), 49, 117 - 133).

US8293923 und US20130274241 offenbaren IRAK4 Inhibitoren mit einer an Position 3 substituierten Indazolstruktur. 2-substituierte Indazole werden nicht beschrieben.

In WO2013106254 und WO2011153588 werden 2,3-disubstituierte Indazolderivate offenbart.

In WO2007091107 werden 2-substituierte Indazolderivate für die Behandlung der Duchenne-Muskeldystrophie beschrieben. Die offenbarten Verbindungen weisen keine 6-Hydroxyalkyl-Substitution auf.

WO2015091426 beschreibt Indazole wie Beispiel 64, die an der Position 2 mit einer Carboxamidseitenkette substituiert sind.

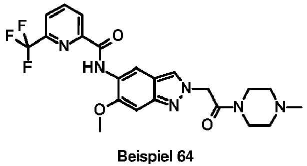

In WO2015104662 werden 2-substituierte Indazole der folgenden allgemeinen Formel offenbart: 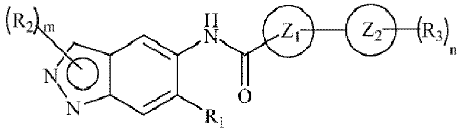 in denen R₂ eine Alkyl- oder Cycloalkylgruppe ist. Explizit beschrieben werden 2-substituierte Indazole mit einer Methyl, 2-Methoxyethyl und Cyclopentylgruppe an der 2-Position (Beispiele 1, 4 und 76). Außerdem wird mit Beispiel 117 ein Indazolderivat mit einem Hydroxyethyl-Subsituenten an der 1-Position beschrieben. Es werden jedoch keine Indazolderivate mit einem 3-Hydroxy-3-methylbutyl-Substituenten an der 1-Position oder 2-Position beschrieben.

Indazole, die an Position 2 eine Hydroxy-substituierte Alkylgruppe aufweisen, sind zwar generisch mit der allgemeinen Formel umfasst, werden aber in WO2015104662 nicht explizit offenbart. Indazole mit einer Alkylgruppe an Position 2, wobei die Alkylgruppe zusätzlich mit einer Methylsulfonylgruppe substituiert ist, sind mit der allgemeinen Formel und den Definitionen für den Substituenten R₂ in WO2015104662 nicht umfasst.

Zusätzlich zum oben beschriebenen Substitutionsmuster am Indazol in 1- und 2-Position, werden in der WO2015104662 Indazole mit einer Substitution an Position 6 beschrieben, für die R₁ die folgende Bedeutung hat: Alkyl, Cyano, -NRₐR_{b} oder optional substituierte Gruppen ausgewählt aus Cycloalkyl, Aryl oder Heterocyclyl, wobei die Substituenten unabhängig voneinander Alkyl, Alkoxy, Halogen, Hydroxyl, Hydroxyalkyl, Amino, Aminoalkyl, Nitro, Cyano, Haloalkyl, Haloalkoxy, -OCOCH₂-O-Alkyl, -OP(O)(O-Alkyl)₂ oder -CH₂-OP(O)(O-Alkyl)₂ sind. Für Indazolverbindungen, in denen R₁ eine Alkylgruppe ist, ist das effektive Anmeldedatum der 7. Januar 2015 (internationales Anmeldedatum der WO2015104662). Die für die Priorität in Anspruch genommenen indischen Anmeldungen 146/CHE/2014 und 3018/CHE/2014 offenbaren keine Indazolverbindungen, für die R₁ eine Alkylgruppe ist.

Damit sind Indazolverbindungen der folgenden allgemeinen Formel 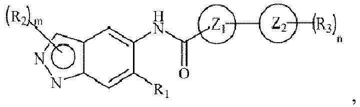 in denen R₁ eine gegebenenfalls substituierte Alkylgruppe ist, das erste Mal am 7. Januar 2015 und damit nach dem Prioritätstag der vorliegenden Anmeldung beschrieben.

Als Beispiele für Substituenten an der Position 6 werden in WO2015104662 für R₁ Cyclopropyl, Cyclohexyl, Cyano, 3-Fluorphenyl und gesättigte heterocyclische Substituenten beschrieben. Indazole mit einer Hydroxy-substituierten Alkylgruppe an Position 6 werden in WO2015104662 nicht explizit beschrieben.

Die Aufgabe der vorliegenden Erfindung besteht darin, neue Verbindungen zur Verfügung zu stellen, die als Inhibitoren der Interleukin-1 Receptor Associated Kinase-4 (IRAK4) wirken.

Die neuen IRAK4 Inhibitoren sind insbesondere zur Behandlung und zur Prävention von proliferativen, metabolischen und entzündlichen Erkrankungen geeignet, die durch ein überreagierendes Immunsystem charakterisiert sind. Besonders genannt seien hier entzündliche Hauterkrankungen, Herz-Kreislauf-Erkrankungen, Lungenerkrankungen, Augenerkrankungen, neurologische Erkrankungen, Schmerzerkrankungen und Krebserkrankungen.

Des Weiteren sind die neuen IRAK4 Inhibitoren geeignet zur Behandlung und Prävention
- von Autoimmun- und inflammatorischen Erkrankungen, insbesondere Rheumatoide Arthritis, Multiple Sklerose, Systemischer Lupus Erythematodes, Spondyloarthritiden und Gicht,
- von Stoffwechselerkrankungen, insbesondere Lebererkrankungen wie Fettleber sowie
- von gynäkologischen Erkrankungen, insbesondere von Endometriose sowie von Endometriose-assoziierten Schmerzen und anderen Endometriose-assoziierten Symptomen wie Dysmenorrhoe, Dyspareunie, Dysurie und Dyschezie.

Gegenstand der vorliegenden Erfindung sind Verbindungen der allgemeinen Formel (I) 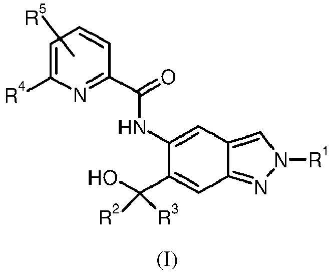 in der:
- R¹: für C₁-C₆-Alkyl steht, wobei der C₁-C₆-Alkylrest unsubstituiert oder ein- oder mehrfach, gleich oder verschieden substituiert ist mit
Halogen, Hydroxy, einem unsubstituierten oder ein- oder mehrfach mit Halogen substituierten C₃-C₆-Cycloalkyl, einem Rest R⁶, R⁷SO₂, R⁷SO oder R⁸O o
der für eine Gruppe steht, ausgewählt aus: 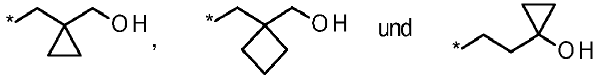 wobei * für die Verknüpfungsstelle der Gruppe mit dem Rest des Moleküls steht;
- R² und R³: immer dieselbe Bedeutung haben und gleichzeitig entweder für Wasserstoff oder C₁-C₆-Alkyl stehen;
- R⁴: für Halogen, Cyano, ein unsubstituiertes oder ein- oder mehrfach, gleich oder verschieden voneinander substituiertes C₁-C₆-Alkyl oder ein unsubstituiertes oder ein- oder mehrfach, gleich oder verschieden voneinander substituiertes C₃-C₆-Cycloalkyl steht, und die Substituenten ausgewählt sind aus der Gruppe Halogen und Hydroxy;
- R⁵: für Wasserstoff, Halogen oder ein unsubstituiertes oder ein- oder mehrfach mit Halogen substituiertes C₁-C₆-Alkyl steht;
- R⁶: für einen unsubstituierten oder ein- oder zweifach mit Methyl substituierten monocyclischen, gesättigten Heterocyclus mit 4 bis 6 Ringatomen steht, der ein Heteroatom oder eine Heterogruppe aus der Reihe O, S, SO und SO₂ enthält;
- R⁷: für C₁-C₆-Alkyl steht, wobei der C₁-C₆-Alkylrest unsubstituiert oder ein- oder mehrfach, gleich oder verschieden mit Halogen, Hydroxy oder C₃-C₆-Cycloalkyl substituiert ist, oder R⁷ steht für C₃-C₆-Cycloalkyl,
- R⁸: für C₁-C₆-Alkyl steht, wobei der C₁-C₆-Alkylrest unsubstituiert oder ein- oder mehrfach, gleich oder verschieden mit Halogen, substituiert ist;
und ihre Diastereomere, Enantiomere, ihre Metabolite, ihre Salze, ihre Solvate oder die Solvate ihrer Salze.

Wenn bei den im Folgenden beschriebenen Synthese-Intermediaten und Ausführungsbeispielen der Erfindung eine Verbindung in der Form eines Salzes der korrespondierenden Base bzw. Säure aufgeführt ist, so ist die exakte stöchiometrische Zusammensetzung eines solchen Salzes, wie es nach dem jeweiligen Herstell- und/oder Reinigungsverfahren erhalten wurde, in der Regel nicht bekannt. Sofern nicht genauer spezifiziert, sind daher Namens- und Strukturformel-Zusätze wie beispielsweise "Hydrochlorid", "Trifluoracetat", "Natrium-Salz" bzw. "x HCl", "x CF₃COOH", "x Na⁺" bei solchen Salzen nicht stöchiometrisch zu verstehen, sondern haben allein deskriptiven Charakter bezüglich der enthaltenen salzbildenden Komponenten.

Sinngemäß gleiches gilt für den Fall, dass Synthese-Intermediate oder Ausführungsbeispiele oder Salze hiervon nach den beschriebenen Herstell- und/oder Reinigungsverfahren in Form von Solvaten, wie beispielsweise Hydraten, erhalten wurden, deren stöchiometrische Zusammensetzung (sofern definierter Art) nicht bekannt ist.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Als Salze sind im Rahmen der vorliegenden Erfindung physiologisch unbedenkliche Salze der erfindungsgemäßen Verbindungen bevorzugt. Umfasst sind aber auch Salze, die für pharmazeutische Anwendungen selbst nicht geeignet sind, aber beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Die erfindungsgemäßen Verbindungen können in Abhängigkeit von ihrer Struktur in unterschiedlichen stereoisomeren Formen existieren, d.h. in Gestalt von Konfigurationsisomeren oder gegebenenfalls auch als Konformationsisomere (Enantiomere und/oder Diastereomere, einschließlich solcher bei Atropisomeren). Die vorliegende Erfindung umfasst deshalb die Enantiomere und Diastereomere und ihre jeweiligen Mischungen. Aus solchen Mischungen von Enantiomeren und/oder Diastereomeren lassen sich die stereoisomer einheitlichen Bestandteile in bekannter Weise isolieren; vorzugsweise werden hierfür chromatographische Verfahren verwendet, insbesondere die HPLC-Chromatographie an achiraler bzw. chiraler Phase.

Sofern die erfindungsgemäßen Verbindungen in tautomeren Formen vorkommen können, umfasst die vorliegende Erfindung sämtliche tautomere Formen.

Die vorliegende Erfindung umfasst auch alle geeigneten isotopischen Varianten der erfindungsgemäßen Verbindungen. Unter einer isotopischen Variante einer erfindungsgemäßen Verbindung wird hierbei eine Verbindung verstanden, in welcher mindestens ein Atom innerhalb der erfindungsgemäßen Verbindung gegen ein anderes Atom der gleichen Ordnungszahl, jedoch mit einer anderen Atommasse als der gewöhnlich oder überwiegend in der Natur vorkommenden Atommasse ausgetauscht ist. Beispiele für Isotope, die in eine erfindungsgemäße Verbindung inkorporiert werden können, sind solche von Wasserstoff, Kohlenstoff, Stickstoff, Sauerstoff, Phosphor, Schwefel, Fluor, Chlor, Brom und Iod, wie 2H (Deuterium), 3H (Tritium), 13C, 14C, 15N, 170, 180, 32P, 33P, 33S, 34S, 35S, 36S, 18F, 36Cl, 82Br, 123I, 124I, 129I und 131I. Bestimmte isotopische Varianten einer erfindungsgemäßen Verbindung, wie insbesondere solche, bei denen ein oder mehrere radioaktive Isotope inkorporiert sind, können von Nutzen sein beispielsweise für die Untersuchung des Wirkmechanismus oder der Wirkstoffverteilung im Körper; aufgrund der vergleichsweise leichten Herstell- und Detektierbarkeit sind hierfür insbesondere mit 3H- oder 14C-Isotopen markierte Verbindungen geeignet. Darüber hinaus kann der Einbau von Isotopen, wie beispielsweise von Deuterium, zu bestimmten therapeutischen Vorteilen als Folge einer größeren metabolischen Stabilität der Verbindung führen, wie beispielsweise eine Verlängerung der Halbwertszeit im Körper oder eine Reduktion der erforderlichen Wirkdosis; solche Modifikationen der erfindungsgemäßen Verbindungen können daher gegebenenfalls auch eine bevorzugte Ausführungsform der vorliegenden Erfindung darstellen. Isotopische Varianten der erfindungsgemäßen Verbindungen können nach den dem Fachmann bekannten Verfahren hergestellt werden, so beispielsweise nach den weiter unten beschriebenen Methoden und den für die Ausführungsbeispiele angegebenen Vorschriften, indem entsprechende isotopische Modifikationen der jeweiligen Reagenzien und/oder Ausgangsverbindungen eingesetzt werden.

Ein weiterer Gegenstand der vorliegenden Erfindung sind alle möglichen kristallinen und polymorphen Formen der erfindungsgemäßen Verbindungen, wobei die Polymorphe entweder als einzelne Polymorphe oder als Gemisch mehrerer Polymorphe in allen Konzentrationsbereichen vorliegen können.

Außerdem umfasst die vorliegende Erfindung auch Prodrugs der erfindungsgemäßen Verbindungen. Der Begriff "Prodrugs" bezeichnet hierbei Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Rahmen der vorliegenden Erfindung haben die Substituenten, soweit nicht anders spezifiziert, die folgenden Bedeutungen:
Alkyl steht im Rahmen der Erfindung für einen linearen oder verzweigten Alkylrest mit der jeweils angegebenen Anzahl an Kohlenstoffatomen. Beispielhaft seien Methyl, Ethyl, n-Propyl, Isopropyl, n-Butyl, iso-Butyl, 1-Methylpropyl, 2-Methylpropyl, tert.-Butyl, n-Pentyl, 1-Ethylpropyl, 1-Methylbutyl, 2-Methylbutyl, 3-Methylbutyl, 2,2-Dimethylpropyl, n-Hexyl, 1-Methylpentyl, 2-Methylpentyl, 3-Methylpentyl, 4-Methylpentyl, 1-Ethylbutyl und 2-Ethylbutyl genannt. Bevorzugt sind Methyl, Ethyl, n-Propyl, n-Butyl, 2-Methylbutyl, 3-Methylbutyl und 2,2-Dimethylpropyl.

Cycloalkyl steht im Rahmen der Erfindung für einen monocyclischen, gesättigten Alkylrest mit der jeweils angegebenen Anzahl an Kohlenstoffatomen. Beispielhaft und vorzugsweise seien Cyclopropyl, Cyclobutyl, Cyclopentyl und Cyclohexyl genannt.

Alkoxy steht im Rahmen der Erfindung für einen linearen oder verzweigten Alkoxyrest mit der jeweils angegebenen Anzahl an Kohlenstoffatomen. 1 bis 6 Kohlenstoffatome sind bevorzugt. Beispielhaft seien Methoxy, Ethoxy, n-Propoxy, Isopropoxy, 1-Methylpropoxy, n-Butoxy, iso-Butoxy, tert.-Butoxy, n-Pentoxy, iso-Pentoxy, 1-Ethylpropoxy, 1-Methylbutoxy, 2-Methylbutoxy, 3-Methylbutoxy und n-Hexoxy genannt. Besonders bevorzugt ist ein linearer oder verzweigter Alkoxyrest mit 1 bis 4 Kohlenstoffatomen. Beispielhaft und vorzugsweise seien Methoxy, Ethoxy, n-Propoxy, 1-Methylpropoxy, n-Butoxy und iso-Butoxy genannt.

Halogen steht im Rahmen der Erfindung für Fluor, Chlor und Brom. Bevorzugt ist Fluor.

Hydroxy steht im Rahmen der Erfindung für OH.

Ein monocyclischer, gesättigter Heterocyclus steht für einen monocyclischen, gesättigten Heterocyclus mit 4 bis 6 Ringatomen, der ein Heteroatom oder eine Heterogruppen aus der Reihe O, S, SO und SO₂ enthält. Ein Heterocylcus mit einem Heteroatom oder einer Heterogruppe aus der Reihe O, SO und SO₂ ist bevorzugt. Beispielsweise seien genannt: Oxetan, Tetrahydrofuran, Tetrahydro-2H-pyran-4-yl, 1,1-Dioxidotetrahydro-2H-thiopyran-3-yl, 1,1-Dioxidotetrahydro-2H-thiopyran-2-yl, 1,1-Dioxidotetrahydro-2H-thiopyran-4-yl, 1,1-Dioxidotetrahydrothiophen-3-yl, 1,1-Dioxidotetrahydrothiophen-2-yl, 1,1-Dioxidothietan-2-yl oder 1,1-Dioxidothietan-3-yl. Besonders bevorzugt sind hierbei Oxetan und Tetrahydrofuran. Ganz besonders bevorzugt ist Oxetan-3-yl.

Ein Symbol * an einer Bindung bedeutet die Verknüpfungsstelle im Molekül.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt.

Eine bevorzugte Ausführungsform von R¹ ist ein mit 1, 2 oder 3 Fluoratomen substituierter C₂-C₆-Alkylrest. Besonders bevorzugt sind 2,2,2-Trifluorethyl, 3,3,3-Trifluorpropyl und 4,4,4-Trifluorbutyl. Ganz besonders bevorzugt ist ein 4,4,4-Trifluorbutylrest.

Eine weitere bevorzugte Ausführungsform von R¹ ist ein C₂-C₆-Alkylrest, der mit einer oder zwei Hydroxygruppe(n) oder einem C₁-C₃-Alkoxy oder einem dreifach mit Fluor substituierten C₁-C₃-Alkoxy substituiert ist. Besonders bevorzugt ist ein C₂-C₅-Alkylrest, der mit Hydroxy oder C₁-C₃-Alkoxy oder mit Trifluormethoxy oder 2,2,2-Trifluorethoxy substituiert ist. Ganz besonders bevorzugt sind 3-Hydroxy-3-methylbutyl, 3-Methoxypropyl, 3-Hydroxypropyl, 3-Trifluormethoxypropyl, 2-Methoxyethyl oder 2-Hydroxyethyl. Insbesondere ist der 3-Hydroxy-3-methylbutylrest bevorzugt.

Weiterhin bevorzugt steht R¹ für einen mit einer C₁-C₆-Alkyl-SO₂-Gruppe substituierten C₂-C₆-Alkylrest. Ein mit Methyl-SO₂ substituierter C₂-C₄-Alkylrest ist besonders bevorzugt. Insbesondere sind für R¹ 2-(Methylsulfonyl)ethyl oder 3-(Methylsulfonyl)propyl bevorzugt. Aus der letztgenannten Gruppe ist 2-(Methylsulfonyl)ethyl besonders bevorzugt.

Weiterhin bevorzugt steht R¹ für ein mit Oxetanyl, Tetrahydrofuranyl, Tetrahydro-2H-pyran-4-yl, 1,1-Dioxidotetrahydro-2H-thiopyran-3-yl, 1,1-Dioxidotetrahydro-2H-thiopyran-2-yl, 1,1-Dioxido-tetrahydro-2H-thiopyran-4-yl, 1,1 -Dioxidotetrahydrothiophen-3-yl, 1,1-Dioxidotetrahydrothiophen-2-yl, 1,1-Dioxidothietan-2-yl oder 1,1-Dioxidothietan-3-yl substituierter C₁-C₃ Alkylrest. Besonders bevorzugt ist ein mit einer Oxetangruppe subsituierter C₁-C₃ Alkylrest. Insbesondere ist eine Oxetan-3-ylmethylgruppe für R¹ bevorzugt.

Für R² und R³, die immer dieselbe Bedeutung haben, sind Wasserstoff oder Methyl bevorzugt. Dabei ist Methyl besonders bevorzugt.

Im Fall von R⁴ ist ein unsubstituierter oder ein ein- oder mehrfach mit Halogen substituierter Ci-C₃-Alkylrest oder ein mit einer Hydroxygruppe substituierter C1-C₃-Alkylrest oder ein mit einer Hydroxygruppe und drei Fluoratomen substituierter C₁-C₃-Alkylrest bevorzugt.

Besonders bevorzugt für R⁴ sind folgende Reste: Methyl, Ethyl, Trifluor-C₁-C₃-alkyl, Difluor-C₁-C₃-Alkyl, Hydroxymethyl, 1-Hydroxyethyl, 2-Hydroxypropan-2-yl und 2,2,2-Trifluor-1-hydroxyethyl. Besonders bevorzugt für R⁴ sind die Reste Methyl, Trifluormethyl und Difluormethyl. Hierbei ist ein Trifluormethylrest besonders bevorzugt.

Eine bevorzugte Ausführungsform von R⁵ ist Wasserstoff, Fluor, Chlor oder C₁-C₃-Alkyl. Besonders bevorzugt ist R⁵ Wasserstoff, Fluor oder Methyl. Ganz besonders bevorzugt ist R⁵ Wasserstoff oder Fluor.

Besonders bevorzugt sind außerdem Verbindungen, in denen R⁴ Methyl oder Trifluormethyl bedeutet und R⁵ Fluor. Ganz besonders bevorzugt sind Verbindungen bei denen R⁴ für Methyl steht und R⁵ für Fluor steht, wobei R⁵ in ortho-Position zu R⁴ steht.

Für R⁶ sind als bevorzugte Ausführungsformen Oxetanyl, Tetrahydrofuranyl, Tetrahydro-2H-pyran-4-yl, 1,1-Dioxidotetrahydro-2H-thiopyran-3-yl, 1,1-Dioxidotetrahydro-2H-thiopyran-2-yl, 1,1-Dioxidotetrahydro-2H-thiopyran-4-yl, 1,1-Dioxidotetrahydrothiophen-3-yl, 1,1-Dioxidotetrahydrothiophen-2-yl, 1,1-Dioxidothietan-2-yl oder 1,1-Dioxidothietan-3-yl zu nennen. Besonders bevorzugt ist hierbei Oxetanyl. Ganz besonders bevorzugt ist Oxetan-3-yl.

R⁷ steht ausschließlich im Zusammenhang mit den funktionellen Gruppen -SO₂- und -SO-, d.h. für eine mit R⁷ substituierte -SO₂- oder SO-Gruppe. In diesem Zusammenhang ist für R⁷ C₁-C₄-Alkyl bevorzugt, wobei der C₁-C₄-Alkylrest unsubstituiert oder einfach mit Hydroxy oder mit Cyclopropyl oder mit drei Fluoratomen substituiert ist. Weiterhin bevorzugt ist für R⁷ ein Cyclopropylrest. Besonders bevorzugt für R⁷ sind Methyl, Ethyl oder Hydroxyethyl. Ganz besonders ist Methyl für R⁷ bevorzugt.

Das bedeutet, dass im Falle eines mit R⁷SO₂- oder R⁷SO- substituierten C₁-C₆-Alkylrestes im Sinne von R¹ ein mit einem C₁-C₆-Alkyl-SO₂ oder einem C₁-C₆-Alkyl-SO substituiertes C₁-C₆-Alkyl bevorzugt ist. Hierbei sind für R¹ insbesondere Methylsulfonylethyl und Methylsulfonylpropyl bevorzugt. Ganz besonders bevorzugt ist hierbei Methylsulfonylethyl.

Für R⁸ ist ein unsubstituierter C₁-C₄-Alkylrest oder ein dreifach mit Fluor substituierter C₁-C₄-Alkylrest bevorzugt. Besonders bevorzugt sind Methyl, Ethyl, Trifluormethyl oder 2,2,2-Trifluorethyl. Ganz besonders bevorzugt sind Methyl, Trifluormethyl oder 2,2,2-Trifluorethyl.

Bevorzugt sind Verbindungen der Formel (I), in der
- R¹: für C₁-C₆-Alkyl steht, wobei der C₁-C₆-Alkylrest unsubstituiert oder ein- oder mehrfach, gleich oder verschieden substituiert ist mit Fluor, Hydroxy, einem Rest R⁶, R⁷SO₂, R⁷SO oder R⁸O;
- R² und R³: immer dieselbe Bedeutung haben und gleichzeitig entweder für Wasserstoff oder C₁-C₃-Alkyl stehen;
- R⁴: für Halogen, Cyano oder C₁-C₃-Alkyl steht, wobei der Ci-Cs-Alkylrest unsubstituiert oder ein- oder mehrfach, gleich oder verschieden substituiert ist mit Halogen oder Hydroxy;
- R⁵: für Wasserstoff, Fluor, Chlor oder C₁-C₃-Alkyl steht;
- R⁶: für Oxetanyl oder Tetrahydrofuranyl steht;
- R⁷: für C₁-C₄-Alkyl steht, wobei der C₁-C₄-Alkylrest unsubstituiert oder einfach mit Hydroxy oder mit Cyclopropyl oder mit drei Fluoratomen substituiert ist;
- R⁸: für unsubstituiertes C₁-C₄-Alkyl oder dreifach mit Fluor substituiertes C₁-C₄-Alkyl steht;
sowie ihre Diastereomere, Enantiomere, ihre Metabolite, ihre Salze, ihre Solvate oder die Solvate ihrer Salze.

Weiterhin bevorzugt sind Verbindungen der Formel (I), in der
- R¹: für C₂-C₆-Alkyl steht, wobei C₂-C₆-Alkyl unsubstituiert ist, oder C₂-C₆-Alkyl ein-, zwei- oder dreifach mit Fluor substituiert ist oder C₂-C₆-Alkyl einfach mit Hydroxy, R⁶, R⁷SO₂, oder R⁸O substituiert ist oder in der R¹ für ein mit Oxetanyl subsituiertes C₁-C₃ Alkyl steht;
- R² und R³: immer dieselbe Bedeutung haben und gleichzeitig entweder für Wasserstoff oder Methyl stehen;
- R⁴: für einen unsubstituierten oder einen ein- oder mehrfach mit Halogen substituierten C₁-C₃-Alkylrest oder einen mit einer Hydroxygruppe substituierten C₁-C₃-Alkylrest oder einen mit einer Hydroxygruppe und drei Fluoratomen substituierten C₁-C₃-Alkylrest steht;
- R⁵: für Wasserstoff, Fluor oder C₁-C₃ Alkyl steht;
- R⁷: für C₁-C₃-Alkyl steht:
- R⁸: für C₁-C₄-Alkyl steht, wobei der C₁-C₄-Alkylrest unsubstituiert oder ein-, zwei- oder dreifach mit Fluor substituiert ist;
sowie ihre Diastereomere, Enantiomere, ihre Metabolite, ihre Salze, ihre Solvate oder die Solvate ihrer Salze.

Besonders bevorzugt sind außerdem Verbindungen der allgemeinen Formel (I), in der
- R¹: für einen C₂-C₅-Alkylrest, der mit Hydroxy oder C₁-C₃-Alkoxy oder Trifluormethoxy oder 2,2,2-Trifluorethoxy oder Trifluormethyl substituiert ist oder für einen mit Methyl-SO₂ substituierten C₂-C₄-Alkylrest oder für einen mit Oxetan-3-yl subsituierten C₁-C₂ Alkylrest steht;
- R² und R³: immer dieselbe Bedeutung haben und gleichzeitig für Wasserstoff oder Methyl stehen;
- R⁴: für Methyl, Ethyl, Trifluor-C₁-C₃-alkyl, Difluor-C₁-C₃-Alkyl, Hydroxymethyl, 1-Hydroxyethyl, 2-Hydroxypropan-2-yl und 2,2,2-Trifluor-1-hydroxyethyl steht und
- R⁵: für Wasserstoff, Fluor oder Methyl steht;
sowie ihre Diastereomere, Enantiomere, ihre Metabolite, ihre Salze, ihre Solvate oder die Solvate ihrer Salze.

Ganz besonders bevorzugt sind Verbindungen, in denen
- R¹: für 4,4,4-Trifluorbutyl, 3-Hydroxy-3-methylbutyl, 3-Hydroxybutyl, 3-Methoxypropyl, 3-Hydroxypropyl, 3-Hydroxy-2-methylpropyl, 3-Hydroxy-2,2-dimethylpropyl, 3-Trifluormethoxypropyl, 2-Methoxyethyl, 2-Hydroxyethyl, 2-(Methylsulfonyl)ethyl oder 3-(Methylsulfonyl)propyl steht;
- R² und R³: gleichzeitig für Methyl oder Wasserstoff stehen und
- R⁴: für Difluormethyl, Trifluormethyl oder Methyl steht und
- R⁵: für Wasserstoff oder Fluor steht;
sowie ihre Diastereomere, Enantiomere, ihre Metabolite, ihre Salze, ihre Solvate oder die Solvate ihrer Salze.

Ganz besonders bevorzugt sind außerdem Verbindungen, in denen
- R': für 3-Hydroxy-3-methylbutyl, 3-Hydroxybutyl, 3-Hydroxy-2-methylpropyl, 3-Hydroxy-2,2-dimethylpropyl, 3-(Methylsulfonyl)propyl oder 2-(Methylsulfonyl)ethyl steht;
- R² und R³: gleichzeitig für Methyl stehen;
- R⁴: für Difluormethyl oder Trifluormethyl steht; und
- R⁵: für Wasserstoff steht;
sowie ihre Diastereomere, Enantiomere, ihre Metabolite, ihre Salze, ihre Solvate oder die Solvate ihrer Salze.

Weiterhin besonders bevorzugt sind außerdem Verbindungen, in denen
- R¹: für 3-Hydroxy-3-methylbutyl, 3-Hydroxybutyl, 3-Hydroxy-2-methylpropyl, 3-Hydroxy-2,2-dimethylpropyl, 3-(Methylsulfonyl)propyl oder 2-(Methylsulfonyl)ethyl steht;
- R² und R³: gleichzeitig für Methyl stehen;
- R⁴: für Methyl steht und
- R⁵: für Fluor steht, wobei R⁵ in ortho-Position zu R⁴ steht;
sowie ihre Diastereomere, Enantiomere, ihre Metabolite, ihre Salze, ihre Solvate oder die Solvate ihrer Salze.

Gegenstand der vorliegenden Erfindung sind insbesondere die folgenden Verbindungen:
1) N-[6-(2-Hydroxypropan-2-yl)-2-(2-methoxyethyl)-2H-indazol-5-yl]-6-(trifluormethyl)pyridin-2-carboxamid
2) N-[6-(Hydroxymethyl)-2-(2-methoxyethyl)-2H-indazol-5-yl]-6-(trifluormethyl)pyridin-2-carboxamid
3) N-[6-(2-Hydroxypropan-2-yl)-2-(3-methoxypropyl)-2H-indazol-5-yl]-6-(trifluormethyl)pyridin-2-carboxamid
4) N-[6-(Hydroxymethyl)-2-(3-methoxypropyl)-2H-indazol-5-yl]-6-(trifluormethyl)pyridin-2-carboxamid
5) N-[2-(2-Hydroxyethyl)-6-(2-hydroxypropan-2-yl)-2H-indazol-5-yl]-6-(trifluormethyl)pyridin-2-carboxamid
6) N-[6-(2-Hydroxypropan-2-yl)-2-(3-hydroxypropyl)-2H-indazol-5-yl]-6-(trifluormethyl)pyridin-2-carboxamid
7) N-[2-(2-Hydroxyethyl)-6-(hydroxymethyl)-2H-indazol-5-yl]-6-(trifluormethyl)pyridin-2-carboxamid
8) N-[6-(2-Hydroxypropan-2-yl)-2-(oxetan-3-ylmethyl)-2H-indazol-5-yl]-6-(trifluormethyl)pyridin-2-carboxamid
9) N-[6-(Hydroxymethyl)-2-(oxetan-3-ylmethyl)-2H-indazol-5-yl]-6-(trifluormethyl)pyridin-2-carboxamid
10) N-{6-(2-Hydroxypropan-2-yl)-2-[3-(methylsulfonyl)propyl]-2H-indazol-5-yl}-6-(trifluormethyl)pyridin-2-carboxamid
11) N-[2-(3-Hydroxy-3-methylbutyl)-6-(2-hydroxypropan-2-yl)-2H-indazol-5-yl]-6-(trifluormethyl)pyridin-2-carboxamid
12) N- {6-(2-Hydroxypropan-2-yl)-2- [2-(methylsulfonyl)ethyl]-2H-indazol-5-yl} -6-(trifluormethyl)pyridin-2-carboxamid
13) 6-(Difluormethyl)-N-[2-(3-hydroxy-3-methylbutyl)-6-(2-hydroxypropan-2-yl)-2H-indazol-5-yl]pyridin-2-carboxamid
14) 6-(Difluormethyl)-N-{6-(2-hydroxypropan-2-yl)-2-[2-(methylsulfonyl)ethyl]-2H-indazol-5-yl}pyridin-2-carboxamid
15) 6-(Difluormethyl)-N-[6-(2-hydroxypropan-2-yl)-2-(3-hydroxypropyl)-2H-indazol-5-yl]pyridin-2-carboxamid
16) N-[6-(2-Hydroxypropan-2-yl)-2-(4,4,4-trifluorbutyl)-2H-indazol-5-yl]-6-(trifluormethyl)pyridin-2-carboxamid
17) N- {6-(2-Hydroxypropan-2-yl)-2- [3-(trifluormethoxy)propyl] -2H-indazol-5-yl} -6-(trifluormethyl)pyridin-2-carboxamid
18) N-{6-(2-Hydroxypropan-2-yl)-2-[3-(2,2,2-trifluorethoxy)propyl]-2H-indazol-5-yl}-6-(trifluormethyl)pyridin-2-carboxamid
19) 5-Fluor-N-[2-(3-hydroxy-3-methylbutyl)-6-(2-hydroxypropan-2-yl)-2H-indazol-5-yl]-6-methylpyridin-2-carboxamid
20) N-[2-(3-Hydroxy-3-methylbutyl)-6-(2-hydroxypropan-2-yl)-2H-indazol-5-yl]-6-methylpyridin-2-carboxamid
21) 6-(2-Hydroxypropan-2-yl)-N-[6-(2-hydroxypropan-2-yl)-2-(4,4,4-trifluorbutyl)-2H-indazol-5-yl]pyridin-2-carboxamid
22) N-{2-[2-(1-Hydroxycyclopropyl)ethyl]-6-(2-hydroxypropan-2-yl)-2H-indazol-5-yl}-6-(trifluormethyl)pyridin-2-carboxamid.

Ein weiterer Gegenstand der Erfindung ist ein Verfahren zur Herstellung von Verbindungen der allgemeinen Formel (III) aus Verbindungen der allgemeinen Formel (II) 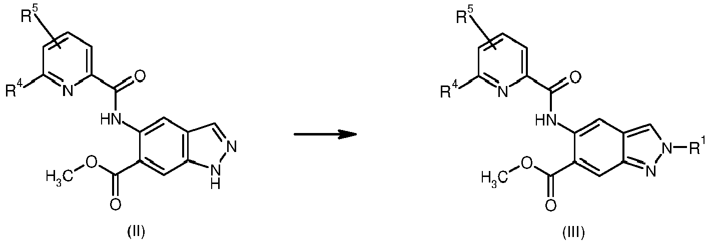 worin
- R¹: für 4,4,4-Trifluorbutyl, 3-Hydroxy-3-methylbutyl, 3-Methoxypropyl, 3-Hydroxypropyl, 3-Hydroxy-2-methylpropyl, 3-Hydroxy-2,2-dimethylpropyl, 3-Trifluormethoxypropyl, 2-Methoxyethyl, 2-Hydroxyethyl, 2-(Methylsulfonyl)ethyl, 3-(Methylsulfonyl)propyl oder 2-(1-Hydroxycyclopropyl)ethyl ist;
- R⁴: für Difluormethyl, Trifluormethyl oder Methyl steht; und
- R⁵: für Wasserstoff oder Fluor steht;
durch die Reaktion von (II) mit entsprechend substituierten Alkylhalogeniden oder Alkyl-4-methylbenzolsulfonaten in Gegenwart von Kaliumcarbonat.

Ein weiterer Gegenstand der Erfindung sind Verbindungen der allgemeinen Formel (III), 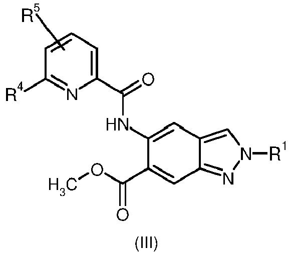 worin
- R¹: für 4,4,4-Trifluorbutyl, 3-Hydroxy-3-methylbutyl, 3-Methoxypropyl, 3-Hydroxypropyl, 3-Hydroxybutyl, 3-Hydroxy-2-methylpropyl, 3-Hydroxy-2,2-dimethylpropyl, 3-Trifluormethoxypropyl, 2-Methoxyethyl, 2-Hydroxyethyl, 2-(Methylsulfonyl)ethyl, 3-(Methylsulfonyl)propyl oder 2-(1-Hydroxycyclopropyl)ethyl ist;
- R⁴: für Difluormethyl, Trifluormethyl oder Methyl steht; und
- R⁵: für Wasserstoff oder Fluor steht,
sowie ihre Diastereomere, Enantiomere, ihre Metabolite, ihre Salze, ihre Solvate oder die Solvate ihrer Salze.

Bevorzugt sind insbesondere folgende Verbindungen der allgemeinen Formel (III), nämlich Methyl-5-{[(5-fluor-6-methylpyridin-2-yl)carbonyl]amino}-2-(3-hydroxy-3-methylbutyl)-2H-indazol-6-carboxylat und

Methyl-2-(3-hydroxy-3-methylbutyl)-5-({[6-(trifluormethyl)pyridin-2-yl]carbonyl}amino)-2H-indazol-6-carboxylat.

Die Verbindungen der allgemeinen Formel (III) sind geeignet zur Herstellung einer Teilmenge der Verbindungen der allgemeinen Formel (I).

Darüber hinaus sind die Verbindungen der allgemeinen Formel (III) Inhibitoren der Interleukin-1 Receptor Associated Kinase-4 (IRAK4).

Ein weiterer Gegenstand der Erfindung ist ein Verfahren zur Herstellung der erfindungsgemäßen Verbindungen der allgemeinen Formel (I) aus Verbindungen der Formel (III) 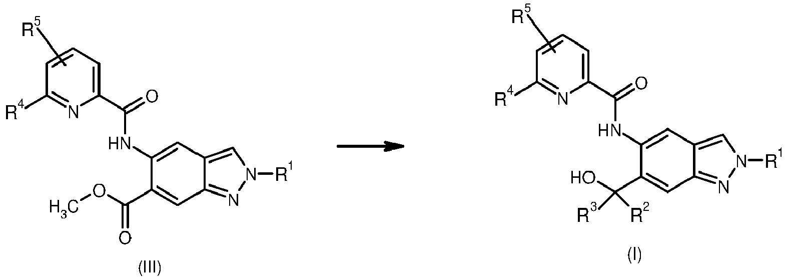 worin
- R¹: 4,4,4-Trifluorbutyl, 3-Hydroxy-3-methylbutyl, 3-Hydroxybutyl, 3-Methoxypropyl, 3-Hydroxypropyl, 3-Hydroxy-2-methylpropyl, 3-Hydroxy-2,2-dimethylpropyl, 3-Trifluormethoxypropyl, 2-Methoxyethyl, 2-Hydroxyethyl, 3-(Methylsulfonyl)propyl oder 2-(1-Hydroxycyclopropyl)ethyl ist;
- R² und R³: für Methyl stehen;
- R⁴: für Difluormethyl, Trifluormethyl oder Methyl steht; und
- R⁵: für Wasserstoff oder Fluor steht.
durch eine Grignard-Reaktion mit Methylmagnesiumbromid.

Die erfindungsgemäßen Verbindungen wirken als Inhibitoren der IRAK4 Kinase, und zeigen ein nicht vorhersehbares, wertvolles pharmakologisches Wirkspektrum.

Daher ist neben den weiter oben genannten ein weiterer Gegenstand der vorliegenden Erfindung die Verwendung der erfindungsgemäßen Verbindungen zur Behandlung und/oder Prophylaxe von Krankheiten bei Menschen und Tieren.

Die Behandlung und/oder Prophylaxe von gynäkologischen Erkrankungen, entzündlichen Hauterkrankungen, Herz-Kreislauf-Erkrankungen, Lungenerkrankungen, Augenerkrankungen, Autoimmunerkrankungen, Schmerzerkrankungen, Stoffwechselerkrankungen, Gicht, Lebererkrankungen, des metabolischen Syndroms, der Insulinresistenz und von Krebserkrankungen mit den erfindungsgemäßen IRAK4 Inhibitoren ist besonders bevorzugt.

Die erfindungsgemäßen Verbindungen sind geeignet für die Prophylaxe und/oder Behandlung von verschiedenen Erkrankungen und krankheitsbedingten Zuständen, insbesondere von TLR- (außer TLR3) und/oder IL-1-Rezeptorfamilie-vermittelten Erkrankungen bzw. Erkrankungen, dessen Pathologie direkt durch IRAK4 vermittelt ist. Als IRAK4-assoziierte Erkrankungen sind Multiple Sklerose, Atherosklerose, Herzinfarkt, Alzheimer, viral-induzierte Myokarditis, Gicht, Vogt-Koyanagi-Harada-Syndrom, Lupus erythematodes, Psoriasis, Spondyloarthritiden und Arthritis zu benennen.

Die erfindungsgemäßen Verbindungen können ferner für die Prophylaxe und/oder Behandlung von MyD88- und TLR (außer TLR3)-vermittelte Erkrankungen eingesetzt werden. Dies umfasst Multiple Sklerose, Rheumatoide Arthritis, Spondyloarthritiden (insbesondere Spondyloarthritis psoriatica und Morbus Bechterew), Metabolisches Syndrom inklusive Insulinresistenz, Diabetes mellitus, Osteoarthritis, Sjögren-Syndrom, Riesenzellarteriitis, Sepsis, Poly- und Dermatomyositis, Hauterkrankungen wie Psoriasis, atopische Dermatitis, Alopecia areata, Acne inversa und Acne vulgaris, pulmonale Erkrankungen wie Lungenfibrose, chronisch obstruktive Lungenerkrankung (COPD), akutes Atemnot-Syndrom (ARDS), akute Lungenschädigung (ALI), interstitielle Lungenerkrankung (ILD), Sarkoidose und pulmonale Hypertonie.

Aufgrund des Wirkmechanismus der erfindungsgemäßen Verbindungen sind sie zur Prophylaxe und/oder Behandlung der TLR-vermittelten Erkrankungen Behcet-Krankheit, Gicht, Endometriose sowie von Endometriose-assoziierten Schmerzen und anderen Endometriose-assoziierten Symptomen wie Dysmenorrhoe, Dyspareunie, Dysurie und Dyschezie geeignet. Des Weiteren sind die erfindungsgemäßen Verbindungen zur Prophylaxe und/oder Behandlung bei Transplantatabstoßung, von Lupus erythematodes, Adult Morbus Still-Krankheit und chronisch entzündlichen Darmerkrankungen wie Kolitis ulcerosa und Morbus Crohn geeignet.

Neben den bereits aufgeführten Erkrankungen ist die Verwendung der erfindungsgemäßen Verbindungen auch zur Behandlung und/oder Prävention folgender Erkrankungen geeignet: Augenerkrankungen wie Keratitis, allergisch bedingte Konjunktivitis, Keratoconjunctivitis sicca, Makuladegeneration und Uveitis; kardiovaskuläre Erkrankungen wie Atherosklerose, myokardialer Reperfusionsschaden, Myokardinfarkt, Hypertonie und neurologische Erkrankungen wie Alzheimer, Schlaganfall und Parkinson.

Der Wirkmechanismus der erfindungsgemäßen Verbindungen ermöglicht ferner die Prophylaxe und/oder Behandlung von TLR- und IL-1 Rezeptorfamilie vermittelten Lebererkrankungen, insbesondere NAFLD, NASH, ASH, Leberfibrose und Leberzirrhose.

Weiterhin ist die Prophylaxe und/oder Behandlung von Juckreiz und Schmerz, insbesondere von akutem, chronischem, entzündlichem und neuropathischem Schmerz durch die erfindungsgemäßen Verbindungen gegeben.

Aufgrund des Wirkmechanismus der erfindungsgemäßen Verbindungen sind sie zur Prophylaxe und/oder Behandlung von onkologischen Erkrankungen wie Lymphome, chronisch lymphatische Leukämie, Melanoma und Leberzellkarzinom, Brustkrebs, Prostatakrebs und Ras-abhängige Tumore geeignet.

Außerdem sind die erfindungsgemäßen Verbindungen geeignet zur Behandlung und/oder Prävention von Erkrankungen, die über die IL-1 Rezeptorfamilie vermittelt sind. Diese Erkrankungen umfassen CAPS (Cryopyrin-assoziierte periodische Syndrome) inklusive FCAS (familiäre Kälteurtikaria), MWS (Muckle-Wells-Syndrom), NOMID- (neonatal-onset multisystem inflammatory disease) und CONCA- (chronic infantile, neurological, cutaneous, and articular) Syndrom, FMF (familiäres Mittelmeerfieber), HIDS (Hyper-IgD-Syndrom), TRAPS (Tumornekrosefaktor-Rezeptor 1-assoziiertes periodisches Syndrom), juvenile idiopathische Arthritis, Adult Morbus Still-Krankheit, Morbus Adamantiades-Behcet, Rheumatoide Arthritis, Psoriasis Arthritis, Morbus Bechterew, Osteoarthritis, Keratoconjunctivitis sicca und Sjögren Syndrome, Multiple Sklerose, Lupus erythematodes, Alopecia areata, Diabetes mellitus Typ 1, Diabetes mellitus Typ 2 und die Folgen eines Myokardinfarktes. Pulmonale Erkrankungen wie Asthma, COPD, idiopathische interstitielle Pneumonie und ARDS, gynäkologische Erkrankungen wie Endometriose sowie Endometriose-assoziierte Schmerzen und andere Endometriose-assoziierte Symptome wie Dysmenorrhoe, Dyspareunie, Dysurie und Dyschezie, chronisch-entzündliche Darmerkrankungen wie Morbus Crohn und Kolitis ulcerosa sind mit einer Dysregulation der IL-1 Rezeptorfamilie assoziiert und für den therapeutischen und/oder prophylaktischen Einsatz der erfindungsgemäßen Verbindungen geeignet.

Die erfindungsgemäßen Verbindungen können ferner für Behandlung und/oder Prävention von IL-1 Rezeptorfamilie-vermittelten neurologischen Erkrankungen wie Hirnschlag, Alzheimer, Schlaganfall, Schädel-Hirn-Trauma und dermatologische Erkrankungen wie Psoriasis, atopische Dermatitis, Acne inversa, Alopecia areata und allergische Kontaktdermatitis eingesetzt werden.

Weiterhin sind die erfindungsgemäßen Verbindungen geeignet für die Behandlung und/oder Prophylaxe von Schmerzerkrankungen, insbesondere von akutem, chronischem, entzündlichem und neuropathischem Schmerz. Hierfür seien vorzugsweise Hyperalgesie, Allodynie, Schmerz bei Arthritis (wie Osteoarthritis, rheumatoider Arthritis und Spondyloarthritis), prämenstrueller Schmerz, Endometriose-assoziierter Schmerz, postoperativer Schmerz, Schmerz bei interstitieller Zystitis, CRPS (komplexes regionales Schmerzsyndrom), Trigeminusneuralgie, Schmerz bei Prostatitis, Schmerzen verursacht durch Rückenmarksverletzungen, entzündungsinduzierter Schmerz, Kreuzschmerzen, Krebsschmerzen, Chemotherapie-assoziierter Schmerz, HIV behandlungsinduzierte Neuropathie, Verbrennungs-induzierter Schmerz und chronischer Schmerz genannt.

Im Weiteren ist Gegenstand der vorliegenden Erfindung auch ein Verfahren zur Behandlung und/oder Prävention von Erkrankungen, insbesondere der zuvor genannten Erkrankungen, unter Verwendung einer wirksamen Menge von mindestens einer der erfindungsgemäßen Verbindungen.

Im Sinne der vorliegenden Erfindung umfasst der Begriff "Behandlung" oder "behandeln" ein Hemmen, Verzögern, Aufhalten, Lindern, Abschwächen, Einschränken, Verringern, Unterdrücken, Zurückdrängen oder Heilen einer Krankheit, eines Leidens, einer Erkrankung, einer Verletzung oder einer gesundheitlichen Störung, der Entfaltung, des Verlaufs oder des Fortschreitens solcher Zustände und/oder der Symptome solcher Zustände. Der Begriff "Therapie" wird hierbei als Synonym mit dem Begriff "Behandlung" verstanden.

Die Begriffe "Prävention", "Prophylaxe" oder "Vorbeugung" werden im Rahmen der vorliegenden Erfindung synonym verwendet und bezeichnen das Vermeiden oder Vermindern des Risikos, eine Krankheit, ein Leiden, eine Erkrankung, eine Verletzung oder eine gesundheitliche Störung, eine Entfaltung oder ein Fortschreiten solcher Zustände und/oder die Symptome solcher Zustände zu bekommen, zu erfahren, zu erleiden oder zu haben.

Die Behandlung oder die Prävention einer Krankheit, eines Leidens, einer Erkrankung, einer Verletzung oder einer gesundheitlichen Störung können teilweise oder vollständig erfolgen.

Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, enthaltend mindestens eine der erfindungsgemäßen Verbindungen und einen oder mehrere weitere Wirkstoffe, insbesondere zur Behandlung und/oder Prävention der zuvor genannten Erkrankungen. Als geeignete Kombinationswirkstoffe seien beispielhaft und vorzugsweise genannt: Allgemein sind Wirkstoffe wie antibakterielle (z.B. Penicilline, Vancomycin, Ciprofloxacin), antivirale (z.B. Aciclovir, Oseltamivir) und antimykotische (z.B. Naftifin, Nystatin) Substanzen und Gammaglobuline, immunomodulatorische und immunosuppressive Verbindungen wie Cyclosporin, Methotrexat^{®}, TNF-Antagonisten (z.B. Humira^{®},, Etanercept, Infliximab), IL-1 Inhibitoren (z.B. Anakinra, Canakinumab, Rilonacept), Phosphodiesterasehemmer (z.B Apremilast), Jak/STAT Inhibitoren (z.B. Tofacitinib, Baricitinib, GLPG0634), Leflunomid, Cyclophosphamid, Rituximab, Belimumab, Tacrolimus, Rapamycin, Mycophenolate mofetil, Interferone, Corticosteroide (z.B. Prednisone, Prednisolone, Methylprednisolone, Hydrocortisone, Betamethason), Cyclophophamide, Azathioprine und Sulfasalazine; Paracetamol, non-steroidale anti-inflammatorische Substanzen (NSAIDS) (z.B. Aspirin, Ibuprofen, Naproxen, Etodolac, Celecoxib, Colchicine) zu nennen.

Für die Tumortherapie seien zu nennen: Immunotherapie (z.B. Aldesleukin, Alemtuzumab, Basiliximab, Catumaxomab, Celmoleukin, Denileukin-Diftitox, Eculizumab, Edrecolomab, Gemtuzumab, Ibritumomab-Tiuxetan, Imiquimod, Interferon-alpha, Interferon-beta, Interferongamma, Ipilimumab, Lenalidomid, Lenograstim, Mifamurtid, Ofatumumab, Oprelvekin, Picibanil, Plerixafor, Polysaccharid-K, Sargramostim, Sipuleucel-T, Tasonermin, Teceleukin, Tocilizumab), antiproliferative Substanzen wie beispielsweise aber nicht ausschließlich Amsacrin, Arglabin, Arsentrioxid, Asparaginase, Bleomycin, Busulfan, Dactinomycin, Docetaxel, Epirubicin, Peplomycin, Trastuzumab, Rituximab, Obinutuzumab, Ofatumumab, Tositumomab, Aromatase Inhibitoren (z.B. Exemestan, Fadrozol, Formestan, Letrozol, Anastrozol, Vorozol), Antiestrogene (z.B. Chlormadinon, Fulvestrant, Mepitiostan, Tamoxifen, Toremifen), Estrogene (z.B. Estradiol, Polyestradiolphosphat, Raloxifen), Gestagene (z.B. Medroxyprogesteron, Megestrol), Topoisomerase I Inhibitoren (z.B. Irinotecan, Topotecan), Topoisomerasen II Inhibitoren (z.B. Amrubicin, Daunorubicin, Elliptiniumacetat, Etoposid, Idarubicin, Mitoxantron, Teniposid), Mikrotubuli-aktive Substanzen (z.B. Cabazitaxel, Eribulin, Paclitaxel, Vinblastin, Vincristin, Vindesin, Vinorelbin), Telomeraseinhibitoren (z.B. Imetelstat), alkylierende Substanzen und Histon-Deacetylasen Inhibitoren (z.B. Bendamustin, Carmustin, Chlormethin, Dacarbazin, Estramustin, Ifosfamid, Lomustin, Mitobronitol, Mitolactol, Nimustin Prednimustin, Procarbazin, Ranimustin, Streptozotocin, Temozolomid, Thiotepa, Treosulfan, Trofosfamid, Vorinostat, Romidepsin, Panobinostat); Substanzen, die Prozesse der Zelldifferenzierung beeinflussen wie Abarelix, Aminoglutethimid, Bexaroten, MMP Inhibitoren (Peptidmimetika, Nicht-Peptidmimetika und Tetracycline wie z.B. Marimastat, BAY 12-9566, BMS-275291, Clodronate, Prinomastat, Doxycycline), mTOR Inhibitoren (z.B. Sirolimus, Everolimus, Temsirolimus, Zotarolimus), Antimetabolite (z.B. Clofarabin, Doxifluridin, Methotrexat, 5-Fluoruracil, Cladribin, Cytarabin, Fludarabin, Mercaptopurin, Methotrexat, Pemetrexed, Raltitrexed, Tegafur, Tioguanin), Platinverbindungen (z.B. Carboplatin, Cisplatin, Cisplatinum, Eptaplatin, Lobaplatin, Miriplatin, Nedaplatin, Oxaliplatin); Antiangiogene Verbindungen (z.B. Bevacizumab), antiandrogene Verbindungen (z.B. Bevacizumab, Enzalutamid, Flutamid, Nilutamid, Bicalutamid, Cyproteron, Cyproteronacetat), Proteasomeinhibitoren (z.B. Bortezomib, Carfilzomib, Oprozomib, ONYX0914), Gonadoliberin-Agonisten und -Antagonisten (z.B. Abarelix, Buserelin, Deslorelin, Ganirelix, Goserelin, Histrelin, Triptorelin, Degarelix, Leuprorelin), Methionine Aminopeptidase Inhibitoren (z.B. Bengamid-Derivate, TNP-470, PPI-2458), Heparanaseinhibitoren (z.B. SST0001, PI-88); Inhibitoren gegen genetisch verändertes Ras-Protein (z.B. Farnesyl-Transferase Inhibitoren wie Lonafarnib, Tipifarnib), HSP90 Inhibitoren (z.B. Geldamycin-Derivate wie 17-Allylaminogeldanamycin, 17-demethoxygeldanamycin (17AAG), 17-DMAG, Retaspimycin Hydrochloride, IPI-493, AUY922, BIIB028, STA-9090, KW-2478), Kinesin Spindleprotein Inhibitoren (z.B. SB715992, SB743921, Pentamidine/Chlorpromazine), MEK (mitogen-activated protein kinase kinase) Inhibitoren (z.B. Trametinib, BAY 86-9766 (Refametinib), AZD6244), Kinase-Inhibitoren (z.B.: Sorafenib, Regorafenib, Lapatinib, Sutent, Dasatinib, Cetuximab, BMS-908662, GSK2118436, AMG 706, Erlotinib, Gefitinib, Imatinib, Nilotinib, Pazopanib, Roniciclib, Sunitinib, Vandetanib, Vemurafenib), Hedgehog Signalinhibitoren (z.B. Cyclopamin, Vismodegib), BTK (Bruton's Tyrosine Kinase) Inhibitor (z.B. Ibrutinib), JAK/pan-JAK (Janus Kinase) Inhibitor (z.B. SB-1578, Baricitinib, Tofacitinib, Pacritinib, Momelotinib, Ruxolitinib, VX-509, AZD-1480, TG-101348), PI3K Inhibitor (z.B. BAY 1082439, BAY 80-6946 (Copanlisib), ATU-027, SF-1126, DS-7423, GSK-2126458, Buparlisib, PF-4691502, BYL-719, XL-147, XL-765, Idelalisib), SYK (Spleen Tyrosine Kinase) Inhibitor (z.B. Fostamatinib, Excellair, PRT-062607), p53-Gentherapie, Bisphosphonate (z.B. Etridonat, Clodronat, Tiludronat, Pamidronat, Alendronsäure, Ibandronat, Risedronat, Zoledronat). Zur Kombination sind außerdem beispielhaft, aber nicht ausschließlich folgende Wirkstoffe zu nennen: Rituximab, Cyclophosphamide, Doxorubicin, Doxorubicin in Kombination mit Estron, Vincristine, Chlorambucil, Fludarabin, Dexamethasone, Cladribin, Prednisone, 131I-chTNT, Abirateron, Aclarubicin, Alitretinoin, Bisantren, Calciumfolinat, Calciumlevofolinat, Capecitabin, Carmofur, Clodronsäure, Romiplostim, Crisantaspase, Darbepoetin-alfa, Decitabin, Denosumab, Dibrospidiumchlorid, Eltrombopag, Endostatin, Epitiostanol, Epoetin-alfa, Filgrastim, Fotemustin, Galliumnitrat, Gemcitabin, Glutoxim, Histamindihydrochlorid, Hydroxycarbamid, Improsulfan, Ixabepilon, Lanreotid, Lentinan, Levamisol, Lisurid, Lonidamin, Masoprocol, Methyltestosteron, Methoxsalen, Methylaminolevulinat, Miltefosin, Mitoguazon, Mitomycin, Mitotan, Nelarabin, Nimotuzumab, Nitracrin, Omeprazol, Palifermin, Panitumumab, Pegaspargase, PEG-epoetin-beta (Methoxy-PEG-epoetin-beta), Pegfilgrastim, Peg-interferon-alfa-2b, Pentazocin, Pentostatin, Perfosfamid, Pirarubicin, Plicamycin, Poliglusam, Porfimer-Natrium, Pralatrexat, Quinagolid, Razoxan, Sizofiran, Sobuzoxan, Natriumglycididazol, Tamibaroten, die Kombination von Tegafur und Gimeracil und Oteracil, Testosteron, Tetrofosmin, Thalidomid, Thymalfasin, Trabectedin, Tretinoin, Trilostan, Tryptophan, Ubenimex, Vapreotid, Yttrium-90-Glasmikrokugeln, Zinostatin, Zinostatin-Stimalamer.

Für die Tumortherapie geeignet ist auch eine Kombination aus nicht-medikamentöser Therapie wie Chemotherapie (z.B. Azacitidin, Belotecan, Enocitabine, Melphalan, Valrubicin, Vinflunin, Zorubicin), Radiotherapie (z.B. I-125-Seeds, Palladium-103-Seed, Radium-223-chlorid) oder Phototherapie (z.B. Temoporfin, Talaporfin), die von einer medikamentösen Behandlung mit den erfindungsgemäßen IRAK4 Inhibitoren begleitet werden oder die nach Beendigung der nichtmedikamentösen Tumortherapie wie Chemotherapie, Radiotherapie oder Phototherapie durch eine medikamentöse Behandlung mit den erfindungsgemäßen IRAK4 Inhibitoren ergänzt werden.

Die erfindungsgemäßen IRAK4 Inhibitoren können außer mit den bereits genannten auch mit folgenden Wirkstoffen kombiniert werden:
Wirkstoffe für die Alzheimertherapie wie beispielsweise Acetylcholinesterase-Hemmern (z.B. Donepezil, Rivastigmine, Galantamin, Tacrin), NMDA (N-Methyl-D-Aspartat)-Rezeptorantagonisten (z.B. Memantine); L-DOPA/Carbidopa (L-3,4-Dihydroxyphenylalanin), COMT (Catechol-O-Methyltransferase)-Hemmer (z.B. Entacapon), Dopaminagonisten (z.B. Ropinrol, Pramipexol, Bromocriptin), MAO-B (Monoaminooxidase-B)-Hemmer (z.B. Selegilin), Anticholinergika (z.B. Trihexyphenidyl) und NMDA-Antagonisten (z.B. Amantadin) zur Behandlung von Parkinson; Beta-Interferon (IFN-beta) (z.B. IFN beta-1b, IFN beta-1a Avonex^{®} und Betaferon^{®}), Glatirameracetat, Immunglobuline, Natalizumab, Fingolimod und Immunsuppressiva wie Mitoxantron, Azathioprin und Cyclophosphamid zur Behandlung der Multiplen Sklerose; Substanzen zur Behandlung von pulmonalen Erkrankungen wie beispielsweise Beta-2-Sympathomimetika (z.B. Salbutamol), Anticholinergika (z.B. Glycopyrronium), Methylxanthine (z.B. Theophyllin), Leukotrienrezeptor-Antagonist (z.B. Montelukast), PDE-4 (Phosphodiesterase Typ 4)-Hemmer (z.B. Roflumilast), Methotrexat, IgE Antikörper, Azathioprin und Cyclophosphamid, Kortisolhaltige Präparate; Substanzen zur Behandlung von Osteoarthritis wie non-steroidale anti-inflammatorische Substanzen (NSAIDs). Neben den zwei genannten Therapien sind für rheumatoide Erkrankungen wie beispielsweise rheumatoide Arthritis, Spondyloarthritiden und juvenile idiopathische Arthritis Methotrexat und Biologika zur B-Zell- und T-Zell-Therapie (z.B. Rituximab, Abatacept) zu nennen. Neurotrophe Substanzen wie Acetylcholinesterase Inhibitoren (z.B. Donepezil), MAO (Monoaminooxidase) Inhibitoren (z.B. Selegilin), Interferone und Antikonvulsivum (z.B. Gabapentin); Wirkstoffe zur Behandlung von kardiovaskulären Erkrankungen wie beta-Blocker (z.B. Metoprolol), ACE Inhibitoren (z.B. Benazepril), Angiotensin-Rezeptorblocker (z.B. Losartan, Valsartan), Diuretika (z.B. Hydrochlorothiazid), Calciumkanal-Blocker (z.B. Nifedipin), Statine (z.B. Simvastatin, Fluvastatin); Anti-Diabetika wie z.B. Metformin, Glinide (z.B. Nateglinid), DPP-4 (Dipeptidyl-Peptidase-4)-Inhibitoren (z.B. Linagliptin, Saxagliptin, Sitagliptin, Vildagliptin), SGLT2 (sodium/glucose cotransporter 2)-Inhibitoren/ Gliflozin (z.B. Dapagliflozin, Empagliflozin), Inkretinmimetika (Hormone Glukoseabhängiges insulinotropes Peptid (GIP)- und Glucagon-like Peptid 1 (GLP-1)-Analoga/Agonisten) (z.B. Exenatid, Liraglutid, Lixisenatide), α-Glucosidase-Hemmer (z.B. Acarbose, Miglitol, Voglibiose) und Sulfonylharnstoffe (z.B. Glibenclamid, Tolbutamid), Insulin-Sensitizer (z.B. Pioglitazon) und Insulintherapie (z.B. NPH-Insulin, Insulin lispro), Substanzen zur Behandlung einer Hypoglykämie zur Behandlung von Diabetes und metabolischem Syndrom. Lipidsenker wie beispielsweise Fibrate (z.B. Bezafibrat, Etofibrat, Fenofibrat, Gemfibrozil), Nikotinsäurederivate (z.B. Nicotinsäure/Laropiprant), Ezetimib, Statine (z.B. Simvastatin, Fluvastatin), Anionenaustauscher (z.B. Colestyramin, Colestipol, Colesevelam). Wirkstoffe wie Mesalazin, Sulfasalazin, Azathioprin, 6-Mercaptopurin oder Methotrexat, probiotische Bakterien (Mutaflor, VSL#3^{®}, Lactobacillus GG, Lactobacillus plantarum, L. acidophilus, L. casei, Bifidobacterium infantis 35624, Enterococcus fecium SF68, Bifidobacterium longum, Escherichia coli Nissle 1917), Antibiotika wie beispielsweise Ciprofloxacin und Metronidazol, Antidiarrhoika wie z.B. Loperamid oder Laxativa (Bisacodyl) zur Behandlung von chronisch-entzündlichen Darmerkrankungen. Immunsuppressiva wie Glucocorticoide und non-steroidale anti-inflammatorische Substanzen (NSAIDs), Cortison, Chloroquin, Cyclosporin, Azathioprin, Belimumab, Rituximab, Cyclophosphamid zur Behandlung von Lupus erythematodes. Beispielhaft aber nicht ausschließlich Calcineurinhemmer (z.B. Tacrolimus und Ciclosporin), Zellteilungshemmer (z.B. Azathioprin, Mycophenolat Mofetil, Mycophenolsäure, Everolimus oder Sirolimus), Rapamycin, Basiliximab, Daclizumab, Anti-CD3-Antikörper, Anti-T-Lymphozytenglobulin/Anti-Lymphozytenglobulin bei Organtransplantation. Vitamin D3-Analoga wie beispielsweise Calcipotriol, Tacalcitol oder Calcitriol; Salicylsäure, Harnstoff, Ciclosporin, Methotrexat, Efalizumab bei dermatologischen Erkrankungen.

Weiterhin seien Arzneimittel genannt, die mindestens eine der erfindungsgemäßen Verbindungen und einen oder mehrere weitere Wirkstoffe enthalten, insbesondere EP4-Inhibitoren (Prostaglandin E2 Receptor 4 Inhibitoren), P2X3- Inhibitoren (P2X Purinoceptor 3), PTGES-Inhibitoren (Prostaglandin E Synthase Inhibitoren) oder AKR1C3-Inhibitoren (Aldo-keto reductase family 1 member C3 Inhibitoren), zur Behandlung und/oder Prävention der zuvor genannten Erkrankungen.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, über das Ohr oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorpher und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Ein weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### Herstellung der erfindungsgemäßen Verbindungen

Die Herstellung der erfindungsgemäßen Verbindungen wird durch die folgenden Syntheseschemata verdeutlicht.

Als Ausgangsmaterial zur Synthese der erfindungsgemäßen Verbindungen werden Carbonsäuren (Intermediat V3) verwendet, welche kommerziell erhältlich sind oder auf literaturbekannten oder analog zu literaturbekannten Wegen (siehe zum Beispiel European Journal of Organic Chemistry 2003, 8, 1559 - 1568, Chemical and Pharmaceutical Bulletin, 1990, 38, 9, 2446 - 2458, Synthetic Communications 2012, 42, 658 - 666, Tetrahedron, 2004 , 60, 51, 11869 - 11874) hergestellt werden können (siehe beispielsweise Syntheseschema 1). Einige Carbonsäuren V3 können ausgehend von Carbonsäureestern (Intermediat V2) durch Verseifung (vergleiche zum Beispiel die Umsetzung von Ethyl-6-(hydroxymethyl)pyridin-2-carboxylat mit wässriger Natriumhydroxidlösung in Methanol, WO200411328) oder - in dem Fall, dass es sich um einen *tert*-Butylester handelt - durch Reaktion mit einer Säure wie zum Beispiel Chlorwasserstoff oder Trifluoressigsäure (vergleiche zum Beispiel Dalton Transactions, 2014 , 43, 19, 7176 - 7190) hergestellt werden. Die Carbonsäuren V3 können auch in Form ihrer Alkalimetallsalze verwendet werden. Die Herstellung der Intermediate V2 kann gegebenenfalls aus den Intermediaten V1 erfolgen, welche als Substituent X' ein Chlor, Brom oder Iod tragen, durch Umsetzung in einer KohlenmonoxidAtmosphäre gegebenenfalls unter Überdruck in Gegenwart eines Phosphinliganden wie zum Beispiel 1,3-Bis(diphenylphoshino)propan, einer Palladium-Verbindung wie zum Beispiel Palladium(II)acetat und einer Base wie zum Beispiel Triethylamin unter Zusatz von Ethanol oder Methanol in einem Lösemittel wie zum Beispiel Dimethylsulfoxid (für Herstellungsmethoden vergleiche zum Beispiel WO2012112743, WO 2005082866, Chemical Communications (Cambridge, England), 2003, 15, 1948 - 1949, WO200661715). Die Intermediate V1 sind entweder kommerziell erhältlich oder können auf literaturbekannten Wegen hergestellt werden. Beispielhafte Herstellungsmethoden sind in WO 2012061926, European Journal of Organic Chemistry, 2002, 2, 327 - 330, Synthesis, 2004, 10, 1619 - 1624, Journal of the American Chemical Society, 2013, 135, 32, 12122 - 12134, Bioorganic and Medicinal Chemistry Letters, 2014, 24, 16, 4039 - 4043, US2007185058, WO2009117421 aufgeführt. 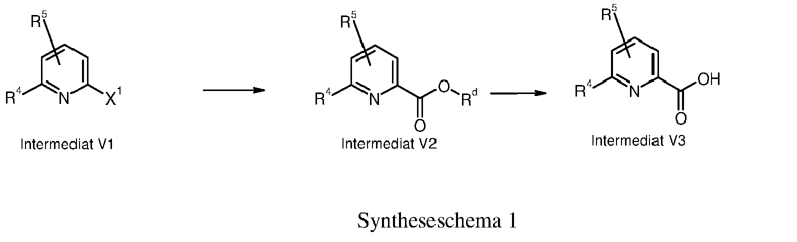
X' bedeutet Chlor, Brom oder Iod.
R^{d} bedeutet Methyl, Ethyl, Benzyl oder *tert*-Butyl.
R⁴, R⁵ haben die in der allgemeinen Formel (I) beschriebenen Definitionen.

Methyl-5-amino-1H-indazol-6-carboxylat (Intermediat 2) kann ausgehend von Methyl-1H-indazol-6-carboxylat (Intermediat 0) gemäß Syntheseschema 2 durch Nitrierung und Reduktion der Nitrogruppe von Intermediat 1 durch Wasserstoff in Gegenwart von Palladium auf Kohle analog zu WO 2008/001883 erhalten werden. Zur Herstellung der Intermediate 3 ausgehend vom Intermediat 2 können verschiedene in der Literatur bekannte Kupplungsreagenzien eingesetzt werden (Amino Acids, Peptides and Proteins in Organic Chemistry. Vol.3 - Building Blocks, Catalysis and Coupling Chemistry, Andrew B. Hughes, Wiley, Kapitel 12 - Peptide-Coupling Reagents, 407-442; Chem. Soc. Rev., 2009, 38, 606). Beispielsweise können 1-(3-Dimethylaminopropyl)-3-ethylcarbodiimidhydrochlorid in Kombination mit 1-Hydroxy-1H-benzotriazol Hydrat (HOBt, WO2012107475; Bioorg. Med. Chem. Lett., 2008 , 18, 2093), (1H-Benzotriazol-1-yloxy)(dimethylamino)-N,N-dimethylmethaniminiumtetrafluoroborat (TBTU, CAS 125700-67-6), (Dimethylamino)-N,N-dimethyl(3H-[1,2,3]triazolo[4,5-b]pyridin-3-yloxy)methaneiminiumhexafluorophosphat (HATU, CAS 148893-10-1), Propanphosphonsäureanhydrid (als Lösung in Ethylacetat oder DMF, CAS68957-94-8) oder Di-1H-imidazol-1-ylmethanon (CDI) als Kupplungsreagenzien verwendet werden, wobei jeweils zur Reaktionsmischung noch eine Base wie Triethylamin oder N-Ethyl-N-isopropylpropan-2-amin gegeben wird. Bevorzugt ist die Verwendung von TBTU und N-Ethyl-N-isopropylpropan-2-amin in THF. 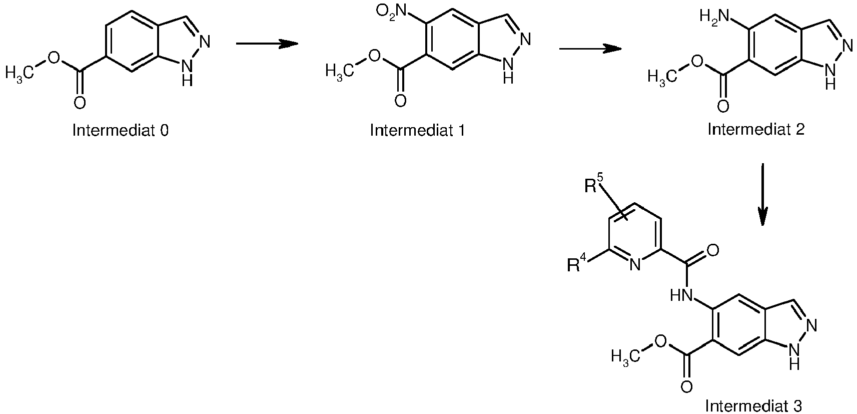 Syntheseschema 2

Die Substituenten R⁴, R⁵ haben die in der allgemeinen Formel (I) angegebenen Definitionen.

Ausgehend von den Intermediaten 3 können 2-substituierte Indazolderivate (Intermediate 4) hergestellt werden (siehe Syntheseschema 3). Hierfür kommen Reaktionen mit gegebenenfalls substituierten Alkylchloriden, Alkylbromiden, Alkyliodiden oder Alkyl-4-methylbenzolsulfonaten in Betracht. Die verwendeten Alkylhalogenide oder Alkyl-4-methylbenzolsulfonate sind kommerziell erhältlich oder können analog zu literaturbekannten Wegen hergestellt werden (für die Herstellung von Alkyl-4-methylbenzolsulfonaten sei zum Beispiel die Umsetzung eines entsprechenden Alkoholes mit 4-Methylbenzolsulfonylchlorid in Gegenwart von Triethylamin oder Pyridin genannt, siehe zum Beispiel Bioorganic and Medicinal Chemistry, 2006, 14, 12 4277 - 4294). Gegebenenfalls kann bei der Verwendung von Alkylchloriden oder Alkylbromiden noch ein Alkalimetalliodid zugegeben werden wie Kaliumiodid oder Natriumiodid. Als Basen können beispielsweise Kaliumcarbonat, Cäsiumcarbonat oder Natriumhydrid verwendet werden. Bei reaktiven Alkylhalogeniden kann in einigen Fällen auch N-Cyclohexyl-N-methylcyclohexanamin zur Verwendung kommen. Als Lösemittel kommen zum Beispiel 1-Methylpyrrolidin-2-on, DMF, DMSO oder THF in Betracht. Gegebenenfalls können die verwendeten Alkylhalogenide oder Alkyl-4-methylbenzolsulfonate funktionelle Gruppen aufweisen, die vorher gegebenenfalls mit einer Schutzgruppe geschützt wurden (vergleiche auch P. G. M. Wuts, T. W. Greene, Greene's Protective Groups in Organic Synthesis, Fourth Edition, ISBN: 9780471697541). Kommen beispielsweise Alkylhalogenide oder Alkyl-4-methylbenzolsulfonate zum Einsatz, die eine oder mehrere Hydroxygruppen aufweisen, so können diese Hydroxygruppen gegebenenfalls durch eine *tert*-Butyl(dimethyl)silyl-Gruppe oder eine ähnliche dem Fachmann geläufige Silicium-haltige Schutzgruppe geschützt vorliegen. Alternativ können die Hydroxygruppen aber auch mit der Tetrahydro-2H-pyran (THP)-Gruppe oder mit der Acetyl- oder Benzoylgruppe geschützt vorliegen. Die verwendeten Schutzgruppen können dann nachfolgend der Synthese von Intermediat 4, aber auch nach der Synthese von (I) abgespalten werden. Wird zum Beispiel eine *tert-*Butyl(dimethylsilyl)gruppe als Schutzgruppe verwendet, so kann diese unter Verwendung von Tetrabutylammoniumfluorid in einem Lösemittel wie zum Beispiel THF abgespalten werden. Eine THP-Schutzgruppe kann zum Beispiel unter Verwendung von 4-Methylbenzolsulfonsäure (ggf. als Monohydrat) abgespalten werden. Acetylgruppen oder Benzoylgruppen können durch Behandlung mit wässriger Natronlauge abgespalten werden.

Gegebenenfalls können die verwendeten Alkylhalogenide oder Alkyl-4-methylbenzolsulfonate funktionelle Gruppen enthalten, die durch dem Fachmann bekannte Oxidations- oder Reduktionsreaktionen umgesetzt werden können (vergleiche zum Beispiel Science of Synthesis, Georg Thieme Verlag). Handelt es sich beispielsweise bei der funktionellen Gruppe um eine Sulfidgruppe, so kann diese durch literaturbekannte Methoden zu einer Sulfoxid- oder Sulfongruppe oxidiert werden. Handelt es sich um eine Sulfoxidgruppe so kann diese ebenfalls zu einer Sulfongruppe oxidiert werden. Beispielsweise kann für diese Oxidationsschritte 3-Chloroperbenzoesäure (CAS 937-14-4) verwendet werden (vergleiche hierzu zum Beispiel auch US201094000 für die Oxidation eines 2-(Methylsulfanyl)ethyl-1H-pyrazol-Derivates zu einem 2-(Methylsulfinyl)ethyl-1H-pyrazol-Derivat und die Oxidation eines weiteren 2-(Methylsulfanyl)ethyl-1H-pyrazol-Derivates zu einem 2-(Methylsulfonyl)ethyl-1H-pyrazol-Derivat). Enthalten die verwendeten Alkylhalogenide oder -tosylate eine Ketogruppe, so kann diese durch dem Fachmann bekannte Reduktionsmethoden zu einer Alkoholgruppe reduziert werden (vergleiche zum Beispiel Chemische Berichte, 1980, 113, 1907 - 1920 für die Verwendung von Natriumborhydrid). Diese Oxidations- oder Reduktionsschritte können nachfolgend der Synthese von Intermediat 4, aber auch nach der Synthese der erfindungsgemäßen Verbindungen der allgemeinen Formel (I) erfolgen. Alternativ kann die Herstellung von Intermediat 4 durch Mitsunobu Reaktion (siehe zum Beispiel K. C. K. Swamy et. al. Chem. Rev. 2009, 109, 2551 - 2651) von Intermediat 3 mit gegebenenfalls substituierten Alkylalkoholen erfolgen. Verschiedene Phosphine wie Triphenylphosphin, Tributylphosphin oder 1,2-Diphenylphosphinoethan in Kombination mit Azodicarbonsäurediisopropylester (CAS 2446-83-5) oder weiteren in der Literatur genannten Diazenderivaten (K. C. K. Swamy et. al. Chem. Rev. 2009, 109, 2551 - 2651) können benutzt werden. Bevorzugt ist die Verwendung von Triphenylphosphin und Azodicarbonsäurediisopropylester. Trägt der Alkylalkohol eine funktionelle Gruppe, so können - wie bei den oben genannten Reaktionen mit Alkylhalogeniden - bekannte Schutzgruppenstrategien verwendet werden (weitere Hinweise sind P. G. M. Wuts, T. W. Greene, Greene's Protective Groups in Organic Synthesis, Fourth Edition, ISBN: 9780471697541 zu entnehmen) sowie - wie bei den oben genannten Reaktionen mit Alkylhalogeniden - Oxidations- oder Reduktionsschritte entsprechend der Synthese von Intermediat 4, aber auch nach der Synthese der erfindungsgemäßen Verbindungen der allgemeinen Formel (I) erfolgen. Ausgehend von Intermediat 4 können erfindungsgemäße Verbindungen der allgemeinen Formel (I) mit der Bedeutung R² und R³ = Ci-C₆-Alkyl (wobei R² und R³ dieselbe Bedeutung haben), durch eine Grignard-Reaktion erhalten werden (vergleiche zum Beispiel die Umsetzung eines Methyl-1H-indazol-6-carboxylat-Derivates mit Methylmagnesiumbromid in EP 2489663). Für die Grignard-Reaktion können Alkylmagnesiumhalogenide verwendet werden. Besonders bevorzugt sind Methylmagnesiumchlorid oder Methylmagnesiumbromid in THF oder Diethylether oder auch in Mischungen aus THF und Diethylether. Alternativ können ausgehend von Intermediat 4 erfindungsgemäße Verbindungen der allgemeinen Formel (I) mit der Bedeutung R² und R³ = Ci-C₆-Alkyl (wobei R² und R³ dieselbe Bedeutung haben), durch eine Reaktion mit einem Alkyllithiumreagenz erhalten werden (vergleiche zum Beispiel die Umsetzung eines Methyl-2-amino-4-chlor-1-methyl-1H-benzimidazol-7-carboxylat-Derivates mit Isopropyllithium oder tert-Butyllithiumin WO2006116412). Ausgehend von Intermediat 4 können erfindungsgemäße Verbindungen der allgemeinen Formel (I) mit der Bedeutung R² = H und R³ = H durch Reduktion mit Lithiumaluminiumhydrid in THF, Lithiumborhydrid in THF oder Natriumborhydrid in THF gegebenenfalls unter Zusatz von Methanol oder Mischungen aus Lithiumborhydrid und Natriumborhydrid hergestellt werden.

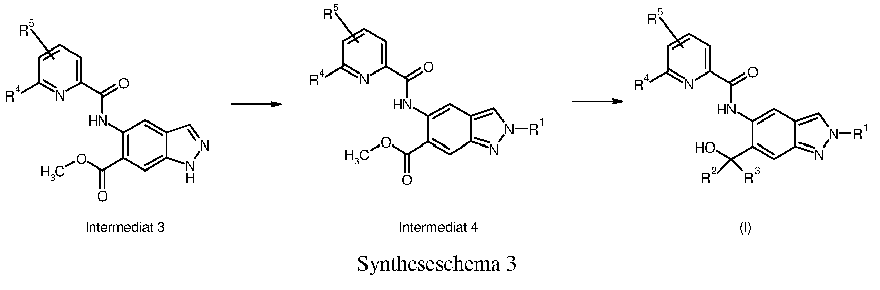

Die Substituenten R¹, R², R³, R⁴, R⁵ haben die in der allgemeinen Formel (I) angegebenen Definitionen.

Ausgehend von Intermediat 3 kann Intermediat 5 mit der Bedeutung R² und R³ = C₁-C₆-Alkyl (wobei R² und R³ dieselbe Bedeutung haben) durch eine Grignard-Reaktion erhalten werden (siehe Syntheseschema 4). Hierfür können geeignete Alkylmagnesiumhalogenide wie zum Beispiel Methylmagnesiumchlorid oder Methylmagnesiumbromid in THF oder in Diethylether oder auch in Mischungen aus THF und Diethylether verwendet werden.

Ausgehend von Intermediat 5 kann dann eine Teilmenge (I-a) mit der Bedeutung R² und R³ = Ci-C₆-Alkyl (wobei R² und R³ dieselbe Bedeutung haben) der erfindungsgemäßen Verbindungen (I) hergestellt werden. Hierfür kommen analog zu Syntheseschema 3 (Herstellung von Intermediat 3) Reaktionen von Intermediat 5 mit gegebenenfalls substituierten Alkylchloriden, Alkylbromiden, Alkyliodiden oder Alkyl-4-methylbenzolsulfonaten in Betracht. Es können Schutzgruppenstrategien analog zu den in Syntheseschema 3 beschriebenen verwendet werden. Alternativ kann zur Herstellung einer Teilmenge (I-a) mit der Bedeutung R² und R³ = C₁-C₆-Alkyl (wobei R² und R³ dieselbe Bedeutung haben) der erfindungsgemäßen Verbindungen (I) die Mitsunobu Reaktion von Intermediat 5 mit gegebenenfalls substituierten Alkylalkoholen verwendet werden (analog zu Syntheseschema 3).

Beinhaltet R¹ der Verbindungen der Formel (I-a) eine geeignete funktionelle Gruppe, so können gegebenenfalls nachfolgend in Analogie zu Syntheseschema 3 Oxidations- bzw. Reduktionsreaktionen verwendet werden zur Herstellung weiterer erfindungsgemäßer Verbindungen.

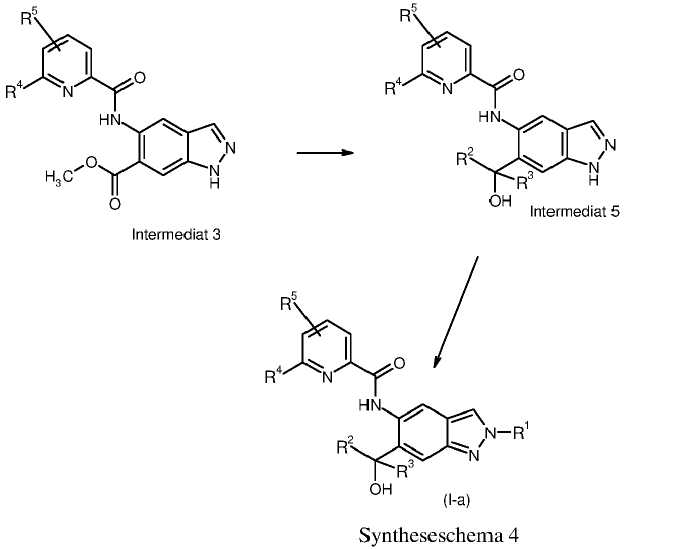

Die Substituenten R¹, R⁴, R⁵ haben die in der allgemeinen Formel (I) angegebenen Definitionen. R² und R³ haben immer dieselbe Bedeutung und stehen gleichzeitig für C₁-C₆-Alkyl.

Ausgehend von Intermediat 1 kann Intermediat 4 auf alternative Weise hergestellt werden (siehe Syntheseschema 5). Zuerst wird Intermediat 1 mit Methoden wie bei Syntheseschema 3 (Herstellung von Intermediat 4 aus Intermediat 3) beschrieben zu Intermediat 6 umgesetzt.

Das Intermediat 6 kann dann durch Reduktion der Nitrogruppe zu Intermediat 7 umgesetzt werden. Beispielsweise kann die Nitrogruppe mit Palladium auf Kohlenstoff unter einer Wasserstoffatmosphäre reduziert werden (vergleiche zum Beispiel WO2013174744 für die Reduktion von 6-Isopropoxy-5-nitro-1H-indazol zu 6-Isopropoxy-1H-indazol-5-amin) oder durch die Verwendung von Eisen und Ammoniumchlorid in Wasser und Ethanol (siehe zum Beispiel auch Journal of the Chemical Society, 1955, 2412 -2419), oder durch die Verwendung von Zinn(II)-chlorid (CAS 7772-99-8). Die Verwendung von Eisen und Ammoniumchlorid in Wasser und Ethanol ist bevorzugt. Die Herstellung von Intermediat 4 aus Intermediat 7 kann analog zu Syntheseschema 2 (Herstellung von Intermediat 3 aus Intermediat 2) erfolgen.

Wie bei Syntheseschema 3 beschrieben, können gegebenenfalls auch bei Syntheseschema 5 Schutzgruppenstrategien verwendet werden. Gegebenenfalls können zusätzlich ausgehend von Intermediat 6 bzw. Intermediat 7 wie bei Syntheseschema 3 beschrieben dem Fachmann bekannte Oxidation- oder Reduktionsreaktionen durchgeführt werden (vergleiche zum Beispiel Science of Synthesis, Georg Thieme Verlag). 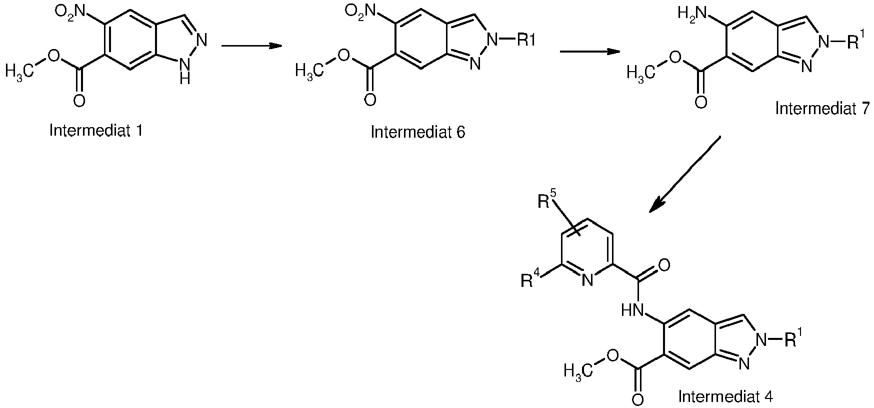 Syntheseschema 5

Die Substituenten R¹, R⁴, R⁵ haben die in der allgemeinen Formel (I) angegebenen Definitionen.

### Synthese der Beispielverbindungen

### Abkürzungen und Erläuterungen

| | |
|---|---|
| DMF | *N*,*N*-Dimethylformamid |
| DMSO | Dimethylsulfoxid |
| THF | Tetrahydrofuran |
| RT | Raumtemperatur |
| d. Th. | der Theorie |
| HPLC | Hochleistungsflüssigkeitschromatographie |
| h | Stunde(n) |
| HCOOH | Ameisensäure |
| MeCN | Acetonitril |
| min | Minute(n) |
| UPLC | Ultra-Hochleistungsflüssigchromatographie |
| DAD | Diodenarraydetektor |
| ELSD | Verdampfungslichtstreudetektor |
| ESI | Elektronenspray-Ionisation |
| SQD | Single Quadrupole Detektor |
| KPG | Kerngezogenes Präzisions-Glasgerät |
| NH₃ | Ammoniak |

Der Begriff *Kochsalzlösung* bedeutet eine gesättigte wässrige Natriumchloridlösung.

Die chemische Bezeichnung der Intermediate und Beispiele wurde unter Verwendung der Software von ACD / LABS (Batch Version 12.01.) erstellt.

### Methoden

In einigen Fällen wurden die erfindungsgemäßen Verbindungen sowie deren Vor- und/oder Zwischenstufen durch LC-MS analysiert:
Methode A1: UPLC (MeCN-HCOOH):
   Instrument: Waters Acquity UPLC-MS SQD 3001; Säule: Acquity UPLC BEH C18 1.7 50x2.1mm; Eluent A: Wasser + 0.1% Vol. Ameisensäure (99%), Eluent B: Acetonitril; Gradient: 0-1.6 min 1-99% B, 1.6-2.0 min 99% B; Fluss 0.8 ml/min; Temperatur: 60 °C.; Injektion: 2 µl; DAD scan: 210-400 nm; MS ESI+, ESI-, scan range 160-1000 m/z; ELSD.
Methode A2: UPLC (MeCN-NH₃):
   Instrument: Waters Acquity UPLC-MS SQD 3001; Säule: Acquity UPLC BEH C18 1.7 50x2.1mm; Eluent A: Wasser + 0.2% Vol. Ammoniak (32%), Eluent B: Acetonitril; Gradient: 0-1.6 min 1-99% B, 1.6-2.0 min 99% B; Fluss 0.8 ml/min; Temperatur: 60 °C; Injektion: 2 µl; DAD scan: 210-400 nm; MS ESI+, ESI-, scan range 160-1000 m/z; ELSD.
Methode A3: (LC-MS)
   Instrument: Agilent 1290 Infinity LC; Säule: Acquity UPLC BEH C18 1.7 50x2.1mm; Eluent A: Wasser + 0.05% Vol. Ameisensäure, Eluent B: Acetonitril + 0.05% Vol. Ameisensäure; Gradient: 0-1.7 min 2-90% B, 1.7-2.0 min 90% B; Fluss 1.2 ml/min; Temperatur: 60 °C; Injektion: 2 µl; DAD scan: 190-390 nm; MS: Agilent TOF 6230.
Methode A4: (LC-MS)
   Instrument: Waters Acquity; Säule: Kinetex (Phenomenex), 50x2 mm; Eluent A: Wasser + 0.05% Vol. Ameisensäure, Eluent B: Acetonitril + 0.05% Vol. Ameisensäure; Gradient: 0-1.9 min 1-99% B, 1.9-2.1 min 99% B; Fluss 1.5 ml/min; Temperatur: 60 °C; Injektion: 0.5 µl; DAD scan: 200 - 400 nm.

In einigen Fällen wurden die erfindungsgemäßen Verbindungen sowie deren Vor- und/oder Zwischenstufen mit folgenden beispielhaften präparativen HPLC-Methoden gereinigt:
Methode P1: System: Waters Autopurificationsystem: Pump 2545, Sample Manager 2767, CFO, DAD 2996, ELSD 2424, SQD; Säule: XBrigde C18 5µm 100x30 mm; Eluent: A: Wasser + 0.1% Vol. Ameisensäure, Eluent B: Acetonitril; Gradient: 0-8 min 10-100% B, 8-10 min 100% B; Flow: 50 mL/min; Temperatur: Raumtemperatur; Lösung: Max. 250 mg / max. 2.5 mL DMSO o. DMF; Injektion: 1 x 2.5 mL; Detection: DAD scan range 210-400 nm; MS ESI+, ESI-, scan range 160-1000 m/z.
Methode P2: System: Waters Autopurificationsystem: Pump 254, Sample Manager 2767, CFO, DAD 2996, ELSD 2424, SQD 3100; Säule: XBrigde C18 5µm 100x30 mm; Eluent: A: Wasser + 0.2% Vol. Ammoniak (32%), Eluent B: Methanol; Gradient: 0-8 min 30-70% B; Fluss: 50 mL/min; Temperatur: Raumtemperatur; Detektion: DAD scan range 210-400 nm; MS ESI+, ESI-, scan range 160-1000 m/z; ELSD.
Methode P3: System: Labomatic, Pump: HD-5000, Fraction Collector: LABOCOL Vario-4000, UV-Detektor: Knauer UVD 2.1S; Säule: XBrigde C18 5µm 100x30 mm; Eluent A: Wasser + 0.2% Vol. Ammoniak (25%), Eluent B: Acetonitril; Gradient: 0-1 min 15% B, 1-6,3 min 15-55% B, 6,3-6,4min 55-100% B, 6,4-7,4min 100% B; Flow: 60 mL/min; Temperatur: Raumtemperatur; Lösung:
   Max. 250 mg / 2mL DMSO; Injektion: 2 x 2mL; Detection: UV 218 nm; Software: SCPA PrepCon5.
Methode P4: System: Labomatic, Pump: HD-5000, Fraction Collector: LABOCOL Vario-4000, UV-Detektor: Knauer UVD 2.1S; Säule: Chromatorex RP C18 10µm 125x30 mm, Eluent: A: Wasser + 0.1% Vol. Ameisensäure, Eluent B: Acetonitril; Gradient: 0 - 15 min 65 - 100% B; Fluss: 60 mL/min; Temperatur: Raumtemperatur; Lösung: Max. 250 mg / 2mL DMSO; Injektion: 2 x 2mL; Detection: UV 254 nm; Software: SCPA PrepCon5.
Methode P5: System: Sepiatec: Prep SFC100, Säule: Chiralpak IA 5µm 250x20 mm; Eluent A: Kohlendioxid, Eluent B: Ethanol; Gradient: isokratisch 20% B; Fluss: 80 mL/min; Temperatur: 40°C; Lösung: Max. 250 mg / 2mL DMSO; Injektion: 5 x 0.4 mL Detektion: UV 254 nm.
Methode P6: System: Agilent: Prep 1200, 2xPrep Pump, DLA, MWD, Gilson: Liquid Handler 215; Säule: Chiralcel OJ-H 5µm 250x20 mm; Eluent A: Hexan, Eluent B: Ethanol; Gradient: isokratisch 30% B; Fluss: 25 mL/min; Temperatur: 25°C; Lösung: 187 mg / 8 mL Ethanol/Methanol; Injektion: 8 x 1.0 mL Detektion: UV 280 nm.
Methode P7: System: Labomatic, Pump: HD-5000, Fraction Collector: LABOCOL Vario-4000, UV-Detektor: Knauer UVD 2.1S; Säule: XBrigde C18 5µm 100x30 mm; Eluent A: Wasser + 0.1% Vol. Ameisensäure, Eluent B: Acetonitril; Gradient: 0-3min: 65%B isokratisch, 3-13min: 65-100% B; Fluss: 60ml/min; Temperatur: Raumtemperatur; Lösung: Max. 250 mg / 2mL DMSO; Injektion: 2 x 2mL; Detektion: UV 254 nm.
Methode P8: System: Agilent: Prep 1200, 2xPrep Pump, DLA, MWD, Gilson: Liquid Handler 215; Säule: Chiralpak IF 5µm 250x20 mm; Eluent A: Ethanol, Eluent B: Methanol; Gradient: isokratisch 50% B; Fluss: 25 mL/min; Temperatur: 25°C; Lösung: 600 mg / 7 mL N,N-Dimethylformamid; Injektion: 10 x 0.7 mL Detektion: UV 254 nm

In einigen Fällen erfolgte die Aufreinigung von Substanzgemischen durch Säulenchromatographie an Kieselgel.

Zur Herstellung einiger der erfindungsgemäßen Verbindungen sowie deren Vorstufen und/oder Zwischenstufen wurde eine säulenchromatographische Reinigung ("Flash-Chromatographie") an Kieselgel durchgeführt unter Verwendung von Geräten Isolera^{®} der Firma Biotage. Hierbei kamen Kartuschen der Firma Biotage wie zum Beispiel die Kartusche "SNAP Cartridge, KP_SIL" unterschiedlicher Größe sowie Kartuschen "Interchim Puriflash Silica HP 15UM flash column" der Firma Interchim unterschiedlicher Größe zum Einsatz.

### Ausgangsverbindungen

### Intermediat V2-1

### Methyl-6-(2-hydroxypropan-2-yl)pyridin-2-carboxylat

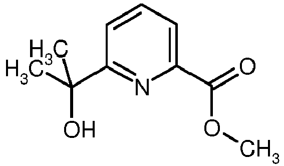

2.00 g (9.26 mmol) 2-(6-Bromopyridin-2-yl)propan-2-ol (CAS 638218-78-7) wurden in 20 ml Methanol und 20 ml DMSO gelöst. Anschließend wurden 250 mg 1,3-Bis(diphenylphoshino)-propan, 130 mg Palladium(II)acetat und 3 ml Triethylamin zugegeben. Das Reaktionsgemisch wurde bei Raumtemperatur dreimal mit Kohlenmonoxid gespült und unter 13 bar Kohlenmonoxidatmosphäre 30 min gerührt. Man entfernte die Kohlenmonoxidatmosphäre durch Anlegen eines Vakuums und rührte unter 14 bar Kohlenmonoxidatmosphäre bei 100°C 24 h. Man entspannte den Autoklaven, versetzte die Reaktionsmischung mit Wasser, extrahierte dreimal mit Ethylacetat, wusch mit gesättigter wässriger Natriumhydrogencarbonatlösung, Kochsalzlösung, filtrierte über einen wasserabweisenden Filter und engte ein. Man erhielt 1.60 g eines Rohproduktes.

UPLC-MS (Methode A1): Rₜ = 0.76 min (UV Detektor: TIC ), gefundene Masse 195.00.

### Intermediat V3-1

### Kalium-6-(2-hydroxypropan-2-yl)pyridin-2-carboxylat

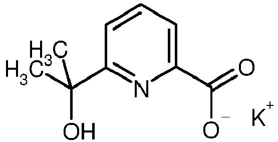

1.60 g des Rohproduktes Intermediat 0-1 wurden in 15 ml Methanol vorgelegt, man addierte 0.74 g Kaliumhydroxid und rührte 16.5 h bei 50°C. Nach Einengen erhielt man 2.1 g eines Rückstandes, welcher ohne weitere Reinigung eingesetzt wurde.

UPLC-MS (Methode A1): Rₜ = 0.47 min (UV Detektor: TIC), gefundene Masse 181.00.

### Intermediat 1-1

### Methyl-5-nitro-1H-indazol-6-carboxylat

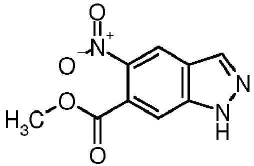

4.60 g (26.1 mmol) Methyl-1H-indazol-6-carboxylat (CAS Nr.: 170487-40-8) wurden in 120 ml Schwefelsäure (96%ig) gelöst und in einem Dreihalskolben mit KPG-Rührer, Tropftrichter und Innenthermometer auf -15°C gekühlt. Zu dieser Lösung wurde innerhalb von 15 min die vorher hergestellte und gekühlte Nitriersäure (10 ml Schwefelsäure 96%ig in 5 ml Salpetersäure 65%ig) zugetropft. Nach Beendigung des Zutropfens wurde 1 h nachgerührt (Innentemperatur bei -13°C). Die Reaktionsmischung wurde auf Eis gegeben, der Niederschlag abgesaugt, mit Wasser gewaschen und im Trockenschrank bei 50°C im Vakuum getrocknet. Es wurden 5.49 g der Titelverbindung erhalten.
UPLC-MS (Methode A2): Rₜ = 0.75 min
MS (ESIpos): m/z = 222(M+H)⁺

¹H NMR (400 MHz, DMSO-d6): δ [ppm] = 3.87 (s, 3 H), 7.96 (s, 1 H), 8.44 (s, 1 H), 8.70 (s, 1 H), 13.98 (br. s., 1 H).

### Intermediat 2-1

### Methyl-5-amino-1H-indazol-6-carboxylat

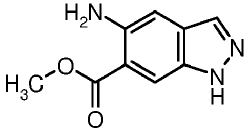

4.40 g (19.8 mmol) Methyl-5-nitro-1H-indazol-6-carboxylat (Intermediat 1-1) wurden in 236 ml Methanol gelöst und mit 1.06 g (0.99 mmol) Palladium auf Aktivkohle 3 h bei 25°C unter Normaldruck Wasserstoff hydriert. Das Reaktionsgemisch wurde über Celite filtriert, der Filter mit Methanol gewaschen und das Filtrat eingeengt. Es wurden 3.53 g der Titelverbindung erhalten.

¹H NMR (300 MHz, DMSO-d6): δ [ppm] = 3.85 (s, 3 H) 6.01 (s, 2 H) 6.98 (s, 1 H) 7.79 - 7.91 (m, 1 H) 7.99 (s, 1 H) 12.84 (br. s., 1 H).

### Intermediat 3-1

### Methyl-5-({[6-(trifluormethyl)pyridin-2-yl]carbonyl}amino)-1H-indazol-6-carboxylat

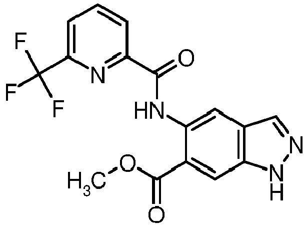

4.95 g (25.9 mmol) 6-(Trifluormethyl)pyridin-2-carbonsäure wurden in 45 ml THF vorgelegt. Man addierte 9.07 g (28.2 mmol) O-(Benzotriazol-1-yl)-N,N,N',N'-tetramethyluroniumtetrafluoroborat und 4.92 ml (28.2 mmol) N-Ethyl-N-isopropylpropan-2-amin und rührte 30 min bei 25°C. Anschließend wurden 4.50 g (23.5 mmol) Methyl-5-amino-1H-indazol-6-carboxylat (Intermediat 2-1) hinzugegeben und 24 h bei 25°C gerührt. Die Reaktionsmischung wurde über einen Membranfilter abgesaugt und der Feststoff mit THF und mit Wasser gewaschen und über Nacht im Trockenschrank getrocknet. Es wurden 7.60 g der Titelverbindung erhalten.
UPLC-MS (Methode A2): Rₜ = 1.16 min
MS (ESIpos): m/z = 365 (M+H)⁺

¹H NMR (400 MHz, DMSO-d6): δ [ppm] = 3.97 (s, 3 H), 8.13 - 8.27 (m, 2 H), 8.30 (s, 1 H), 8.33 - 8.45 (m, 1 H), 8.45 - 8.51 (m, 1 H), 9.15 (s, 1 H), 12.57 (s, 1 H), 13.44 (s, 1 H).

### Intermediat 3-2

### Methyl-5-({[6-(difluormethyl)pyridin-2-yl]carbonyl}amino)-1H-indazol-6-carboxylat

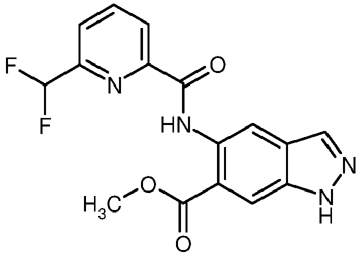

2.85 g (23.5 mmol) 6-(Difluormethyl)pyridin-2-carbonsäure wurden in 30 ml THF vorgelegt. Man addierte 6.05 g (18.8 mmol) O-(Benzotriazol-1-yl)-N,N,N',N'-tetramethyluroniumtetrafluoroborat und 3.3 ml N-Ethyl-N-isopropylpropan-2-amin und rührte 10 Minuten bei Raumtemperatur. Anschließend wurden 3.00 g (15.7 mmol) Methyl-5-amino-1H-indazol-6-carboxylat hinzugegeben und über Nacht bei Raumtemperatur gerührt. Das Reaktionsgemisch wurde mit Wasser versetzt, der Niederschlag wurde abgesaugt und mit Wasser und Dichlormethan mehrfach gewaschen. Man erhielt 1.53 g (27% d. Th.) der Titelverbindung. Man trennte die Phasen des Filtrates, engte die organische Phase ein, versetzte mit wenig Dichlormethan, suspendierte im Ultraschallbad und saugte den Niederschlag ab. Man erhielt weitere 1.03 g der Titelverbindung.

1H-NMR (erste Produktfraktion, 300MHz, DMSO-d6): δ [ppm]= 3.99 (s, 3H), 7.09 (t, 1H), 8.00 (d, 1H), 8.21 - 8.40 (m, 4H), 9.14 (s, 1H), 12.53 (s, 1H), 13.44 (s, 1H).

### Intermediat 3-3

### Methyl-5-({[6-(2-hydroxypropan-2-yl)pyridin-2-yl]carbonyl}amino)-1H-indazol-6-carboxylat

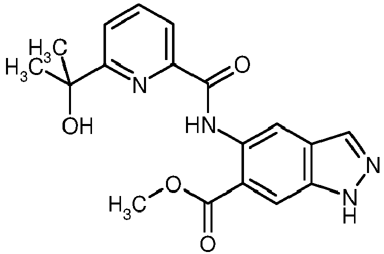

2.10 g Kalium-6-(2-hydroxypropan-2-yl)pyridin-2-carboxylat (Intermediat V3-1) wurden in 15 ml THF vorgelegt. Man addierte 3.69 g (11.5 mmol) O-(Benzotriazol-1-yl)-N,N,N',N'-tetramethyluroniumtetrafluoroborat und 2.00 ml N-Ethyl-N-isopropylpropan-2-amin und rührte 15 min bei Raumtemperatur. Anschließend wurden 1.83 g (9.58 mmol) Methyl-5-amino-1H-indazol-6-carboxylat (Intermediat 2-1) hinzugegeben und 19 h bei Raumtemperatur gerührt. Man versetzte mit Wasser und Ethylacetat, filtrierte den ungelösten Feststoff ab, trennte die Phasen des Filtrates, extrahierte die wässrige Phase zweimal mit Ethylacetat, wusch mit Kochsalzlösung, filtrierte über einen wasserabweisenden Filter, engte ein und reinigte säulenchromatographisch an Kieselgel (Hexan/Ethylacetat). Nach Entfernung der Lösungsmittel wurden 1.56 g der Titelverbindung als gelber Schaum erhalten.

UPLC-MS (Methode A1): Rₜ = 1.00 min (UV Detektor: TIC Smooth), gefundene Masse 354.00. 1H-NMR (500MHz,DMSO-d6): δ [ppm] = 1.63 (s, 6H), 3.97 (s, 3H), 5.37(s ,1H), 7.90 - 7.95 (m, 1H), 8.03-8.07 (m, 2H), 8.23(s, 1H),8.29 (s, 1H), 9.19 (s, 1H), 12.79 (s, 1H), 13.41 (br.s., 1H).

### Intermediat 4-1

### Methyl-2-(oxetan-3-ylmethyl)-5-({[6-(trifluormethyl)pyridin-2-yl]carbonyl}amino)-2H-indazol-6-carboxylat

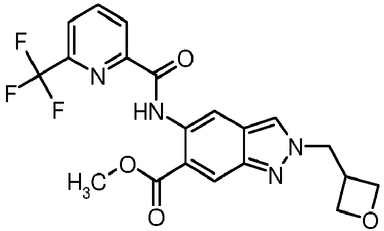

1.00 g (2.66 mmol) Methyl-5-({[6-(trifluormethyl)pyridin-2-yl]carbonyl}amino)-1H-indazol-6-carboxylat (Intermediat 3-1) wurden in 10 ml DMF gelöst und nach Zugabe von 1.10 g (7.99 mmol) Kaliumcarbonat und 221 mg (1.33 mmol) Kaliumiodid 30 min bei 25°C gerührt. Es wurden 603 mg (3.99 mmol) 3-Brommethyl-oxetan zugegeben und 24 h bei 25°C gerührt. Die Reaktionsmischung wurde zwischen Wasser und Ethylacetat verteilt. Es wurde zweimal mit Ethylacetat extrahiert, die vereinigten organischen Phasen über einen wasserabweisenden Filter filtriert und eingeengt. Der Rückstand wurde säulenchromatographisch an Kieselgel (Hexan/Ethylacetat) gereinigt. Es wurden 260 mg der Titelverbindung erhalten.
UPLC-MS (Methode A2): Rₜ = 1.24 min
MS (ESIpos): m/z = 435(M+H)⁺

¹H NMR (400 MHz, DMSO-d6): δ [ppm] = 3.49 - 3.64 (m, 1 H), 3.95 (s, 3 H), 4.49 (t, 2 H), 4.68 (dd, 2 H), 4.81 (d, 2 H), 8.20 (dd, 1 H), 8.35 - 8.41 (m, 1 H), 8.43 - 8.49 (m, 2 H), 8.55 - 8.58 (m, 1 H), 9.06 (s, 1 H), 12.53 (s, 1 H).

### Intermediat 4-2

### Methyl-2-(2-methoxyethyl)-5-({[6-(trifluormethyl)pyridin-2-yl]carbonyl}amino)-2H-indazol-6-carboxylat

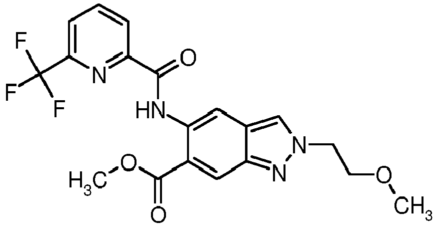

1.00 g (2.75 mmol) Methyl-5-({[6-(trifluormethyl)pyridin-2-yl]carbonyl}amino)-1H-indazol-6-carboxylat (Intermediat 3-1) wurden in 5 ml DMF gelöst und unter Rühren mit 387 µl (4.12 mmol) 2-Bromethyl-methylether, 1.14 g (8.23 mmol) Kaliumcarbonat und 228 mg (1.37 mmol) Kaliumiodid versetzt. Die Reaktionsmischung wurde 24 h bei 25°C gerührt, mit Wasser verdünnt und zweimal mit Ethylacetat extrahiert. Die vereinigten, organischen Phasen wurden über einen wasserabweisenden Filter filtriert und eingeengt. Der Rückstand wurde säulenchromatographisch an Kieselgel (Hexan/Ethylacetat) gereinigt. Es wurden 12 mg der Titelverbindung erhalten.
UPLC-MS (Methode A1): Rₜ = 1.24 min
MS (ESIpos): m/z = 423 (M+H)⁺

¹H NMR (400 MHz, DMSO-d6): δ [ppm] = 3.24 (s, 3 H), 3.86 (t, 2 H), 3.96 (s, 3 H), 4.65 (t, 2 H), 8.21 (dd, 1 H), 8.35 - 8.42 (m, 1 H), 8.43 - 8.51 (m, 2 H), 8.52 (d, 1 H), 9.06 (s, 1 H), 12.53 (s, 1 H).

### Intermediat 4-3

### Methyl-2-(3-methoxypropyl)-5-({[6-(trifluormethyl)pyridin-2-yl]carbonyl}amino)-2H-indazol-6-carboxylat

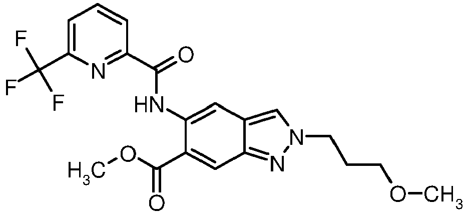

1.00 g (2.75 mmol) Methyl-5-({[6-(trifluormethyl)pyridin-2-yl]carbonyl}amino)-1H-indazol-6-carboxylat (Intermediat 3-1) wurden in 5 ml DMF gelöst und unter Rühren mit 460 µl (4.12 mmol) 1-Brom-3-methoxypropan, 1.14 g (8.23 mmol) Kaliumcarbonat und 228 mg (1.37 mmol) Kaliumiodid versetzt. Die Reaktionsmischung wurde 72 h bei 25°C gerührt, mit Wasser verdünnt und zweimal mit Ethylacetat extrahiert. Die vereinigten organischen Phasen wurden über einen wasserabweisenden Filter filtriert und eingeengt. Der Rückstand wurde säulenchromatographisch an Kieselgel (Hexan/Ethylacetat) gereinigt. Es wurden 28 mg der Titelverbindung erhalten.
UPLC-MS (Methode A1): Rₜ = 1.29 min
MS (ESIpos): m/z = 437 (M+H)⁺

¹H NMR (400 MHz, DMSO-d6): δ [ppm] = 2.17 (quin, 2 H), 3.24 (s, 3 H), 3.33 - 3.36 (m, 2 H), 3.96 (s, 3 H), 4.53 (t, 2 H), 8.21 (dd, 1 H), 8.35 - 8.42 (m, 1 H), 8.45 - 8.49 (m, 2 H), 8.54 (d, 1 H), 9.06 (s, 1 H), 12.54 (s, 1 H).

### Intermediat 4-4

### Methyl-2-(3-hydroxy-3-methylbutyl)-5-({[6-(tritluormethyl)pyridin-2-yl]carbonyl}amino)-2H-indazol-6-carboxylat

### Herstellungsmethode 1

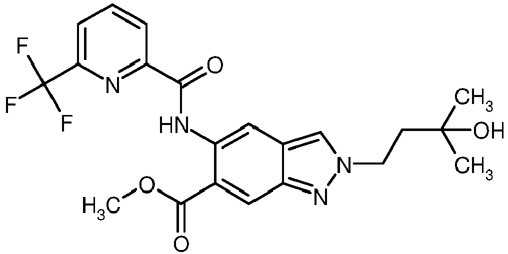

930 mg (2.55 mmol) Methyl-5-({[6-(trifluormethyl)pyridin-2-yl]carbonyl}amino)-1H-indazol-6-carboxylat (Intermediat 3-1), 1.06 g Kaliumcarbonat und 212 mg Kaliumiodid wurden in 9 ml DMF vorgelegt und die Mischung wurde 15 min gerührt. Danach addierte man 0.62 ml 4-Brom-2-methylbutan-2-ol und rührte bei 60°C für 16 h. Man versetzte mit Wasser, extrahierte zweimal mit Ethylacetat und wusch dreimal mit gesättigter Natriumchloridlösung, filtrierte und engte ein. Nach säulenchromatographischer Reinigung an Kieselgel (Hexan/Ethylacetat) wurden 424 mg der Titelverbindung erhalten.
UPLC-MS (Methode A2): Rₜ = 1.21 min (UV Detector: TIC), gefundene Masse 450.00.

¹H-NMR (400MHz, DMSO-d₆): δ [ppm]= 1.16 (s, 6 H) 2.02 - 2.11 (m, 2 H) 3.96 (s, 3 H) 4.51 - 4.60 (m, 3 H) 8.20 (dd, *J*=7.83, 1.01 Hz, 1 H) 8.39 (s, 1 H) 8.45 (s, 2 H) 8.55 (d, *J*=0.76 Hz, 1 H) 9.05 (s, 1 H) 12.52 (s, 1 H)

### Herstellungsmethode 2

1.95 g (7.03 mmol) Methyl-5-amino-2-(3-hydroxy-3-methylbutyl)-2H-indazol-6-carboxylat (Intermediat 7-1) wurden in 30 ml THF vorgelegt. Man addierte 1.48 g (7.73 mmol) 6-(Trifluormethyl)pyridin-2-carbonsäure, 2.71 g (8.44 mmol) O-(Benzotriazol-1-yl)-N,N,N',N'-tetramethyluroniumtetrafluoroborat und 1.47 ml (8.44 mmol) N-Ethyl-N-isopropylpropan-2-amin und rührte 20.5 h bei 25°C. Man versetzte mit Wasser, extrahierte dreimal mit Ethylacetat, wusch mit Kochsalzlösung, filtrierte über einen wasserabweisenden Filter und engte ein. Der Rückstand wurde säulenchromatographisch an Kieselgel (Hexan/Ethylacetat-Gradient) getrennt. Es wurden 2.79 g der Titelverbindung erhalten.
UPLC-MS (Methode A1): Rₜ = 1.23 min (UV Detektor: TIC), gefundene Masse 450.00

### Intermediat 4-5

### Methyl-2-(2-{[tert-butyl(dimethyl)silyl]oxy}ethyl)-5-({[6-(trifluormethyl)pyridin-2-yl]carbonyl}amino)-2H-indazol-6-carboxylat

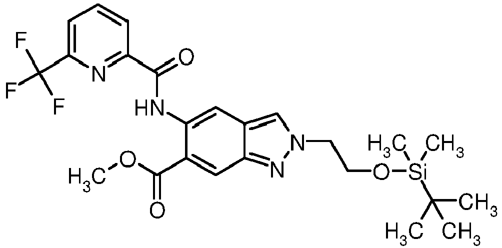

1.00 g (2.66 mmol, 97%) Methyl-5-({[6-(trifluormethyl)pyridin-2-yl]carbonyl}amino)-1H-indazol-6-carboxylat (Intermediat 3-1) wurden in 50 ml DMF vorgelegt, unter Rühren wurde mit 1.10 g (7.99 mmol) Kaliumcarbonat und 221 mg (1.33 mmol) Kaliumiodid versetzt und 30 min bei 25°C gerührt. Anschließend wurden 857 µl (3.99 mmol) (2-Bromethoxy)(tert-butyl)dimethylsilan zugegeben und 24 h bei 25°C gerührt. Die Reaktionsmischung wurde mit Wasser verdünnt und mit Ethylacetat extrahiert. Die vereinigten, organischen Phasen wurden über einen wasserabweisenden Filter filtriert und eingeengt. Der Rückstand wurde säulenchromatographisch an Kieselgel (Hexan/Ethylacetat) gereinigt. Es wurden 400 mg der Titelverbindung erhalten.
UPLC-MS (Methode A1): Rₜ = 1.58 min
MS (ESIpos): m/z = 523(M+H)⁺

¹H NMR (300 MHz, DMSO-d6): δ [ppm] = -0.18 - -0.13 (m, 6 H), 0.74 (s, 9 H), 3.96 (s, 3 H), 4.08 (t, 2 H), 4.57 (t, 2 H), 8.15 - 8.25 (m, 1 H), 8.32 - 8.43 (m, 1 H), 8.43 - 8.52 (m, 3 H), 9.07 (s, 1 H), 12.53 (s, 1 H).

### Intermediat 4-6

### Methyl-2-(3-{[tert-butyl(dimethyl)silyl]oxy}propyl)-5-({[6-(trifluormethyl)pyridin-2-yl]carbonyl}amino)-2H-indazol-6-carboxylat

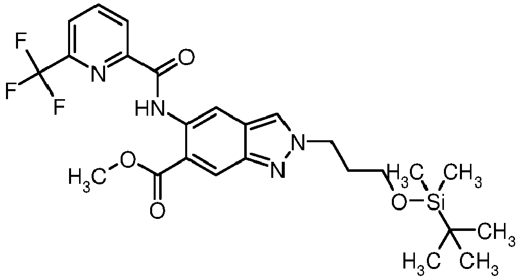

Analog zu Intermediat 4-5 wurden 1.00 g (2.75 mmol) Methyl-5-({[6-(trifluormethyl)pyridin-2-yl]carbonyl}amino)-1H-indazol-6-carboxylat (Intermediat 3-1) in 10 ml DMF gelöst, unter Rühren mit 1.14 g (8.24 mmol) Kaliumcarbonat und 228 mg (1.37 mmol) Kaliumiodid versetzt und 30 min bei 25°C gerührt. Anschließend wurden 1.04 g (4.12 mmol) (3-Brompropoxy)(tert-butyl)dimethylsilan zugegeben und 24 h bei 25°C gerührt. Die Reaktionsmischung wurde filtriert und der Filterkuchen mit Ethylacetat gewaschen. Es wurde zwischen Wasser und Ethylacetat verteilt und zweimal mit Ethylacetat extrahiert. Die vereinigten, organischen Phasen wurden über einen wasserabweisenden Filter filtriert und eingeengt. Nach Reinigung des Rückstandes durch präparative HPLC wurden 428 mg der Titelverbindung erhalten.
UPLC-MS (Methode A1): Rₜ = 1.63 min
MS (ESIpos): m/z = 537(M+H)⁺

¹H NMR (400 MHz, DMSO-d6): δ [ppm] = -0.02 - 0.06 (m, 6 H), 0.87 (s, 9 H), 2.14 (quin, 2 H), 3.62 (t, 2 H), 3.96 (s, 3 H), 4.54 (t, 2 H), 8.20 (d, 1 H), 8.35 - 8.42 (m, 1 H), 8.43 - 8.48 (m, 3 H), 8.49 - 8.53 (m, 1 H), 9.06 (s, 1 H).

### Intermediat 4-7

### Methyl-5-({[6-(2-hydroxypropan-2-yl)pyridin-2-yl]carbonyl}amino)-2-(4,4,4-trifluorbutyl)-2H-indazol-6-carboxylat

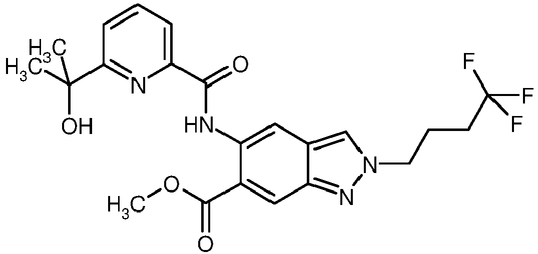

300 mg (0.80 mmol) Methyl-5-({[6-(2-hydroxypropan-2-yl)pyridin-2-yl]carbonyl}amino)-1H-indazol-6-carboxylat (Intermediat 3-3) wurden in 4.5 ml DMF vorgelegt. Man addierte 287 mg (1.21 mmol) 1,1,1-Trifluor-4-iodbutan und 333 mg Kaliumcarbonat und rührte bei 100°C 23 h. Man addierte Wasser und extrahierte dreimal mit Ethylacetat. Man engte ein und reinigte durch präparative HPLC. Man erhielt 72 mg der Titelverbindung.
UPLC-MS (Methode A1): Rₜ = 1.26 min (UV Detektor: TIC), gefundene Masse 464.17.

### Intermediat 4-8

### Methyl-5-{[(5-fluor-6-methylpyridin-2-yl)carbonyl]amino}-2-(3-hydroxy-3-methylbutyl)-2H-indazol-6-carboxylat

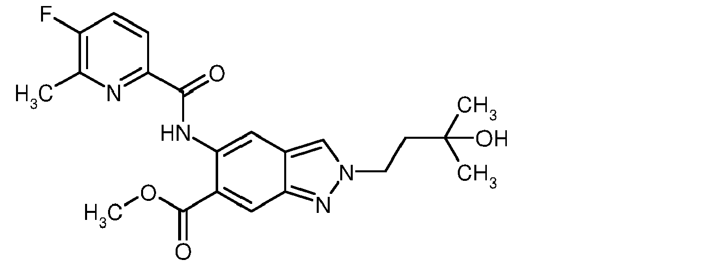

195 mg (0.46 mmol) Methyl-5-amino-2-(3-hydroxy-3-methylbutyl)-2H-indazol-6-carboxylat (Intermediat 7-1) wurden mit 78 mg (0.50 mmol) 5-Fluor-6-methylpyridin-2-carbonsäure analog zu Intermediat 4-4 (Herstellungsmethode 2) in 19.5 h umgesetzt. Es wurden 228 mg eines Rohproduktes nach analoger wässriger Aufarbeitung erhalten.
UPLC-MS (Methode A1): Rₜ = 1.20 min (UV Detektor: TIC), gefundene Masse 414.00.

### Intermediat 4-9

### Methyl-2-(3-hydroxy-3-methylbutyl)-5-{[(6-methylpyridin-2-yl)carbonyl]amino}-2H-indazol-6-carboxylat

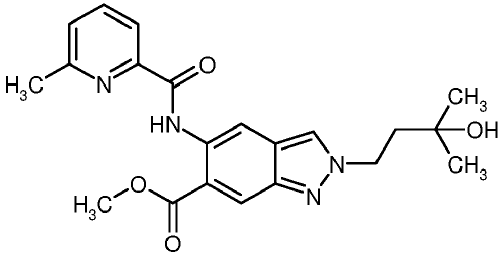

195 mg (0.45 mmol) Methyl-5-amino-2-(3-hydroxy-3-methylbutyl)-2H-indazol-6-carboxylat (Intermediat 7-1) wurden mit 70 mg (0.50 mmol) 6-Methylpyridin-2-carbonsäure analog zu Herstellung von Intermediat 4-4 (Herstellungsmethode 2) in 19.5 h umgesetzt. Es wurden 278 mg der Titelverbindung als Rohprodukt nach analoger wässriger Aufarbeitung erhalten.
UPLC-MS (Methode A1): Rₜ = 1.14 min (UV Detektor: TIC), gefundene Masse 396.00.

### Intermediat 4-10

### Methyl-2-[3-(2,2,2-trifluorethoxy)propyl]-5-({[6-(trifluormethyl)pyridin-2-yl]carbonyl}amino)-2H-indazol-6-carboxylat

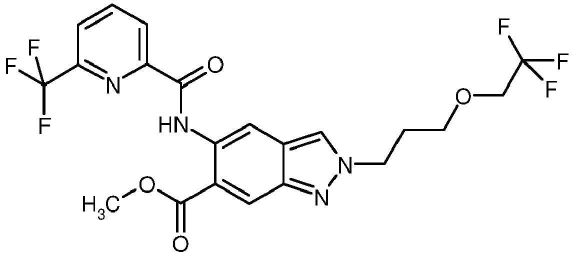

Eine Mischung aus 250 mg (0.58 mmol) Methyl-5-({[6-(trifluormethyl)pyridin-2-yl]carbonyl}amino)-1H-indazol-6-carboxylat (Intermediat 3-1), 193 mg (0.88 mmol) 3-Brompropyl-2,2,2-trifluorethylether, 242 mg Kaliumcarbonat und 145 mg Kaliumiodid in 3 ml DMF 20 h bei 100°C gerührt. Man versetzte mit Wasser, extrahierte mit Ethylacetat, wusch mit Kochsalzlösung und engte ein. Nach Reinigung durch präparative HPLC erhielt man 52 mg der Titelverbindung.
UPLC-MS (Methode A1): Rₜ = 1.39 min (UV Detektor: TIC), gefundene Masse 504.12.

### Intermediat 4-11

### Methyl-5-({[6-(difluormethyl)pyridin-2-yl]carbonyl}amino)-2-(3-hydroxy-3-methylbutyl)-2H-indazol-6-carboxylat

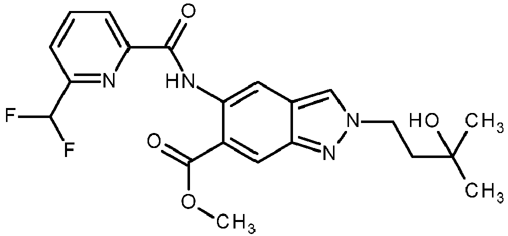

2.00 g Methyl-5-amino-2-(3-hydroxy-3-methylbutyl)-2H-indazol-6-carboxylat (Intermediat 7-1) wurden in 40 ml THF vorgelegt. Es wurden 1.50 g 6-(Difluormethyl)pyridin-2-carbonsäure, 2.78 g *O*-(Benzotriazol-1-yl)-*N*,*N*,*N'*,*N'*-tetramethyluroniumtetrafluoroborat (TBTU, CAS-Nummer 125700-67-6) und 1.5 ml N-Ethyl-N-isopropylpropan-2-amin zugegeben und die Mischung wurde 24 h bei RT gerührt. Man versetzte mit Wasser, extrahierte dreimal mit Ethylacetat, wusch die vereinigten organischen Phasen mit Kochsalzlösung und filtrierte durch einen wasserabweisenden Filter. Man engte ein und reinigte den Rückstand säulenchromatographisch an Kieselgel (Hexan/Ethylacetat). Man erhielt 3.05 g der Titelverbindung als gelben Feststoff.
UPLC-MS (Methode A1): Rt = 1.15 min (UV Detektor: TIC), gefundene Masse 432.00.

¹H-NMR (400MHz, DMSO-d₆): δ [ppm]= 1.17 (s, 6H), 2.04 - 2.11 (m, 2H), 3.99 (s, 3H), 4.52 - 4.60 (m, 3H), 7.10 (t, 1H), 8.00 (dd, 1H), 8.28 - 8.38 (m, 2H), 8.44 - 8.47 (m, 1H), 8.56 (d, 1H), 9.05 (s, 1H), 12.49 (s, 1H).

### Intermediat 5-1

### N-[6-(2-Hydroxypropan-2-yl)-1H-indazol-5-yl]-6-(trifluormethyl)pyridin-2-carboxamid

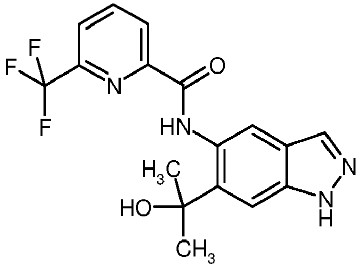

Zu einer im Eiswasser-Kältebad abgekühlten Lösung aus 1.50 g (4.12 mmol) Methyl-5-({[6-(trifluormethyl)pyridin-2-yl]carbonyl}amino)-1H-indazol-6-carboxylat (Intermediat 3-1) in 20 ml THF wurden vorsichtig 6.9 ml (5 Äquivalente) einer 3M Methylmagnesiumbromid-Lösung in Diethylether gegeben. Man rührte 1 h mit Eiswasser-Kältebadkühlung und 19.5 h bei Raumtemperatur. Man gab nochmals 2 Äquivalente der Methylmagnesiumbromid-Lösung zu und ließ weitere 24 h bei Raumtemperatur rühren. Man versetzte mit gesättigter wässriger Ammoniumchloridlösung, rührte die Mischung um und extrahierte dreimal mit Ethylacetat. Die vereinigten organischen Phasen wurden mit Kochsalzlösung gewaschen, über einen wasserabweisenden Filter filtriert und eingeengt. Der Rückstand wurde säulenchromatographisch an Kieselgel (Hexan/Ethylacetat) gereinigt. Es wurden 763 mg der Titelverbindung erhalten.

¹H-NMR (400MHz, DMSO-d₆): δ [ppm]= 1.63 (s, 6H), 5.99 (s, 1H), 7.49 (s, 1H), 8.06 (s, 1H), 8.14 - 8.19 (m, 1H), 8.37 (t, 1H), 8.46 (d, 1H), 8.78 (s, 1H), 12.32 (s, 1H), 12.97 (s, 1H).

### Intermediat 5-2

### 6-(Difluormethyl)-N-[6-(2-hydroxypropan-2-yl)-1H-indazol-5-yl]pyridin-2-carboxamid

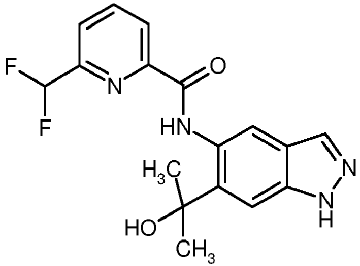

Analog zur Herstellung von Intermediat 5-1 wurden 2.40 g (6.93 mmol) Methyl-5-({[6-(difluormethyl)pyridin-2-yl]carbonyl}amino)-1H-indazol-6-carboxylat (Intermediat 3-2) in 10 ml THF mit drei Portionen 3M Methylmagnesiumbromidlösung in Diethylether (6.9 ml, dann 45 min Rühren bei Raumtemperatur; 11.6 ml, dann 2 h Rühren bei Raumtemperatur; 6.9 ml, dann 2 h Rühren bei Raumtemperatur) umgesetzt. Nach Aufarbeitung wie bei Intermediat 5-1 wurden 2.39 g eines Rohproduktes erhalten, welches ohne weitere Reinigung weiter eingesetzt wurde.

### Intermediat 6-1

### Methyl-2-(3-hydroxy-3-methylbutyl)-5-nitro-2H-indazol-6-carboxylat

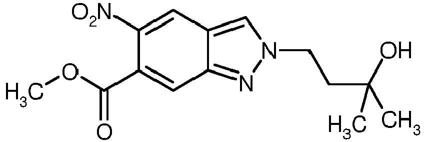

5.00 g (22.6 mmol) Methyl-5-nitro-1H-indazol-6-carboxylat (Intermediat 1-1) wurden in 40 ml DMF vorgelegt. Man addierte 5.65 g (33.9 mmol) 4-Brom-2-methylbutan-2-ol, 9.37 g (67.8 mmol) Kaliumcarbonat und 5.63 g (33.9 mmol) Kaliumiodid und die Mischung wurde 20 h bei 100°C gerührt. Man versetzte mit Wasser, extrahierte dreimal mit Ethylacetat, wusch mit Kochsalzlösung, filtrierte über einen wasserabweisenden Filter und engte ein. Der Rückstand wurde säulchenchromatographisch an Kieselgel (Hexan/Ethylacetat) gereinigt. Der erhaltene Feststoff wurde aus Diethylether ausgerührt, abgesaugt, mit Diethylether nachgewaschen und getrocknet. Es wurden 2.49 g der Titelverbindung erhalten.

UPLC-MS (Methode A1): Rₜ = 0.93 min (UV Detektor: TIC), gefundene Masse 307.00.

¹H-NMR (400MHz, DMSO-d₆): δ [ppm]= 1.15 (s, 6H), 2.02 - 2.11 (m, 2H), 3.84 (s, 3H), 4.54 (s, 1H), 4.58 - 4.65 (m, 2H), 8.05 (s, 1H), 8.69 (s, 1H), 8.86 (s, 1H).

### Intermediat 7-1

### Methyl-5-amino-2-(3-hydroxy-3-methylbutyl)-2H-indazol-6-carboxylat

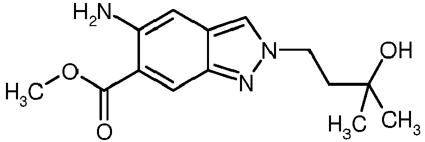

Man addierte 4.53 g Eisen und 217 mg Ammoniumchlorid zu 2.49 g (8.10 mmol) Methyl-2-(3-hydroxy-3-methylbutyl)-5-nitro-2H-indazol-6-carboxylat (Intermediat 6-1) in 30 ml Ethanol und 10 ml Wasser und rührte die Mischung 21.5 h bei 90°C. Man filtrierte über Celite, wusch dreimal mit Ethanol nach, engte das Filtrat ein und versetzte den Rückstand mit Wasser. Man extrahierte dreimal mit Ethylacetat (zur Verbesserung der Phasentrennung wurde mit Kochsalzlösung versetzt). Die vereinigten organischen Phasen wurden mit Kochsalzlösung gewaschen, über einen wasserabweisenden Filter filtriert und eingeengt. Es wurden 1.95 g (85% der Theorie) der Titelverbindung erhalten.

UPLC-MS (Methode A1): Rₜ = 0.67 min (UV Detektor: TIC), gefundene Masse 277.00.

¹H-NMR (400MHz, DMSO-d₆): δ [ppm]= 1.14 (s, 6H), 1.96 - 2.08 (m, 2H), 3.85 (s, 3H), 4.39 - 4.51 (m, 3H), 5.81 (s, 2H), 6.80 (s, 1H), 8.05 (s, 1H), 8.18 (s, 1H).

### Ausführungsbeispiele

### Beispiel 1

### N-[6-(2-Hydroxypropan-2-yl)-2-(2-methoxyethyl)-2H-indazol-5-yl]-6-(trifluormethyl)pyridin-2-carboxamid

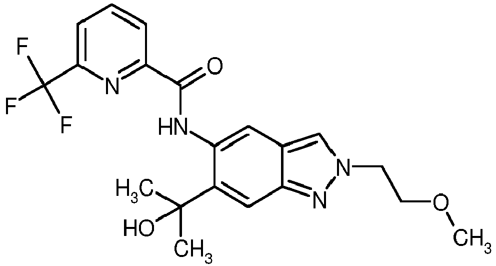

75 mg (0.18 mmol) Methyl-2-(2-methoxyethyl)-5-({[6-(trifluormethyl)pyridin-2-yl]carbonyl}-amino)-2H-indazol-6-carboxylat (Intermediat 4-2) wurden in 500 µl THF gelöst und mit 887 µl (0.89 mmol) einer 1 M Methylmagnesiumbromidlösung in THF versetzt. Die Reaktionsmischung wurde 60 min bei 25°C gerührt. Anschließend wurde vorsichtig 1 ml einer gesättigten, wässrigen Ammoniumchloridlösung hinzugegeben und filtriert. Die wässrige Phase wurde zweimal mit Ethylacetat extrahiert, die organischen Phasen vereint, über einen wasserabweisenden Filter filtriert und eingeengt. Der Rückstand wurde in 3 ml DMSO gelöst und durch präparative HPLC gereinigt. Die produkthaltigen Fraktionen wurden gefriergetrocknet. Es wurden 20 mg der Titelverbindung erhalten.
UPLC-MS (Methode A1): Rₜ = 1.08 min
MS (ESIpos): m/z = 423 (M+H)⁺

¹H NMR (300 MHz, DMSO-d6): δ [ppm] = 1.62 (s, 6 H), 3.22 (s, 3 H), 3.82 (t, 2 H), 4.55 (t, 2 H), 5.96 (s, 1 H), 7.57 (s, 1 H), 8.16 (d1 H), 8.29 - 8.42 (m, 2 H), 8.42 - 8.50 (m, 1 H), 8.71 (s, 1 H), 12.36 (s, 1 H)

### Beispiel 2

### N-[6-(Hydroxymethyl)-2-(2-methoxyethyl)-2H-indazol-5-yl]-6-(trifluormethyl)pyridin-2-carboxamid

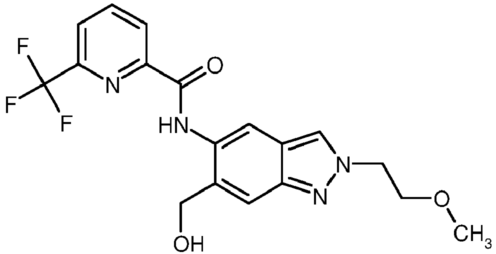

13 mg (0.36 mmol) Lithiumaluminiumhydrid wurden in 1 ml THF suspendiert und auf 0°C gekühlt. 75 mg (0.17 mmol) Methyl-2-(2-methoxyethyl)-5-({[6-(trifluormethyl)pyridin-2-yl]-carbonyl}amino)-2H-indazol-6-carboxylat (Intermediat 4-2) gelöst in 500 µl THF wurden zugetropft und 60 min bei 25°C gerührt. Man verdünnte mit Wasser, extrahierte zweimal mit Ethylacetat und die vereinigten, organischen Phasen wurden mit Kochsalzlösung gewaschen, über einen wasserabweisenden Filter filtriert, eingeengt und im Vakuum getrocknet. Man erhielt 13 mg der Titelverbindung.
UPLC-MS (Methode A2): Rₜ = 0.99 min
MS (ESIpos): m/z = 394 (M+H)⁺

¹H NMR (400 MHz, DMSO-d6): δ [ppm] = 3.23 (s, 3 H), 3.83 (t, 2 H), 4.56 (t, 2 H), 4.69 (d, 2 H), 5.77 (t, 1 H), 7.57 (s, 1 H), 8.19 (d, 1 H), 8.33 - 8.41 (m, 2 H), 8.43 - 8.47 (m, 1 H), 8.51 (s, 1 H), 11.20 (s, 1 H)

### Beispiel 3

### N-[6-(2-Hydroxypropan-2-yl)-2-(3-methoxypropyl)-2H-indazol-5-yl]-6-(trifluormethyl)pyridin-2-carboxamid

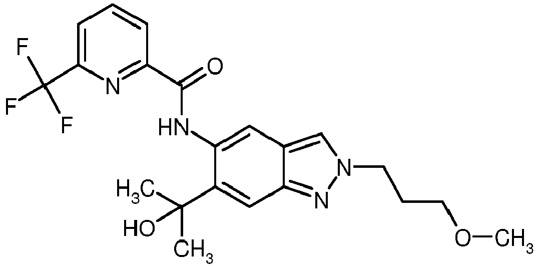

75 mg (0.17 mmol) Methyl-2-(3-methoxypropyl)-5-({[6-(trifluormethyl)pyridin-2-yl]carbonyl}-amino)-2H-indazol-6-carboxylat (Intermediat 4-3) wurden in 500 µl THF gelöst und mit 859 µl (0.86 mmol) einer 1 M Methylmagnesiumbromidlösung in THF versetzt. Die Reaktionsmischung wurde 60 min bei 25°C gerührt. Anschließend wurde vorsichtig 1 ml einer gesättigten Ammoniumchloridlösung hinzugegeben und filtriert. Die wässrige Phase wurde zweimal mit Ethylacetat extrahiert, die organischen Phasen vereint, über einen wasserabweisenden Filter filtriert und eingeengt. Der Rückstand wurde in 3 ml DMSO gelöst und durch präparative HPLC gereinigt. Die produkthaltigen Fraktionen wurden gefriergetrocknet. Es wurden 25 mg der Titelverbindung erhalten.
UPLC-MS (Methode A1): Rₜ = 1.13 min
MS (ESIpos): m/z = 437 (M+H)⁺

¹H NMR (400 MHz, DMSO-d6): δ [ppm] = 1.62 (s, 6 H), 2.14 (quin, 2 H), 3.23 (s, 3 H), 3.26 - 3.32 (m, 2 H), 4.44 (t, 2 H), 5.95 (s, 1 H), 7.58 (s, 1 H), 8.16 (d, 1 H), 8.31 - 8.40 (m, 2 H), 8.43 - 8.48 (m, 1 H), 8.72 (s, 1 H), 12.36 (s, 1 H).

### Beispiel 4

### N-[6-(Hydroxymethyl)-2-(3-methoxypropyl)-2H-indazol-5-yl]-6-(trifluormethyl)pyridin-2-carboxamid

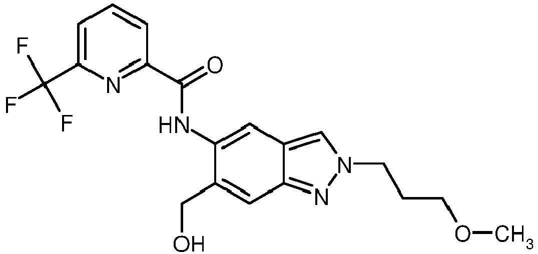

13 mg Lithiumaluminiumhydrid wurden in THF suspendiert und die Mischung auf 0°C abgekühlt. Man tropfte 75 mg (0.17 mmol) Methyl-2-(3-methoxypropyl)-5-({[6-(trifluormethyl)pyridin-2-yl]carbonyl}amino)-2H-indazol-6-carboxylat (Intermediat 4-3) in THF zu und ließ innerhalb von 30 min auf Raumtemperatur kommen. Man verdünnte mit Wasser, filtrierte, wusch den Rückstand mit Ethylacetat und extrahierte das Filtrat mit Ethylacetat. Die vereinigten Ethylacetatphasen wurden mit Kochsalzlösung gewaschen, über einen wasserabweisenden Filter filtriert und eingeengt. Man reinigte den Rückstand durch präparative HPLC.

¹H NMR (300 MHz, DMSO-d₆): δ [ppm] = 2.14 (quin, 2 H), 3.23 (s, 3 H), 3.29 (t, 2 H), 4.45 (t, 2 H), 4.68 (d, 2 H), 5.77 (t, 1 H), 7.58 (s, 1 H), 8.18 (d, 1 H), 8.32 - 8.48 (m, 3 H), 8.51 (s, 1 H), 11.21 (s, 1 H).

### Beispiel 5

### N-[2-(2-Hydroxyethyl)-6-(2-hydroxypropan-2-yl)-2H-indazol-5-yl]-6-(trifluormethyl)pyridin-2-carboxamid

### Stufe A:

Herstellung von N-[2-(2-{[tert-Butyl(dimethyl)silyl]oxy}ethyl)-6-(2-hydroxypropan-2-yl)-2H-indazol-5-yl]-6-(trifluormethyl)pyridin-2-carboxamid

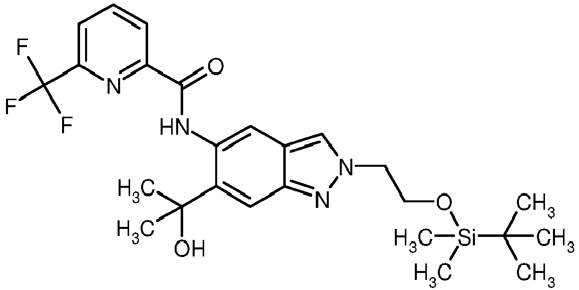

100 mg (0.19 mmol) Methyl-2-(2-{[tert-butyl(dimethyl)silyl]oxy}ethyl)-5-({[6-(trifluormethyl)pyridin-2-yl]carbonyl}amino)-2H-indazol-6-carboxylat (Intermediat 4-5) wurden in 1 ml THF gelöst und mit 669 µl (0.67 mmol) einer 1 M Methylmagnesiumbromidlösung in THF versetzt. Die Reaktionsmischung wurde 60 min bei 25°C gerührt. Es wurden nochmals 287 µl (0.29 mmol) einer 1 M Methylmagnesiumbromidlösung in THF hinzugegeben und 3 h bei 25°C gerührt. Anschließend wurden vorsichtig 20 ml einer gesättigten Ammoniumchloridlösung hinzugegeben und filtriert. Die wässrige Phase wurde zweimal mit Ethylacetat extrahiert, die organischen Phasen vereint, über Magnesiumsulfat getrocknet, filtriert, eingeengt und im Vakuum getrocknet. Man erhielt 50 mg N-[2-(2-{[tert-Butyl(dimethyl)silyl]oxy}ethyl)-6-(2-hydroxypropan-2-yl)-2H-indazol-5-yl]-6-(trifluormethyl)pyridin-2-carboxamid.
UPLC-MS (Methode A2): Rₜ = 1.51 min
MS (ESIpos): m/z = 523(M+H)⁺

¹H NMR (300 MHz, DMSO-d6): δ [ppm] = -0.17 - -0.09 (m, 6 H), 0.78 (s, 9 H), 1.62 (s, 6 H), 4.04 (t, 2 H), 4.47 (t, 2 H), 5.98 (s, 1 H), 7.57 (s, 1 H), 8.16 (d, 1 H), 8.29 (s, 1 H), 8.37 (t, 1 H), 8.45 (d, 1 H), 8.73 (s, 1 H), 12.38 (s, 1 H).

### Stufe B:

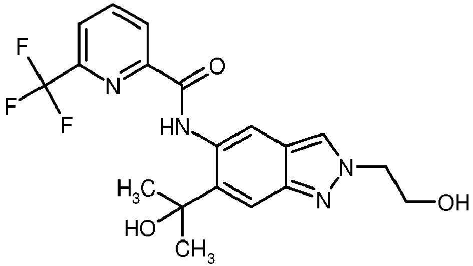

50 mg (96 µmol) N-[2-(2-{[tert-Butyl(dimethyl)silyl]oxy}ethyl)-6-(hydroxymethyl)-2H-indazol-5-yl]-6-(trifluormethyl)pyridin-2-carboxamid wurden in 1.0 ml THF gelöst und mit 144 µL (0.14 mmol) einer 1 M Lösung Tetrabutylammoniumfluorid in THF versetzt. Die Reaktionsmischung wurde 1 h bei Raumtemperatur gerührt. Man verdünnte mit Wasser, extrahierte zweimal mit Ethylacetat und die vereinigten organischen Phasen wurden mit gesättigter Natriumchloridlösung gewaschen, über einen wasserabweisenden Filter filtriert und eingeengt. Man erhielt 36 mg N-[2-(2-Hydroxyethyl)-6-(2-hydroxypropan-2-yl)-2H-indazol-5-yl]-6-(trifluormethyl)pyridin-2-carboxamid (Beispiel 5).

¹H-NMR (400MHz, DMSO-d₆): d [ppm] = 1.62 (s, 6H), 3.86 (q, 2H), 4.43 (t, 2H), 4.95 (t, 1H), 5.94 (s, 1H), 7.57 (s, 1H), 8.16 (dd, 1H), 8.30 (s, 1H), 8.37 (t, 1H), 8.45 (d, 1H), 8.72 (s, 1H), 12.36 (s, 1H).
UPLC-MS (Methode A2): Rₜ = 0.97 min (UV Detektor: TIC), gefundene Masse 408.00.

### Beispiel 6

### N-[6-(2-Hydroxypropan-2-yl)-2-(3-hydroxypropyl)-2H-indazol-5-yl]-6-(trifluormethyl)pyridin-2-carboxamid

### Stufe A:

### Herstellung von N-[2-(3-{[tert-Butyl(dimethyl)silyl]oxy}propyl)-6-(2-hydroxypropan-2-yl)-2H-indazol-5-yl]-6-(trifluormethyl)pyridin-2-carboxamid

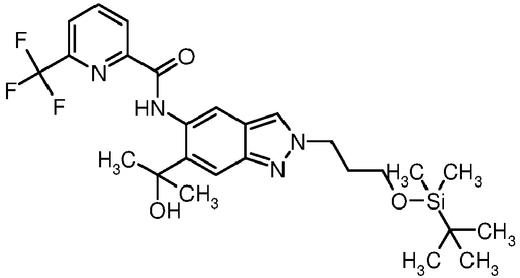

50 mg (0.09 mmol) Methyl-2-(3-{[tert-butyl(dimethyl)silyl]oxy}propyl)-5-({[6-(trifluormethyl)pyridin-2-yl]carbonyl}amino)-2H-indazol-6-carboxylat (Intermediat 4-6) wurden in 500 µl THF gelöst und mit 326 µl (0.33 mmol) einer 1 M Methylmagnesiumbromidlösung in THF versetzt. Die Reaktionsmischung wurde 60 min bei 25°C gerührt. Anschließend wurden vorsichtig 20 ml einer gesättigten Ammoniumchloridlösung hinzugegeben und zweimal mit Ethylacetat extrahiert. Die vereinigten organischen Phasen wurden über einen wasserabweisenden Filter filtriert, eingeengt und im Vakuum getrocknet. Der Rückstand wurde durch präparative HPLC gereinigt. Es wurden 40 mg N-[2-(3-{[tert-Butyl(dimethyl)silyl]oxy}propyl)-6-(2-hydroxypropan-2-yl)-2H-indazol-5-yl]-6-(trifluormethyl)pyridin-2-carboxamid erhalten.
UPLC-MS (Methode A1): Rₜ = 1.58 min
MS (ESIpos): m/z = 537(M+H)⁺

¹H NMR (300 MHz, DMSO-d6): δ [ppm] = 0.02 - 0.05 (m, 6 H), 0.84 - 0.91 (m, 9 H), 1.62 (s, 6 H), 2.02 - 2.18 (m, 2 H), 3.55 - 3.62 (m, 2 H), 4.45 (t, 2 H), 5.96 (s, 1 H), 7.57 (s, 1 H), 8.16 (d, 1 H), 8.31 (s, 1 H), 8.33 - 8.42 (m, 1 H), 8.45 (d, 1 H), 8.72 (s, 1 H), 12.37 (s, 1 H).

### Stufe B:

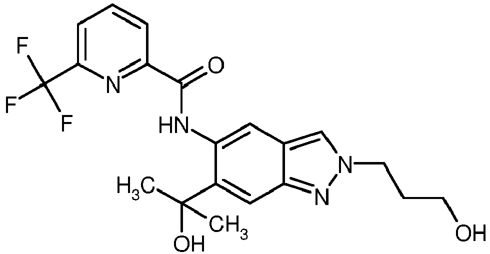

37 mg (0.07 mmol) N-[2-(3-{[tert-Butyl(dimethyl)silyl]oxy}propyl)-6-(2-hydroxypropan-2-yl)-2H-indazol-5-yl]-6-(trifluormethyl)pyridin-2-carboxamid wurden in 500 µl THF gelöst und mit 207 µL (0.21 mmol) einer 1 M Lösung Tetrabutylammoniumfluorid in THF versetzt. Die Reaktionsmischung wurde 2 h bei 25°C gerührt. Man verdünnte mit Wasser, extrahierte zweimal mit Ethylacetat und die vereinigten organischen Phasen wurden mit gesättigter Natriumchloridlösung gewaschen, filtriert und eingeengt. Nach Reinigung durch präparative HPLC wurden 10 mg N-[6-(2-Hydroxypropan-2-yl)-2-(3-hydroxypropyl)-2H-indazol-5-yl]-6-(trifluormethyl)pyridin-2-carboxamid (Beispiel 6, enthielt Nebenkomponente) erhalten.
UPLC-MS (Methode A2): Rₜ = 1.00 min
MS (ESIpos): m/z = 423 (M+H)⁺

¹H NMR ausgewählte Signale (400 MHz, DMSO-d6): δ [ppm] = 1.61 (s), 2.00 - 2.12 (m), 3.38 (t, 2 H), 4.44 (t, 2 H), 4.62 (br. s., 1 H), 5.93 (br. s., 1 H), 7.55 (s, 1 H), 8.13 (d, 1 H), 8.27 - 8.38 (m, 2 H), 8.43 (d, 1 H), 8.71 (s, 1 H), 12.30 (br. s., 1 H).

### Beispiel 7

### N-[2-(2-Hydroxyethyl)-6-(hydroxymethyl)-2H-indazol-5-yl]-6-(trifluormethyl)pyridin-2-carboxamid

### Stufe A:

### N-[2-(2-{ [tert-Butyl(dimethyl)silyl]oxy}ethyl)-6-(hydroxymethyl)-2H-indazol-5-yl]-6-(trifluormethyl)pyridin-2-carboxamid

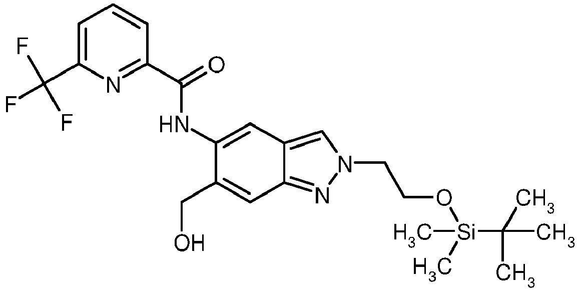

100 mg (0.19 mmol) Methyl-2-(2-{[tert-butyl(dimethyl)silyl]oxy}ethyl)-5-({[6-(trifluormethyl)pyridin-2-yl]carbonyl}amino)-2H-indazol-6-carboxylat (Intermediat 4-5) wurden in 1 ml THF gelöst und mit 191 µl (0.38 mmol) einer 2 M Lithiumborhydridlösung versetzt. Man ließ 24 h bei 25°C rühren. Es wurden 14 mg (0.38 mmol) Natriumborhydrid und 500 µl Methanol zugegeben und 4 h bei 25°C gerührt. Es wurden nochmals 14 mg (0.38 mmol) Natriumborhydrid zugegeben und 24 h bei 25°C gerührt. Die Reaktionsmischung wurde vorsichtig mit Wasser versetzt und die organische Phase abgetrennt. Anschließend wurde zweimal mit Ethylacetat extrahiert und die vereinigten organischen Phasen mit gesättigter Natriumchloridlösung gewaschen, über einen wasserabweisenden Filter filtriert und eingeengt. Der Rückstand wurde in 2 ml DMSO aufgenommen und durch präparative HPLC gereinigt. Man erhielt 30 mg N-[2-(2-{[tert-Butyl(dimethyl)silyl]oxy}ethyl)-6-(hydroxymethyl)-2H-indazol-5-yl]-6-(trifluormethyl)pyridin-2-carboxamid.
UPLC-MS (Methode A2): Rₜ = 1.44 min
MS (ESIpos): m/z = 495(M+H)⁺

¹H NMR (300 MHz, DMSO-d6): δ [ppm] = -0.16 - -0.12 (m, 6 H), 0.75 - 0.79 (m, 9 H), 4.05 (t, 2 H), 4.48 (t, 2 H), 4.69 (d, 2 H), 5.75 - 5.77 (m, 1 H), 7.57 (s, 1 H), 8.18 (dd, 1 H), 8.30 - 8.33 (m, 1 H), 8.38 (t, 1 H), 8.45 (d, 1 H), 8.51 (s, 1 H), 11.20 (s, 1 H).

### Stufe B:

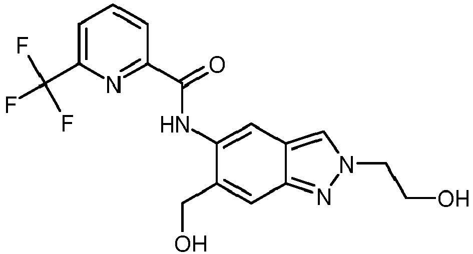

33 mg (0.07 mmol) N-[2-(2-{[tert-Butyl(dimethyl)silyl]oxy}ethyl)-6-(hydroxymethyl)-2H-indazol-5-yl]-6-(trifluormethyl)pyridin-2-carboxamid wurden in 1 ml THF gelöst und mit 100 µL (0.10 mmol) einer 1 M Lösung Tetrabutylammoniumfluorid in THF versetzt. Die Reaktionsmischung wurde 1 h bei 25°C gerührt. Man verdünnte mit Wasser, extrahierte zweimal mit Ethylacetat und die vereinigten organischen Phasen wurden mit gesättigter Natriumchloridlösung gewaschen, über einen wasserabweisenden Filter filtriert, eingeengt und im Vakuum getrocknet. Es wurden 25 mg N-[2-(2-Hydroxyethyl)-6-(hydroxymethyl)-2H-indazol-5-yl]-6-(trifluormethyl)pyridin-2-carboxamid (Beispiel 7) erhalten.
UPLC-MS (Methode A2): Rₜ = 0.87 min
MS (ESIpos): m/z = 381 (M+H)⁺

¹H NMR (300 MHz, DMSO-d6): δ [ppm] = 3.87 (q, 2 H), 4.44 (t, 2 H), 4.69 (d, 2 H), 4.98 (t, 1 H), 5.70 - 5.81 (m, 1 H), 7.57 (s, 1 H), 8.11 - 8.23 (m, 1 H), 8.31 - 8.42 (m, 2 H), 8.43 - 8.49 (m, 1 H), 8.51 (s, 1 H), 11.20 (s, 1 H).

### Beispiel 8

### N-[6-(2-Hydroxypropan-2-yl)-2-(oxetan-3-ylmethyl)-2H-indazol-5-yl]-6-(trifluormethyl)pyridin-2-carboxamid

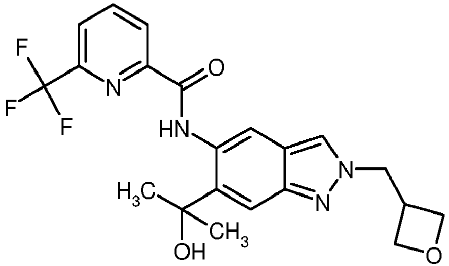

50 mg (0.12 mmol) Methyl-2-(oxetan-3-ylmethyl)-5-({[6-(trifluormethyl)pyridin-2-yl]carbonyl}amino)-2H-indazol-6-carboxylat (Intermediat 4-1) wurden in 500 µl THF gelöst und mit 576 µl (0.58 mmol) einer 1 M Methylmagnesiumbromidlösung in THF versetzt. Die Reaktionsmischung wurde 60 min bei 25°C gerührt. Anschließend wurden vorsichtig 20 ml einer gesättigten wässrigen Ammoniumchloridlösung hinzugegeben und eingeengt. Die wässrige Phase wurde zweimal mit Ethylacetat extrahiert, die organischen Phasen vereint, über Magnesiumsulfat getrocknet, filtriert und eingeengt. Der Rückstand wurde in 2.0 ml DMSO gelöst und durch präparative HPLC gereinigt. Die produkthaltigen Fraktionen wurden gefriergetrocknet. Es wurden 30 mg der Titelverbindung erhalten.
UPLC-MS (Methode A2): Rₜ = 1.03 min
MS (ESIpos): m/z = 435 (M+H)⁺

¹H NMR (400 MHz, DMSO-d6): δ [ppm] = 1.62 (s, 6 H), 3.45 - 3.61 (m, 1 H), 4.48 (t, 2 H), 4.66 (dd, 2 H), 4.72 (d, 2 H), 5.94 (s, 1 H), 7.57 (s, 1 H), 8.16 (d, 1 H), 8.33 - 8.42 (m, 2 H), 8.42 - 8.47 (m, 1 H), 8.72 (s, 1 H), 12.36 (s, 1 H).

### Beispiel 9

### N-[6-(Hydroxymethyl)-2-(oxetan-3-ylmethyl)-2H-indazol-5-yl]-6-(trifluormethyl)pyridin-2-carboxamid

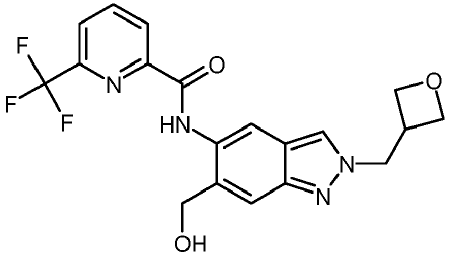

75 mg (0.17 mmol) Methyl-2-(oxetan-3-ylmethyl)-5-({[6-(trifluormethyl)pyridin-2-yl]carbonyl}amino)-2H-indazol-6-carboxylat (Intermediat 4-1) wurden in 1 ml einer Mischung aus THF/Methanol (1:1) gelöst und mit 8 mg (0.21 mmol) Natriumborhydrid versetzt. Man ließ 60 min bei 25 °C rühren. Die Reaktionsmischung wurde eingeengt und der Rückstand mit Wasser versetzt. Die Suspension wurde 15 min kräftig gerührt, der Feststoff abgesaugt, zweimal mit Wasser und zweimal mit Diethylether gewaschen und im Vakuum getrocknet. Es wurden 48 mg der Titelverbindung erhalten.
UPLC-MS (Methode A2): Rₜ = 0.94 min
MS (ESIpos): m/z = 407 (M+H)⁺

¹H NMR (300 MHz, DMSO-d6): δ [ppm] = 3.55 (s, 1 H), 4.48 (t, 2 H), 4.61 - 4.77 (m, 6 H), 7.57 (s, 1 H), 8.18 (dd, 1 H), 8.33 - 8.49 (m, 3 H), 8.51 (s, 1 H), 11.21 (s, 1 H).

### Beispiel 10

### N-{6-(2-Hydroxypropan-2-yl)-2-[3-(methylsulfonyl)propyl]-2H-indazol-5-yl}-6-(trifluormethyl)pyridin-2-carboxamid

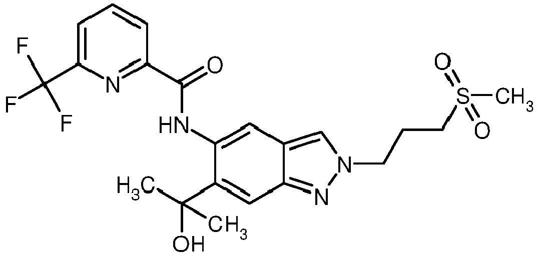

Eine Mischung aus 500 mg (1.32 mmol) N-[6-(2-Hydroxypropan-2-yl)-1H-indazol-5-yl]-6-(trifluormethyl)pyridin-2-carboxamid (Intermediat 5-1), 569 mg Kaliumcarbonat und 114 mg Kaliumiodid in 5.0 ml DMF wurde 15 min bei Raumtemperatur gerührt. Man addierte 414 mg 1-Brom-3-(methylsulfonyl)propan und rührte bei Raumtemperatur über Nacht. Man versetzte mit Wasser, extrahierte zweimal mit Ethylacetat, wusch mit Kochsalzlösung und engte ein. Der Rückstand wurde säulenchromatographisch gereinigt (Dichlormethan-Methanol-Gradient). Nach Ausrühren der Produktfraktion aus Diethylether wurden 59 mg der Titelverbindung erhalten.
UPLC-MS (Methode A2): Rₜ = 1.02 min
MS (ESIpos): m/z = 485 (M+H)+

¹H-NMR (300MHz, DMSO-d₆): δ [ppm]= 1.63 (s, 6H), 2.26 - 2.42 (m, 2H), 2.99 (s, 3H), 3.06 - 3.16 (m, 2H), 4.55 (t, 2H), 5.96 (s, 1H), 7.60 (s, 1H), 8.16 (d, 1H), 8.33 - 8.48 (m, 3H), 8.73 (s, 1H), 12.37 (s, 1H).

### Beispiel 11

### N-[2-(3-Hydroxy-3-methylbutyl)-6-(2-hydroxypropan-2-yl)-2H-indazol-5-yl]-6-(trifluormethyl)pyridin-2-carboxamid

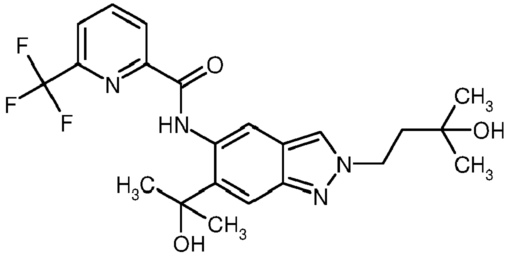

### Herstellungsmethode 1

705 mg (1.57 mmol) Methyl-2-(3-hydroxy-3-methylbutyl)-5-({[6-(trifluormethyl)pyridin-2-yl]carbonyl}amino)-2H-indazol-6-carboxylat (Intermediat 4-4) wurden in 10 ml THF vorgelegt und im Eiswasser-Kältebad gekühlt. Man addierte 2.6 ml (5.0 Äquivalente) 3M Methylmagnesiumbromidlösung (in Diethylether) und ließ 1 h unter Eisbadkühlung und 4.5 h bei Raumtemperatur rühren. Man addierte erneut 1 Äquivalent der Methylmagnesiumbromidlösung und ließ 20.5 h bei Raumtemperatur rühren. Erneut addierte man 1 Äquivalent der Methylmagnesiumbromidlösung und ließ 22 h bei Raumtemperatur rühren. Das Reaktionsgemisch wurde mit gesättigter wässriger Ammoniumchloridlösung versetzt, gerührt und dreimal mit Ethylacetat extrahiert. Die vereinigten organischen Phasen wurden mit Kochsalzlösung gewaschen, über einen wasserabweisenden Filter filtriert und eingeengt. Man erhielt 790 mg eines Rückstandes, der durch präparative HPLC gereinigt wurde. Man erhielt 234 mg der Titelverbindung und 164 mg einer Produktfraktion, die mit Diethylether ausgerührt wurde. Nach Absaugen und anschließender Trocknung wurden weitere 146 mg der Titelverbindung erhalten.
UPLC-MS (Methode A1): Rₜ = 1.10 min (UV Detektor: TIC), gefundene Masse 450.00.

¹H-NMR (400MHz, DMSO-d₆): δ [ppm]= 1.14 (s, 6H), 1.61 (s, 6H), 1.99 - 2.08 (m, 2H), 4.42 - 4.55 (m, 3H), 5.93 (s, 1H), 7.56 (s, 1H), 8.15 (dd, 1H), 8.32 - 8.39 (m, 2H), 8.41 - 8.47 (m, 1H), 8.70 (s, 1H), 12.34 (s, 1H).

### Herstellungsmethode 2

Eine Mischung aus 500 mg (1.37 mmol) N-[6-(2-Hydroxypropan-2-yl)-1H-indazol-5-yl]-6-(trifluormethyl)pyridin-2-carboxamid (Intermediat 5-1), 569 mg Kaliumcarbonat und 114 mg Kaliumiodid in 5 ml DMF wurde 15 min bei Raumtemperatur gerührt. Man addierte 344 mg (1.5 Äquivalente) 4-Brom-2-methylbutan-2-ol und erwärmte 2 h auf 100°C. Man addierte erneut 0.5 Äquivalente 4-Brom-2-methylbutan-2-ol und rührte bei Raumtemperatur für 16 h. Die Mischung wurde mit Wasser versetzt, zweimal mit Ethylacetat extrahiert, die vereinigten organischen Phasen wurden mit gesättigter Kochsalzlösung gewaschen und über einen wasserabweisenden Filter filtriert und eingeengt. Der Rückstand wurde durch säulenchromatographische Reinigung an Kieselgel (Hexan/Ethylacetat) gereinigt. Man erhielt 100 mg einer Produktfraktion, die mit Diethylether ausgerührt wurde. Nach Trocknen wurden 60 mg der Titelverbindung erhalten.

¹H-NMR (400MHz, DMSO-d₆): δ [ppm]= 1.14 (s, 6 H), 1.61 (s, 6H), 1.99 - 2.07 (m, 2 H), 4.43 - 4.52 (m, 3 H) 5.94 (s, 1 H) 7.57 (s, 1 H) 8.15 (dd, 1H) 8.33 - 8.40 (m, 2 H), 8.42 - 8.48 (m, 1 H), 8.71 (s, 1 H), 12.35 (s, 1 H)

### Beispiel 12

### N-{6-(2-Hydroxypropan-2-yl)-2-[2-(methylsulfonyl)ethyl]-2H-indazol-5-yl}-6-(trifluormethyl)pyridin-2-carboxamid

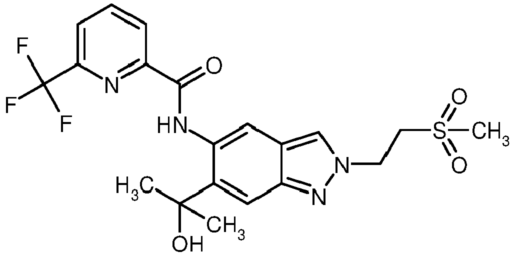

160 mg (0.44 mmol) N-[6-(2-Hydroxypropan-2-yl)-1H-indazol-5-yl]-6-(trifluormethyl)pyridin-2-carboxamid (Intermediat 5-1) wurden zusammen mit 182 mg Kaliumcarbonat und 36 mg Kaliumiodid in 1.0 ml DMF suspendiert und die Mischung wurde 15 min bei Raumtemperatur gerührt. Dann wurden 123 mg 2-Bromethyl-methylsulfon (0.66 mmol) zugegeben und über Nacht bei Raumtemperatur gerührt. Man addierte Wasser, extrahierte zweimal mit Ethylacetat, wusch mit gesättigter wässriger Natriumchloridlösung, filtrierte über einen wasserabweisenden Filter und engte ein. Nach Reinigung des Rückstandes durch präparative HPLC wurden 20 mg der Titelverbindung erhalten.
UPLC (Methode A2): Rₜ = 1.01 min;
MS (ESIpos): m/z = 471 (M+H)+

¹H NMR (400 MHz, DMSO-d₆): δ [ppm]= 1.63 (s, 6 H), 2.90 (s, 3 H), 3.85 (t, 2 H), 4.86 (t, 2 H), 5.97 (s, 1 H), 7.59 (s, 1 H), 8.13 - 8.19 (m, 1 H), 8.37 (s, 1 H), 8.41 - 8.48 (m, 2 H), 8.74 (s, 1 H), 12.37 (s, 1 H).

### Beispiel 13

### 6-(Difluormethyl)-N-[2-(3-hydroxy-3-methylbutyl)-6-(2-hydroxypropan-2-yl)-2H-indazol-5-yl]pyridin-2-carboxamid

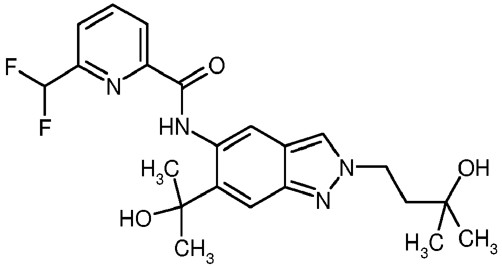

### Herstellungsmethode 1

Ein Mischung aus 250 mg 6-(Difluormethyl)-N-[6-(2-hydroxypropan-2-yl)-1H-indazol-5-yl]pyridin-2-carboxamid (Rohprodukt Intermediat 5-2), 144 mg Kaliumiodid und 239 mg Kaliumcarbonat in 2.5 ml DMF wurde 15 min bei Raumtemperatur gerührt. Man addierte 145 mg (0.87 mmol) 4-Brom-2-methylbutan-2-ol, rührte bei 110°C 3 h, addierte erneut 96 mg 4-Brom-2-methylbutan-2-ol und rührte bei 110°C 4 h. Man versetzte mit Wasser, extrahierte zweimal mit Ethylacetat, wusch mit halbgesättigter wässriger Kochsalzlösung, filtrierte über einen wasserabweisenden Filter und engte ein. Man reinigte säulenchromatographisch an Kieselgel (Hexan/Ethylacetat). Es wurden 61 mg der Titelverbindung erhalten.
UPLC-MS (Methode A1): Rₜ = 1.00 min (UV Detektor: TIC), gefundene Masse 432.00.

¹H-NMR (300MHz, DMSO-d₆): δ [ppm]= 1.14 (s, 6H), 1.63 (s, 6H), 1.97 - 2.08 (m, 2H), 4.41 - 4.55 (m, 3H), 5.99 (s, 1H), 7.03 (t, 1H), 7.56 (s, 1H), 7.94 - 8.00 (m, 1H), 8.24 - 8.38 (m, 3H), 8.71 (s, 1H), 12.49 (s, 1H).

### Herstellungsmethode 2

Analog zur Herstellung von Beispiel 11 (Herstellungsmethode 1) wurden 3.00 g Methyl-5-({[6-(difluormethyl)pyridin-2-yl]carbonyl}amino)-2-(3-hydroxy-3-methylbutyl)-2H-indazol-6-carboxylat (Intermediat 4-11) mit 3M Methylmagnesiumbromidlösung (in Diethylether) umgesetzt. Nach Reinigung des Rohproduktes durch Ausrühren aus Diethylether und nachfolgend durch präparative HPLC wurden 1.37 g der Titelverbindung erhalten.

### Beispiel 14

### 6-(Difluormethyl)-N-{6-(2-hydroxypropan-2-yl)-2-[2-(methylsulfonyl)ethyl]-2H-indazol-5-yl}pyridin-2-carboxamid

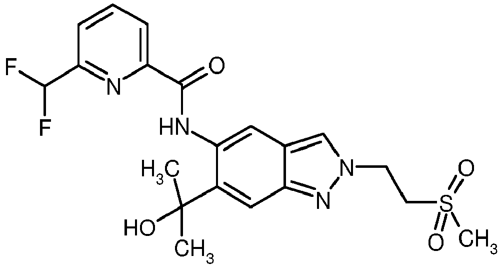

Ein Mischung aus 250 mg 6-(Difluormethyl)-N-[6-(2-hydroxypropan-2-yl)-1H-indazol-5-yl]pyridin-2-carboxamid (Rohprodukt Intermediat 5-2), 144 mg Kaliumiodid und 239 mg Kaliumcarbonat in 2.5 ml DMF wurde 15 min bei Raumtemperatur gerührt. Man addierte 162 mg (0.87 mmol) 2-Bromethyl-methylsulfon und rührte bei 110°C 3 h. Man versetzte mit Wasser, extrahierte zweimal mit Ethylacetat, wusch mit halbgesättigter wässriger Kochsalzlösung, filtrierte über einen wasserabweisenden Filter und engte ein. Der Rückstand wurde durch präparative HPLC gereinigt und die Produktfraktionen zusätzlich noch durch säulenchromatographische Reinigung an Kieselgel (Hexan-Ethylacetat) gereinigt. Es wurden 40 mg der Titelverbindung erhalten.

¹H-NMR (400MHz, DMSO-d₆): δ [ppm]= 1.65 (s, 6H), 2.90 (s, 3H), 3.85 (t, 2H), 4.85 (t, 2H), 6.03 (s, 1H), 7.04 (t, 1H), 7.59 (s, 1H), 7.98 (d, 1H), 8.25 - 8.36 (m, 2H), 8.43 (s, 1H), 8.75 (s, 1H), 12.52 (s, 1H).

### Beispiel 15

### 6-(Difluormethyl)-N-[6-(2-hydroxypropan-2-yl)-2-(3-hydroxypropyl)-2H-indazol-5-yl]pyridin-2-carboxamid

### Stufe A:

### Herstellung von N-[2-(3-{[tert-Butyl(dimethyl)silyl]oxy}propyl)-6-(2-hydroxypropan-2-yl)-2H-indazol-5-yl]-6-(difluormethyl)pyridin-2-carboxamid

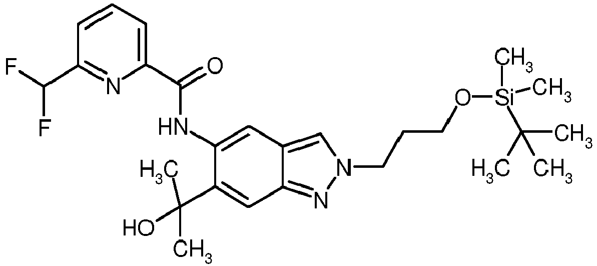

Ein Mischung aus 250 mg 6-(Difluormethyl)-N-[6-(2-hydroxypropan-2-yl)-1H-indazol-5-yl]pyridin-2-carboxamid (Intermediat 5-2), 48 mg Kaliumiodid und 239 mg Kaliumcarbonat in 2.5 ml DMF wurde 15 min bei Raumtemperatur gerührt. Man addierte 219 mg (0.87 mmol, 1.5 Äquivalente) (3-Brompropoxy)(tert-butyl)dimethylsilan und rührte 3 h bei 110°C. Man gab erneut 1 Äquivalent (3-Brompropoxy)(tert-butyl)dimethylsilan zu und rührte 4 h bei 100°C. Man versetzte mit Wasser, extrahierte mit Ethylacetat, wusch mit wässriger Kochsalzlösung, filtrierte durch einen wasserabweisenden Filter und engte ein. Der Rückstand wurde säulenchromatographisch gereinigt (Hexan/Ethylacetat). Es wurden 92 mg der Titelverbindung erhalten.

### Stufe B:

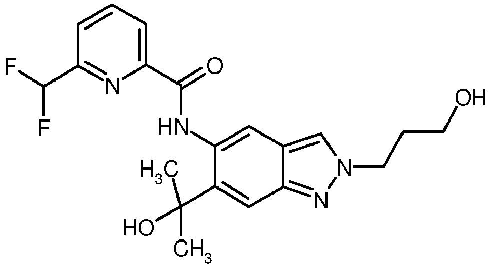

Analog zur Herstellung von Beispiel 6, Stufe B, wurden 92 mg N-[2-(3-{[tert-Butyl(dimethyl)silyl]oxy }propyl)-6-(2-hydroxypropan-2-yl)-2H-indazol-5-yl]-6-(difluormethyl)pyridin-2-carboxamid mit 0.53 ml einer 1 M Lösung Tetrabutylammoniumfluorid in THF innerhalb von 1 h umgesetzt. Nach wässriger Aufarbeitung wie bei Beispiel 6 und Reinigung durch präparative HPLC wurden 46 mg der Titelverbindung erhalten.
UPLC-MS (Methode A1): Rₜ = 0.92 min (UV Detektor: TIC), gefundene Masse 404.00.

¹H-NMR (400MHz, DMSO-d₆): δ [ppm]= 1.64 (s, 6H), 2.05 (quin, 2H), 3.35 - 3.46 (m, 2H), 4.45 (t, 2H), 4.64 (t, 1H), 5.99 (s, 1H), 7.04 (t, 1H), 7.57 (s, 1H), 7.95 - 7.99 (m, 1H), 8.25 - 8.36 (m, 3H), 8.73 (s, 1H), 12.50 (s, 1H).

### Beispiel 16

### N-[6-(2-Hydroxypropan-2-yl)-2-(4,4,4-trifluorbutyl)-2H-indazol-5-yl]-6-(trifluormethyl)pyridin-2-carboxamid

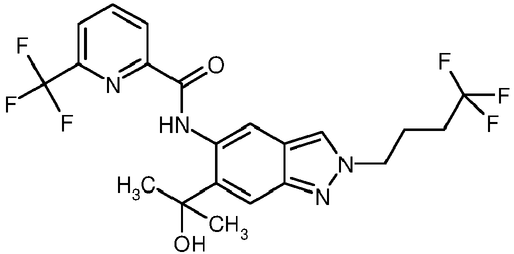

Eine Mischung aus 210 mg (0.58 mmol) N-[6-(2-Hydroxypropan-2-yl)-1H-indazol-5-yl]-6-(trifluormethyl)pyridin-2-carboxamid (Intermediat 5-1) in 3 ml DMF wurde mit 0.11 ml (0.87 mmol) 1,1,1-Trifluor-4-iodbutan und 239 mg Kaliumcarbonat versetzt und die Mischung wurde 6 h bei 80°C gerührt. Nach Addition von Wasser wurde dreimal mit Ethylacetat extrahiert und die vereinigten organischen Phasen wurden mit gesättigter Kochsalzlösung durch einen wasserabweisenden Filter filtriert und eingeengt. Das Rohprodukt wurde durch präparative HPLC gereinigt. Es wurden 19 mg der Titelverbindung erhalten.
UPLC-MS (Methode A1): Rₜ = 1.27 min (UV Detektor: TIC), gefundene Masse 474.15.

¹H-NMR (400MHz, DMSO-d₆): δ [ppm]= 1.62 (s, 6H), 2.10 - 2.33 (m), 4.49 (t, 2H), 5.94 (s, 1H), 7.59 (s, 1H), 8.13 - 8.18 (m, 1H), 8.32 - 8.41 (m, 2H), 8.41 - 8.47 (m, 1H), 8.72 (s, 1H), 12.35 (s, 1H).

### Beispiel 17

### N-{6-(2-Hydroxypropan-2-yl)-2-[3-(trifluormethoxy)propyl]-2H-indazol-5-yl}-6-(trifluormethyl)pyridin-2-carboxamid

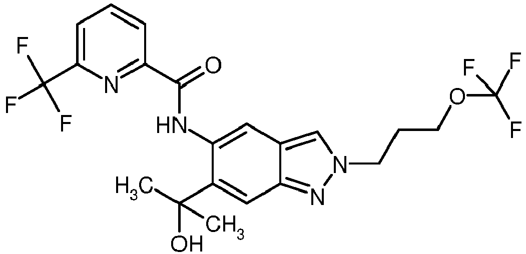

150 mg (0.33 mmol) N-[6-(2-Hydroxypropan-2-yl)-1H-indazol-5-yl]-6-(trifluormethyl)pyridin-2-carboxamid (Intermediat 5-1) wurden in 2 ml THF vorgelegt. Man addierte 58 mg (0.40 mmol) 3-(Trifluormethoxy)propan-1-ol, 131 mg Triphenylphosphin und 71 µl Diisopropyl-azodicarboxylat (DIAD, CAS 2446-83-5) und rührte 19 h bei Raumtemperatur. Man versetzte mit 0.83 ml Natronlauge (2M) und rührte 5 h bei 40°C. Man verdünnte mit Wasser, extrahierte dreimal mit Ethylacetat, engte die vereinigten organischen Phasen ein und reinigte durch präparative HPLC. Es wurden 16 mg der Titelverbindung als Rohprodukt erhalten.
UPLC-MS (Methode A2): Rₜ = 1.26 min (UV Detektor: TIC), gefundene Masse 490.14.

¹H-NMR (400MHz, DMSO-d₆, ausgewählte Signale): δ [ppm]= 1.61 (s, 6H), 1.84 (d, 1H), 2.32 (quint., 2H), 4.08 (t, 2H), 4.51 (t, 2H), 7.58 (s, 1H), 8.15 (d, 1H), 8.31 - 8.39 (m, 2H), 8.44 (d, 1H), 8.72 (s, 1H), 12.35 (s, 1H).

### Beispiel 18

### N-{6-(2-Hydroxypropan-2-yl)-2-[3-(2,2,2-trifluorethoxy)propyl]-2H-indazol-5-yl}-6-(trifluormethyl)pyridin-2-carboxamid

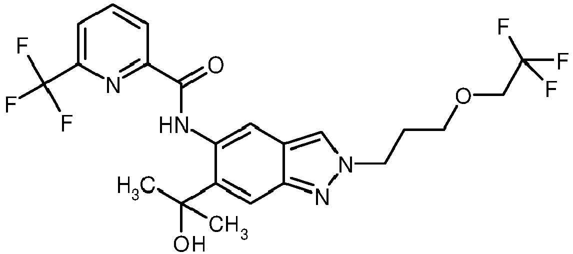

Analog zur Herstellung von Beispiel 11 (Herstellungsmethode 1) wurden 52 mg (0.10 mmol) Methyl-2-[3-(2,2,2-trifluorethoxy)propyl]-5-({[6-(trifluormethyl)pyridin-2-yl]carbonyl}amino)-2H-indazol-6-carboxylat (Intermediat 4-10) in 3 ml THF mit 2 mal 171 µl 3M Magnesiumbromidlösung in Diethylether umgesetzt. Nach Reinigung durch präparative HPLC wurden 12 mg der Titelverbindung erhalten.
UPLC-MS (Methode A1): Rₜ = 1.25 min (UV Detektor: TIC), gefundene Masse 504.16.

¹H-NMR (500 MHz, DMSO-d₆): δ [ppm] = 1.63 (s, 6H), 2.20(quin, 2H), 3.58(t, 2H),4.05(q, 2H), 4.47(t, 2H),5.94(s, 1H), 7.58 (s, 1H), 8.15 (dd, 1H), 8.32 (s, 1H), 8.36 (t, 1H), 8.45(d, 1H), 8.73 (s, 1H), 12.36 (s,1H).

### Beispiel 19

### 5-Fluor-N-[2-(3-hydroxy-3-methylbutyl)-6-(2-hydroxypropan-2-yl)-2H-indazol-5-yl]-6-methylpyridin-2-carboxamid

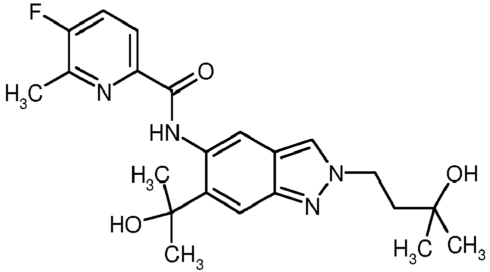

228 mg (0.31 mmol) Methyl-5-{[(5-fluor-6-methylpyridin-2-yl)carbonyl]amino}-2-(3-hydroxy-3-methylbutyl)-2H-indazol-6-carboxylat (Intermediat 4-8) wurden in 4.5 ml THF vorgelegt und mit einem Eiskältebad abgekühlt. Man addierte 0.63 ml 3M Methylmagnesiumbromidlösung (in Diethylether) und ließ 2 h unter Eisbadkühlung und 21 h bei Raumtemperatur rühren. Das Reaktionsgemisch wurde mit gesättigter wässriger Ammoniumchloridlösung versetzt und dreimal mit Ethylacetat extrahiert. Die vereinigten organischen Phasen wurden eingeengt. Der Rückstand wurde durch präparative HPLC gereinigt. Es wurden 82 mg der Titelverbindung erhalten.
UPLC-MS (Methode A2): Rₜ = 1.03 min (UV Detektor: TIC), gefundene Masse 414.21.

¹H-NMR (400MHz, DMSO-d₆): δ [ppm]= 1.13 (s, 6H), 1.63 (s, 6H), 1.99 - 2.05 (m, 2H), 2.55 - 2.59 (m, 3H), 4.42 - 4.50 (m, 3H), 5.95 (s, 1H), 7.54 (s, 1H), 7.83 (t, 1H), 8.05 (dd, 1H), 8.31 (s, 1H), 8.68 (s, 1H), 12.33 (s, 1H).

### Beispiel 20

### N-[2-(3-Hydroxy-3-methylbutyl)-6-(2-hydroxypropan-2-yl)-2H-indazol-5-yl]-6-methylpyridin-2-carboxamid

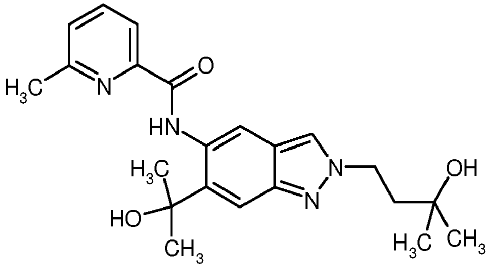

278 mg (0.48 mmol) Methyl-2-(3-hydroxy-3-methylbutyl)-5-{[(6-methylpyridin-2-yl)carbonyl]amino}-2H-indazol-6-carboxylat (Intermediat 4-9) wurden in 5.0 ml THF vorgelegt und mit einem Eiskältebad abgekühlt. Man addierte 0.97 ml 3M Methylmagnesiumbromidlösung (in Diethylether) und ließ 2 h unter Eisbadkühlung und 20.5 h bei Raumtemperatur rühren. Man addierte erneut 0.48 ml 3M Methylmagnesiumbromidlösung und ließ 67 h bei Raumtemperatur rühren. Man versetzte mit gesättigter wässriger Ammoniumchloridlösung, extrahierte dreimal mit Ethylacetat, wusch mit Kochsalzlösung, filtrierte über einen wasserabweisenden Filter und engte ein. Der Rückstand wurde durch präparative HPLC gereinigt. Es wurden 111 mg der Titelverbindung erhalten.
UPLC-MS (Methode A2): Rₜ = 0.97 min (UV Detektor: TIC), gefundene Masse 396.22.

¹H-NMR (400MHz, DMSO-d₆): δ [ppm]= 1.15 (s, 6H), 1.64 (s, 6H), 2.00 - 2.08 (m, 2H), 2.61 (s, 3H), 4.41 - 4.59 (m, 3H), 5.92 (s, 1H), 7.50 (dd, 1H), 7.56 (s, 1H), 7.90 - 7.99 (m, 2H), 8.33 (s, 1H), 8.70 (s, 1H), 12.39 (s, 1H).

### Beispiel 21

### 6-(2-Hydroxypropan-2-yl)-N-[6-(2-hydroxypropan-2-yl)-2-(4,4,4-trifluorbutyl)-2H-indazol-5-yl]pyridin-2-carboxamid

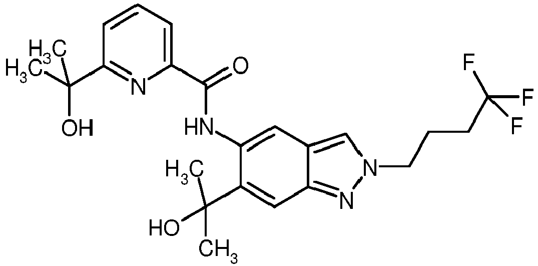

Eine Lösung aus 72 mg (0.16 mmol) Methyl-5-({[6-(2-hydroxypropan-2-yl)pyridin-2-yl]carbonyl}amino)-2-(4,4,4-trifluorbutyl)-2H-indazol-6-carboxylat (Intermediat 4-7) in 10 ml THF wurde in einem Eiswasser-Kältebad gekühlt. Man addierte 0.26 ml einer 3M Methylmagnesiumbromidlösung in Diethylether, rührte 2 h und danach 20 h bei Raumtemperatur. Man addierte erneut 1 Äquivalent der 3M Methylmagnesiumbromidlösung und rührte 24 h bei Raumtemperatur. Man addierte gesättigte wässrige Ammoniumchloridlösung, extrahierte dreimal mit Ethylacetat, wusch mit Kochsalzlösung und engte ein. Nach präparativer HPLC wurden 22 mg (31% d. Th.) der Titelverbindung erhalten.
UPLC-MS (Methode A2): Rₜ = 1.15 min (UV Detektor: TIC), gefundene Masse 464.20.

¹H-NMR (400MHz, DMSO-d₆): δ [ppm]= 1.56 (s, 6H), 1.64 (s, 6H), 2.07 - 2.34 (m, 4H), 4.49 (t, 2H), 5.32 (s, 1H), 6.05 (s, 1H), 7.60 (s, 1H), 7.87 (dd, 1H), 7.99 - 8.05 (m, 2H), 8.35 (s, 1H), 8.79 (s, 1H), 12.45 (s, 1H).

### Beispiel 22

### N-{2-[2-(1-Hydroxycyclopropyl)ethyl]-6-(2-hydroxypropan-2-yl)-2H-indazol-5-yl}-6-(trifluormethyl)pyridin-2-carboxamid

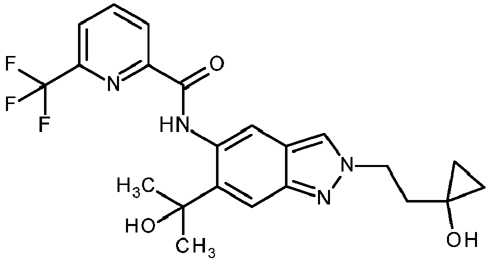

250 mg (0.69 mmol) N-[6-(2-Hydroxypropan-2-yl)-1H-indazol-5-yl]-6-(trifluormethyl)pyridin-2-carboxamid (Intermediat 5-1) wurden in 5 ml DMSO vorgelegt. Es wurden 159 mg (0.96 mmol) 1-(2-Bromethyl)cyclopropanol, 285 mg Kaliumcarbonat und 171 mg Kaliumiodid zugegeben und die Mischung wurde 5 h bei 100°C gerührt. Es wurde Wasser zugegeben und dreimal mit Ethylacetat extrahiert. Die vereinigten, organischen Phasen wurden mit Kochsalzlösung filtriert durch einen wasserabweisenden Filter und eingeengt. Der Rückstand wurde durch präparative HPLC (Säule: Waters XBrigde C18 5µ 100x30mm, Eluent A: Wasser + 0.1 Volumen-% Ameisensäure (99%), Eluent B: Acetonitril) gereinigt. Nach einer Gefriertrocknung erhielt man 45 mg der Titelverbindung.

¹H-NMR (500MHz, DMSO-d₆): δ [ppm]= 0.18 - 0.22 (m, 2H), 0.48 - 0.52 (m, 2H), 1.62 (s, 6H), 2.08 (t, 2H), 4.54 - 4.60 (m, 2H), 5.36 (s, 1H), 5.96 (s, 1H), 7.57 (s, 1H), 8.16 (dd, 1H), 8.34 - 8.39 (m, 2H), 8.45 (d, 1H), 8.72 (s, 1H), 12.36 (s, 1H).

### Bewertung der physiologischen Wirksamkeit

### IRAK4-Kinaseassay

Die IRAK4-inhibitorische Aktivität der erfindungsgemäßen Substanzen wurde in dem nachfolgend beschriebenen IRAK4-TR-FRET-Assay gemessen (TR-FRET = Time Resolved Fluorescence Resonance Energy Transfer).

Rekombinantes Fusionsprotein aus N-terminalem GST (Glutathion-S-Transferase) und humanem IRAK4, exprimiert in Bakulovirus-infizierten Insektenzellen (Hi5, BTI-TN-5B1-4, Zelllinie gekauft von Invitrogen, Katalog-Nr. B855-02) und gereinigt via Affinitätschromatographie, wurde als Enzym verwendet. Als Substrat für die Kinasereaktion wurde das biotinylierte Peptid Biotin-Ahx-KKARFSRFAGSSPSQASFAEPG (C-Terminus in Amid-Form) verwendet, das z.B. bei der Firma Biosyntan GmbH (Berlin-Buch) gekauft werden kann.

Für den Assay wurden 11 verschiedene Konzentrationen im Bereich von 20 µM bis 0,073 nM aus einer 2 mM Lösung der Testsubstanz in DMSO hergestellt. 50 nl der jeweiligen Lösung wurden in eine schwarze low-volume 384well-Mikrotiterplatte (Greiner Bio-One, Frickenhausen, Deutschland) pipettiert, 2 µl einer Lösung von IRAK4 in Assaypuffer [50 mM HEPES pH 7.5, 5mM MgCl2, 1.0 mM Dithiothreitol, 30 µM aktiviertes Natriumorthovanadat, 0,1 % (w/v) bovines gamma-Globulin (BGG) 0,04% (v/v) Nonidet-P40 (Sigma)] hinzugegeben und die Mischung für 15 min inkubiert, um eine Vorbindung der Substanzen an das Enzym vor der Kinasereaktion zu ermöglichen. Dann wurde die Kinasereaktion gestartet durch Zugabe von 3 µl einer Lösung von Adenosinetriphosphat (ATP, 1,67 mM = Endkonzentration in 5 µl Assayvolumen ist 1 mM) und Peptidsubstrat (0,83 µM = Endkonzentration in 5 µl Assayvolumen ist 0,5 µM) in Assaypuffer und die resultierende Mischung für die Reaktionszeit von 45 min bei 22°C inkubiert. Die Konzentration des IRAK4 wurde an die jeweilige Aktivität des Enzyms angepasst und so eingestellt, dass der Assay im linearen Bereich arbeitete. Typische Konzentrationen lagen in der Größenordnung von etwa 0,2 nM. Die Reaktion wurde gestoppt durch Zugabe von 5 µl einer Lösung von TR-FRET-Detektionsreagenzien [0,1 µM Streptavidin-XL665 (Cisbio Bioassays; Frankreich, Katalog-Nr. 610SAXLG) und 1,5 nM Antiphosho-Serin Antikörper [Merck Millipore, "STK Antibody", Katalog-Nr. 35-002] und 0,6 nM LANCE EU-W1024-markierter anti-Maus-IgG-Antikörper (Perkin-Elmer, Produkt-Nr. AD0077. Alternativ kann ein Terbium-Kryptat-markierter anti-Maus-IgG-Antikörper von Cisbio Bioassays verwendet werden) in wässriger EDTA-Lösung (100 mM EDTA, 0.4 % [w/v] bovines Serumalbumin [BSA] in 25 mM HEPES pH 7,5].

Die resultierende Mischung wurde 1 h bei 22°C inkubiert, um die Bildung eines Komplexes aus dem biotinylierten phosphorylierten Substrat und den Detektionsreagenzien zu ermöglichen. Anschließend wurde die Menge des phosphorylierten Substrates ausgewertet durch eine Messung des Resonanz-Energietransfers vom Europium-Chelat markierten anti-Maus-IgG-Antikörper zum Streptavidin-XL665. Hierzu wurden in einem TR-FRET-Messgerät, z.B. einem Rubystar (BMG Labtechnologies, Offenburg, Germany) oder einem Viewlux (Perkin-Elmer), die Fluoreszenz-Emissionen bei 620 nm and 665 nm nach Anregung bei 350 nm gemessen. Das Verhältnis der Emissionen bei 665 nm und 622 nm wurde als Maß für die Menge des phosphorylierten Substrates genommen. Die Daten wurden normalisiert (Enzymreaktion ohne Testsubstanz = 0 % Inhibition, alle anderen Assaykomponenten aber kein Enzym = 100 % Inhibition). Üblicherweise wurden die Testsubstanzen auf derselben Mikrotiterplatte bei 11 verschiedenen Konzentrationen im Bereich von 20 µM bis 0,073 nM getestet (20 µM, 5,7 µM, 1,6 µM, 0,47 µM, 0,13 µM, 38 nM, 11 nM, 3,1 nM, 0,89 nM, 0,25 nM und 0,073 nM). Die Verdünnungsreihen wurden vor dem Assay hergestellt (2 mM bis 7,3 nM in 100 % DMSO) durch serielle Verdünnungen. Die IC₅₀-Werte wurden kalkuliert mit einem 4-Parameter-Fit.

**Tabelle 1: ICso-Werte der Beispielverbindungen im IRAK4 Kinase Assay**

| Beispiel | IC₅₀ [nM] |
|---|---|
| 1 | 30.6 |
| 2 | 135.6 |
| 3 | 7.2 |
| 4 | 52.7 |
| 5 | 264.5 |
| 6 | 35.7 |
| 7 | 867.3 |
| 8 | 15.0 |
| 9 | 103.8 |
| 10 | 18.5 |
| 11 | 3.4 |
| 12 | 10.7 |
| 13 | 1.3 |
| 14 | 10.8 |
| 15 | 12.3 |
| 16 | 21.5 |
| 17 | 36.0 |
| 18 | 47.5 |
| 19 | 8.9 |
| 20 | 13.3 |
| 21 | 117.2 |
| 22 | 3.7 |

Die inhibitorische Aktivität der erfindungsgemäßen Substanzen der allgemeinen Formel (III) an IRAK4 wurde ebenfalls in dem oben beschriebenen IRAK4-TR-FRET-Assay gemessen. Exemplarisch sind die Verbindung Intermediat 4-2 mit einem IC₅₀ = 21.7 nM, Intermediat 4-3 mit einem IC₅₀ = 13.0 nM und Intermediat 4-4 mit einem IC₅₀ = 6.2 nM genannt.

### TNF-α Ausschüttung in THP-1 Zellen

Mithilfe dieses Tests können Substanzen auf ihre Fähigkeit hin getestet werden, TNF-α (Tumornekrosefaktor-alpha) Ausschüttung in THP-1 Zellen (humane monozytische akute Leukämie-Zellinie) zu inhibieren. TNF-α ist ein Zytokin, welches in inflammatorischen Prozessen beteiligt ist. Die TNF-α Ausschüttung wird in diesem Test durch Inkubation mit bakteriellem Lipopolysaccharid (LPS) ausgelöst.

THP-1 Zellen wurden in kontinuierlicher Suspensions-Zellkultur [RPMI 1460 Medium mit L-Glutamax (Gibco, Kat.-Nr. 61870-044) supplementiert mit foetalem Kälberserum (FCS) 10% (Invitrogen, Kat.-Nr. 10082-147), 1% Penicillin/Streptomycin (Gibco BRL, Kat.-Nr. 15140-114)] gehalten und sollten eine Zellkonzentration von 1×10⁶ Zellen/ml nicht überschreiten. Der Assay erfolgte im Zellkulturmedium (RPMI 1460 Medium mit L-Glutamax supplementiert mit FCS 10%).

Jeweils 2-2.5 µl der Zellsuspension (entspricht 4000 Zellen) wurden pro well in eine 384-well Testplatte Greiner Kat.-Nr. 784076) dispensiert, in der sich jeweils 40-50 nl Substanz in 100% DMSO gelöst befanden. Hierbei wurden jeweils 10 verschiedene Konzentrationen im Bereich von 20 µM bis 0,073 nM je Substanz eingesetzt. Die Zellen wurden 15 min bei Raumtemperatur inkubiert. Anschließend wurden 2-2.5 µl pro well 0.1 µg/ml LPS (Sigma, *Escherichia coli* 055:B5, Kat.-Nr. L5418) gelöst in Zellkulturmedium dispensiert (finale Konzentration 0.05 µg/ml). Als Neutralkontrolle wurden Zellen mit 0.05 µg/ml LPS und 1% DMSO und als Inhibitorkontrolle nur mit 1% DMSO behandelt.

Die Platten wurden 30 sec bei 80g zentrifugiert und 17 h bei 37°C, 5% CO₂ und 95% Luftfeuchtigkeit inkubiert. Zur Bestimmung der Menge an TNF-α wurde der TNF-alpha HTRF Detection Kit (Cisbio, Kat.-Nr. 62TNFPEB/C) verwendet. Hierzu wurden jeweils 2 µl der Detektionslösung, bestehend aus Anti-TNF-α-XL665 Konjugat und Anti-TNF-α-Kryptat Konjugat gelöst nach Anweisung des Herstellers im Rekonstitutionspuffer, für den HTRF (Homogeneous Time-Resolved Fluorescence) Test zugegeben. Nach Zugabe wurde entweder 3 h bei Raumtemperatur oder über Nacht bei 4°C inkubiert. Anschließend wurden die Signale mit einem HTRF-fähigen Messgerät wie dem BMG PheraStar bei 620/665nm ausgelesen.

Die Wirkung der Substanzen wird als Verhältnis zwischen Neutral- und Inhibitorkontrolle in Prozent ausgedrückt. Die IC₅₀-Werte wurden mit einem 4-Parameter-Fit kalkuliert.

**Tabelle 2: ICso-Werte der Beispielverbindungen bezüglich der TNF-α Ausschüttung in THP-1 Zellen**

| Beispiel | IC₅₀ [µM] |
|---|---|
| 1 | 1.0 |
| 2 | 15.1 |
| 3 | 0.7 |
| 4 | 5.6 |
| 5 | 5.4 |
| 6 | 0.9 |
| 7 | 16.4 |
| 8 | 1.0 |
| 9 | 6.5 |
| 10 | 1.0 |
| 11 | 0.2 |
| 12 | 0.3 |
| 13 | 0.1 |
| 14 | 0.2 |
| 15 | 0.2 |
| 16 | 0.2 |
| 17 | 0.5 |
| 18 | 0.3 |
| 19 | 0.1 |
| 20 | 0.2 |
| 21 | 1.8 |

### In vitro LPS (Lipopolysaccharide)-induzierte Zytokinproduktion in humanen PBMCs (peripheral blood mononuclear cells)

Die Wirkung der erfindungsgemäßen Verbindungen der allgemeinen Formel (I) auf die induzierte Zytokinproduktion in humanen PBMCs wurde untersucht. Hierbei erfolgte die Induktion der Zytokinproduktion durch LPS, einen TLR4 Liganden, welcher zur Aktivierung des IRAK4-vermittelten Signalweges führt.

Die Gewinnung der humanen PBMCs erfolgte aus anti-koaguliertem humanem Vollblut. Hierzu wurde in Leucosep-Röhrchen 15 ml Ficoll-Paque (Biochrom, Kat.-Nr. L6115) vorgelegt und 20 ml Humanblut hinzugefügt. Nach Zentrifugation des Blutes bei 800g für 15 min bei Raumtemperatur wurde Plasma inklusive der Thrombozyten abgenommen und verworfen. Die PBMCs wurden in Zentrifugenröhrchen überführt und mit PBS (phosphatgepufferte Salzlösung) (Gibco, Kat.-Nr. 14190) aufgefüllt. Die Zellsuspension wurde bei 250g für 10 min bei Raumtemperatur zentrifugiert und der Überstand verworfen. Die Resuspension der PBMCs erfolgte in Komplettmedium (RPMI 1640, ohne L-Glutamin (PAA, Kat.-Nr. E15-039), 10% FCS; 50 U/ml Penicillin, 50 µg/ml Streptomycin (PAA, Kat.-Nr. P11-010) und 1% L-Glutamin (Sigma, Kat.-Nr. G7513)).

Auch der Assay erfolgte in Komplettmedium. Die PBMCs wurden in 96-well Platten mit einer Zelldichte von 2.5×10⁵ Zellen/well ausgesät. Die erfindungsgemäßen Verbindungen wurden in einem konstanten Volumen von 100% DMSO seriell verdünnt und in dem Assay mit 8 verschiedenen Konzentrationen im Bereich von 10 µM bis 3 nM so eingesetzt, so dass die finale DMSO Konzentration 0.4% DMSO betrug. Die Zellen wurden damit für 30 min vor der eigentlichen Stimulation vorinkubiert. Zur Induktion der Zytokinsekretion erfolgte eine Stimulation mit 0.1 µg/ml LPS (Sigma, *Escherichia coli* 0128:B12, Kat.-Nr. L2887) für 24 Stunden. Die Bestimmung der Zellviabilität erfolgte unter Verwendung des CellTiter-Glo Luminescent Assay (Promega, Kat.-Nr. G7571 (G755/G756A)) nach Anweisung des Herstellers. Die Bestimmung der Menge an sekretiertem TNF-α im Zellkulturüberstand erfolgte mittels Human Prolnflammatory 9-Plex Tissue Culture Kit (MSD, Kat.-Nr. K15007B) nach Anweisung des Herstellers. Exemplarisch sind die Beispielverbindung 11 und die Beispielverbindung 12 mit einer Aktivität ≤ 1µM genannt.

### In vitro TLR-4/TLR-7-induzierte Interleukin (IL)-23 Sekretion von humanen Dendritischen Zellen (DCs)

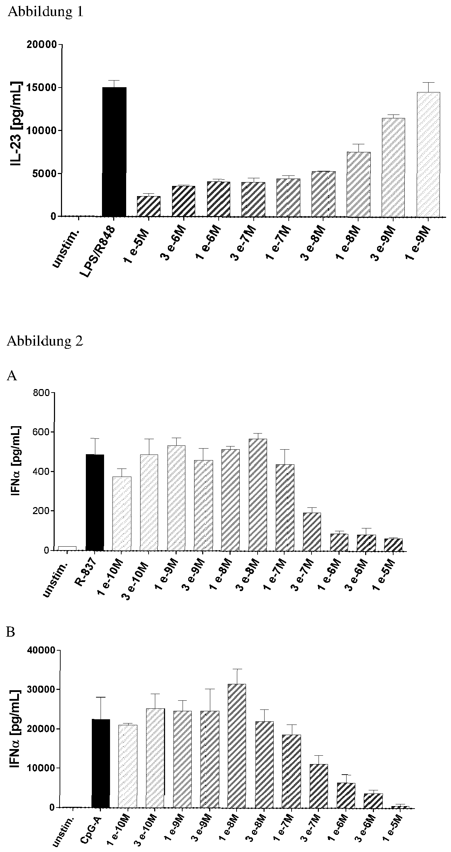

Die Wirkung der erfindungsgemäßen Verbindungen der allgemeinen Formel (I) auf die induzierte Produktion des pro-inflammatorischen Zytokins IL-23, welches eine essentielle Rolle für die Generation von TH-17 Zellen spielt, wurde in humanen DCs untersucht. Es wird beschrieben, dass TH-17 Zellen eine entscheidende Rolle in der Pathogenese von Erkrankungen wie Rheumatoide Arthritis, Psoriatrische Arthritis, Morbus Bechterew (Ankylosing Spondylitis) oder auch Multiple Skerose spielen (Lubberts, Nat. Rev. Rheumatol., 2015; Marinoni et al., Auto. Immun. Highlights, 2014; Isailovic et al., J. Autoimmun., 2015; Staschke et al., J Immunol., 2009). Zum Nachweis der Wirkung der erfindungsgemäßen Verbindungen auf die IL-23 Produktion wurden humane primäre Monozyten (isoliert aus humanen PBMCs mit Hilfe magnetischer Separation [Miltenyi Biotech, Monocyte Isolation Kit, Kat.-Nr. 130-091-153] unter Zusatz von Wachstumsfaktoren (rekombinantes humanes GM-CSF [PeproTech, Kat.-Nr. 300-03] und IL-4 [PeproTech, Kat.-Nr. 200-04]) in Komplettmedium (VLE (very low endotoxin) RPMI 1640 [Biochrom AG, Kat.-Nr. FG1415], 10% Fetal Bovine Serum (FBS) [Gibco, Kat.-Nr. 10493-106]; 50 µM β-Mercaptoethanol (Gibco, Kat.-Nr. 31350], 50 U/ml Penicillin und Streptomycin [Gibco, Kat.-Nr. 15140-114]) über 6 Tage in Kultur zu Des differenziert. Nach erfolgter Ernte der Des wurden diese im Komplettmedium resuspendiert und in einer Zelldichte von 2×10⁵ Zellen/well in einer 96-well Platten (Costar, Kat.-Nr. 3599) ausgesät. Die erfindungsgemäßen Verbindungen wurden in einem konstanten Volumen von 100% DMSO seriell verdünnt und in dem Assay mit 9 verschiedenen Konzentrationen im Bereich von 10 µM bis 1 nM eingesetzt. Hierbei wurde darauf geachtet, dass für jede der eingesetzten 9 Konzentrationen die enthaltene DMSO Konzentration immer 0.1% DMSO betrug. Es erfolgte eine 30minütige Vorinkubation der Des mit den erfindungsgemäßen Verbindungen. Hiernach wurden dann die DCs mittels 10ng/ml LPS (Sigma, *Escherichia coli* Serotyp 0127:B8, Kat.-Nr. L3129) (TLR4 Ligand) und 2,5µg/ml des TLR-7/8-Ligand R848 (Invivogen, Kat.-Nr. tlrl-r848-5), welche beide die Aktivierung des IRAK4-vermittelten Signalweges bewirken, für 24 Stunden im Brutschrank (37°C, 95%rH, 5% COz) zur IL-23 Produktion stimuliert. Nach dieser 24-stündigen Inkubationszeit wurden die Überstände abgenommen und mit Hilfe eines kommerziell erhältlichen hIL-23 ELISAs (eBiosciences, Kat.-Nr. 88-7237-88), der nach Herstellerangaben durchgeführt wurde, analysiert. Die Ergebnisse der Hemmung von IL-23 in humanen Des sind beispielhaft für die Beispielverbindung 12 in Abbildung 1 gezeigt.

### In vitro TLR-7/8 bzw. TLR-9-induzierte IFNα Produktion von humanen plasmazytoiden Dendritischen Zellen (pDCs)

Mit Hilfe dieses Tests kann die Wirkung der erfindungsgemäßen Verbindungen der allgemeinen Formel (I) auf die Produktion von IFNα (Interferon-alpha), ein Schlüsselzytokin in der Pathogenese des Systemischen Lupus Erythematodes (Mathian et al., Arthritis Rheum, 2009; Crow M.K., Rheum Dis Clin N Am, 2010), in humanen pDCs untersucht werden. Hierzu wurden wie oben beschrieben humane PBMCs aus Vollblut isoliert und nachfolgend die plasmazytoiden Des (pDCs) aus diesen mit Hilfe eines kommerziell erhältlichen Zellseparations-Kits (Miltenyi Biotech, Plasmacytoid Dendritic Cell Isolation Kit II, Kat.-Nr. 130-097-415) isoliert. Die so erhaltenen pDCs wurden in Komplettmedium (RPMI 1640 + GlutaMax [*Gibco,* Kat.-Nr. 61870-010] supplementiert mit 10% FBS [Gibco, Kat.-Nr. 10493-106] und 50 U Penicillin/Streptomycin [Gibco, Kat.-Nr. 15140-114]) resuspendiert und in einer Zelldichte von 5×10⁴ Zellen/well in einer 96er-Mikrotiterplatte (Costar, Kat.-Nr. 3599) ausplattiert. Die erfindungsgemäßen Verbindungen wurden in einem konstanten Volumen von 100% DMSO seriell verdünnt und in dem Assay mit 9 verschiedenen Konzentrationen im Bereich von 10 µM bis 1 nM eingesetzt. Hierbei wurde darauf geachtet, dass für jede der eingesetzten 9 Konzentrationen die enthaltene DMSO Konzentration immer 0.1% DMSO betrug. Es erfolgte eine 30minütige Vorinkubation der pDCs mit den erfindungsgemäßen Verbindungen. Die Stimulation der pDCs erfolgte entweder mit einem TLR7/8 Liganden (Imiquimod, R837, Invivogen, Kat.-Nr. tlrl-imq) oder mit einem TLR-9 Liganden (CPG-A, ODN2216, Invivogen, Kat.-Nr. tlrl-2216-1) und führte zur Aktivierung der IRAK4-vermittelten Signalwege. Nach 24-stündiger Inkubation wurden die Zellkulturüberstände entnommen und mittels eines kommerziell erhältlichen humanen IFNα ELISAs (IFNalpha Multi-Subtype ELISA Kit, pbl Assay Science, Kat.-Nr. 41105-1) analysiert. Die Ergebnisse der Hemmung von IFNα in humanen plasmazytoiden Des sind beispielhaft für die Beispielverbindung 12 in Abbildung 2 gezeigt.

### In vivo TLR-vermitteltes Inflammationsmodel

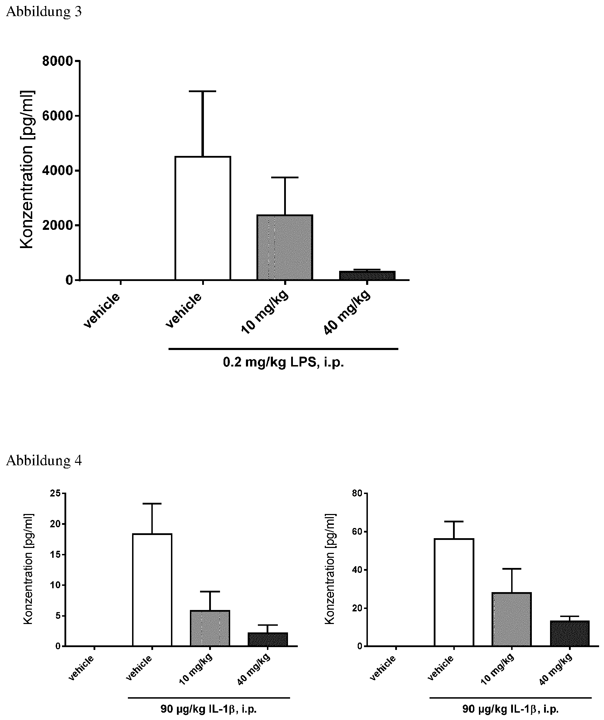

Die erfindungsgemäßen Verbindungen der allgemeinen Formel (I) wurden in einem *in vivo* TLR-vermittelten Inflammationsmodell bezüglich ihrer *in vivo* Wirksamkeit untersucht. Dieses mechanistische Modell zeigt insbesondere die potentielle Wirkung der erfindungsgemäßen Verbindungen auf TLR4-vermittelte Erkrankungen, da ein LPS-vermitteltes Inflammationsmodell verwendet wurde. Hierbei wurden weibliche Balb/c Mäuse (ca. 8 Wochen alt; Charles River Laboratories, Deutschland) in Gruppen von jeweils 5 Tieren aufgeteilt. Die Kontrollgruppe wurde mit dem Vehikel, in welchem die Substanz gelöst wurde (Substanzvehikel), als auch mit dem Vehikel, in welchem das LPS gelöst wurde, behandelt. Neben den Substanzbehandlungsgruppen wurde auch der Positivkontrollgruppe jeweils 0,2 mg LPS/kg Körpergewicht (Sigma, Kat.-Nr. L4391) (Lipopolysaccharides from *E. coli* 0111:B4) intraperitoneal (i.p.) verabreicht. Die Positivkontrollgruppe erhielt außerdem das oben beschriebene Substanzvehikel. Die Substanzgabe erfolgte oral 16 Stunden vor Induktion der Inflammation durch die Gabe von LPS. Zur Untersuchung der Wirkung der erfindungsgemäßen Verbindungen auf die Inflammation erfolgte bei den Tieren nach 1,5 Stunden die finale Blutentnahme. Die Bestimmung der Konzentration bestimmter Zytokine erfolgte im Plasma durch Verwendung des Mouse Prolnflammatory 7-Plex Tissue Culture Kit (MSD, Kat.-Nr. K15012B) nach Anweisung des Herstellers. IRAK4 Inhibitoren sind effektiv im TLR-vermittelten Inflammationsmodel. Abbildung 3 zeigt die Menge an TNF-α im Plasma, die im Vergleich zur LPS-induzierten Konzentration durch die Gabe von Beispielverbindung 11 Dosis-abhängig reduziert ist.

### In vivo IL-1β-vermitteltes Inflammationsmodel

Zur Erfassung der potentiellen Wirksamkeit der erfindungsgemäßen Verbindungen der allgemeinen Formel (I) in IL-1β-vermittelten Erkrankungen, wurde weiblichen Balb/c Mäusen (ca. 8 Wochen, Charles River Laboratories, Deutschland) IL-1β i.p. appliziert und die Wirkung der erfindungsgemäßen Verbindungen auf die IL-1β-vermittelte Zytokinsekretion untersucht. Die Gruppengröße betrug jeweils 5 Tiere. Die Kontrollgruppe wurde mit den Vehikeln, welche zur Lösung der Substanz und des IL-1β eingesetzt wurden, behandelt. Den Substanzbehandlungsgruppen und der Positivkontrollgruppe wurde jeweils 90 µg IL-1β /kg Körpergewicht (R&D, Kat.-Nr. 401-ML/CF) i.p verabreicht. Die Substanz bzw. dessen Vehikel in der Positivkontrollgruppe wurde 6 Stunden vor der IL-1β Verabreichung appliziert. Die Bestimmung von TNF-α im Plasma nach finaler Blutentnahme erfolgte 2 Stunden nach Verabreichung des IL-1β mittels des Mouse ProInflammatory 7-Plex Tissue Culture Kit (MSD, Kat.-Nr. K15012B) nach Anweisung des Herstellers. Die Gabe von IL-1β führt zu einer erhöhten Konzentration an TNF-α im Plasma, welches durch die Behandlung mit den Beispielverbindungen 11 und 12 inhibiert wird. Dies wird durch Abbildung 4 verdeutlicht.

### In vivo Adjuvanz-induziertes Arthritismodell

Zur Ermittlung der anti-inflammatorischen Aktivität der erfindungsgemäßen Verbindungen der allgemeinen Formel (I) wurden diese in einem Arthritismodel hinsichtlich ihrer *in vivo* Wirksamkeit untersucht. Hierzu wurden männlichen Lewis-Ratten (ca. 100-125g, Charles River Laboratories, Deutschland) je 100µl einer Complete-Freund'schen Adjuvanz (CFA)-Lösung (M. *tuberculosis* H37Ra [Difo Lab, Kat.-Nr. -231141] gelöst in Incomplete Freund*'*schen Adjuvanz [Difco Lab, Kat.-Nr. -263910]) subkutan in die Schwanzwurzel an Tag 0 appliziert. Die Gruppengröße betrug jeweils n= 8 Ratten. Es wurde sowohl eine gesunde Kontrollgruppe als auch eine Erkrankungs-Kontrollgruppe im Experiment mitgeführt. Beide Kontrollgruppen wurden jeweils nur mit dem Vehikel der Prüfsubstanz p.o. behandelt. Die Behandlung mit unterschiedlichen Dosierungen der Prüfsubstanz wurde präventiv, d.h. ab Tag 0, per oraler Gabe durchgeführt. An Tag 0 wurde zudem die Ausgangssituation der Tiere bezüglich des Disease Activity Scores (Bewertung des Schweregrads der Arthritis anhand eines Punktesystems) bestimmt. In diesem Punktesystem (Score) werden je nach Ausmaß der Gelenkentzündung Punkte von 0 bis 4 für das Vorhandensein eines Erythems inklusive einer Gelenkschwellung (0= keine; 1=leicht; 2= moderat; 3=deutlich; 4=massiv) für beide Hinterpfoten vergeben und addiert. Zur Ermittlung der anti-entzündlichen Wirksamkeit der Verbindungen wurde ab Tag 8, an dem die Tiere erstmals Zeichen einer Arthritis zeigen, und weiterfolgend 3 Mal in der Woche der Erkrankungsstatus der Tiere mittels Disease Activity Score bis zum Ende (Tag 20) bewertet. Die statistische Analyse erfolgte unter Verwendung der einfaktoriellen Varianzanalyse (ANOVA; Analysis of Variance) und dem Vergleich zur Kontrollgruppe mittels multipler Vergleichsanalyse (Dunnett-Test).

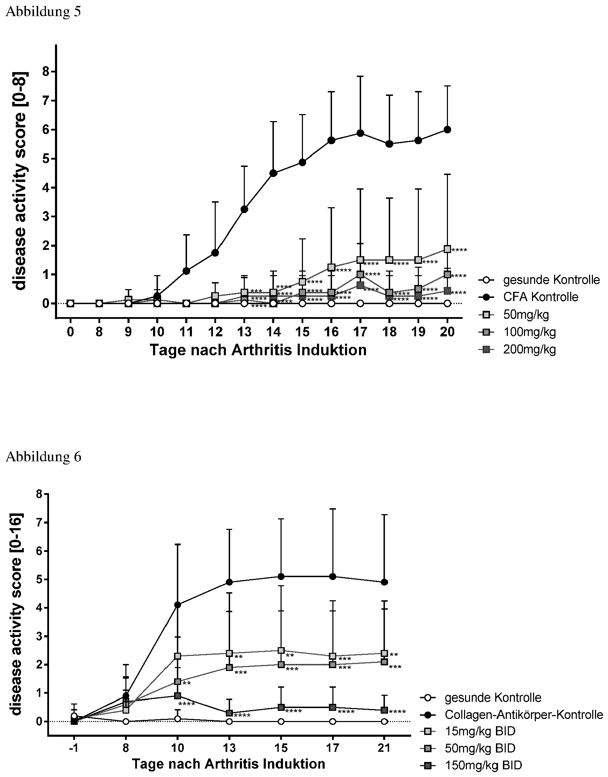

Die s.c. Applikation von CFA in Ratten führt zu einer akuten Arthritis mit deutlicher Gelenkentzündung in Ratten. Diese induzierte Arthritis konnte durch die Behandlung mit der Beispielverbindung 11 inhibiert werden. Dies wird durch Abbildung 5 verdeutlicht.

### In vivo Collagen-Antikörper-induziertes Arthritismodel in der Maus

Die anti-inflammatorische Wirkung der erfindungsgemäßen Verbindungen der allgemeinen Formel (I) wurde in einem weiteren murinen Arthritismodell untersucht. Hierzu wurden weibliche Balb/c Mäuse (ca. 9 Wochen alt; Charles River Laboratories, Kingston, Kanada) an Tag 0 je 200µl eines Collagen-Antikörper-Cocktails (10mg/ml; ArthritoMab, MD Bioproducts) intravenös in die Schwanzvene injiziert (mit Ausnahme der mitgeführten gesunden Kontrollgruppe). An Tag 6 erhielten diese Mäuse dann eine weitere intraperitoneale Injektion mit je 200µl LPS. Die Gruppengröße betrug jeweils n= 10 Mäuse. Es wurde sowohl eine gesunde Kontrollgruppe als auch eine Erkrankungs-Kontrollgruppe im Experiment mitgeführt. Beide Kontrollgruppen wurden jeweils nur mit dem Vehikel der Prüfsubstanz p.o. behandelt. Die Behandlung mit unterschiedlichen Dosierungen der Prüfsubstanz wurde präventiv, d.h. ab Tag 0, per oraler Gabe durchgeführt. Im Verlauf des Experimentes wurde das Ausmaß der Erkrankung anhand des Punktevergabesystems für den Disease Activity Score an allen vier Pfoten bewertet. Diese Punktevergabe sieht vor, dass für eine gesunde Pfote keine Punkte vergeben werden, wohingegen für die jeweilige entstehende Ausdehnung der Gelenkentzündung von den Zehen über das Mittelfußgelenk bis hin zum Fußgelenk jeweils Punkte von 1 [milde Entzündung z.B. der Zehe(n)] bis 4 [starke Entzündung mit Ausdehnung über die gesamte Pfote] wie nachfolgend erklärt vergeben werden:
- 0 = normal
- 1 = Erythem und milde Schwellung begrenzt auf Mittelfuß (tarsal) oder Fußgelenk oder Zehen
- 2 = Erythem und milde Schwellung, welche sich vom Fußgelenk bis zum Mittelfuß (2 Segmente) ausdehnt
- 3 = Erythem und moderate Schwellung, welche sich vom Fußgelenk bis zu den metatarsal Gelenken ausdehnt
- 4 = Erythem und starke Schwellung, welche auf Fußgelenk, Fuß und Zehen übergreift

Für diesen Parameter wurde zuvor die Ausgangssituation einen Tag vor Start des Experimentes (Tag -1) ermittelt und nachfolgend ab Tag 8 dreimal pro Woche dieser Disease Activity Score bewertet. Die statistische Analyse erfolgte unter Verwendung der einfaktoriellen Varianzanalyse ANOVA und dem Vergleich zur Kontrollgruppe mittels multipler Vergleichsanalyse (Dunnett-Test).

Die i.v. Applikation eines Collagen-Antikörper-Cocktails inklusive der nachfolgenden i.p. Applikation von LPS in Mäusen führt zu einer akuten Arthritis mit deutlicher Gelenkentzündung in Mäusen. Diese induzierte Arthritis konnte durch die Behandlung mit der Beispielverbindung 12 inhibiert werden. Dies wird durch Abbildung 6 verdeutlicht.

### In vivo NASH Mausmodell

Zur experimentellen Induktion einer NASH wird 45 männlichen C57BL/6 Mäuse in einem Alter von 2 Tagen jeweils 200 µg Streptozotocin (STZ; Sigma-Aldrich, USA) subkutan injiziert. Ab einem Alter von 4 Wochen werden diese Tiere bis zum Versuchsende mit einer Hochfett-Diät (HFD; 57 kcal% Fett, Kat.-Nr. #HFD32 der Firma CLEA, Japan) ad libitum gefüttert. Mit einem Alter von 6 Wochen werden die Tiere randomisiert auf 3 Gruppen aufgeteilt (15 Tiere pro Gruppe). Während eine der Gruppen keine Behandlung erhält, werden die anderen 2 Gruppen entweder mit Vehikel oder der Prüfsubstanz über 4 Wochen täglich oral behandelt. Nach Abschluss der 4-wöchigen Behandlung werden alle Tiere unter Narkose schmerzfrei getötet, die Leber entnommen und für die histologische Untersuchung in Bouin's Lösung (H. Denk, "Fixierung histologischer Präparate". In: P. Böck (ed.): "Romeis Mikroskopische Technik", Urban & Schwarzenberg, München-Wien-Baltimore 1989, 17. Auflage, Seite 97, ISBN 3-541-11227-1) fixiert. Danach werden die Leberproben in Paraffin eingebettet und 5 µm dicke Paraffinschnitte hergestellt. Von jeder Leber werden histologische Schnitte a) für die Bestimmung des NAFLD Activity Scores (NAS) mit Hematoxylin-Eosin (HE), und b) für die Bestimmung der Leberfibrosierung mit Pikro-Sirusrot (Waldeck, Deutschland) gefärbt. Der NAFLD Activity Score wird modifiziert in Anlehnung an die empfohlenen Kriterien von D.E. Kleiner et al., Hepatology 41 (2005), 1313-1321 (Tabelle 1) an den Hematoxylin-Eosin Schnitten bestimmt. Für die histologische Quantifizierung von fibrotischen Flächen werden mikroskopisch bei 200facher Vergrößerung jeweils 5 digitale Fotos (DFC280; Leica, Deutschland) pro Schnitt angefertigt und mit Hilfe der ImageJ Software (National Institue of Health, USA) der prozentuale Anteil an Fibrose ermittelt.

### In vivo db/db Mausmodell

Es werden 30 männliche db/db Mäuse in einem Alter von 8 Wochen eingesetzt. Hierbei handelt es sich um ein anerkanntes Modell für Fettleibigkeit (Adipositas), Insulinresistenz und Diabetes Typ 2 (Aileen JF King; The use of animal models in diabetes research; British Journal of Pharmacology 166 (2012), 877-894). Während des Versuches erhalten die Tiere eine Standarddiät (RM1(E) 801492, SDS) und Leitungswasser ad libitum. Die Tiere werden randomisiert auf 3 Gruppen aufgeteilt (10 Tiere pro Gruppe) und über 6 Wochen oral mit der Prüfsubstanz behandelt. Den Tieren wird während des Untersuchungszeitraumes zu unterschiedlichen Zeitpunkten (vor Therapiebeginn, 3 Wochen nach Therapiebeginn und 2 Tage vor Therapieende) Blut zur Bestimmung von Parametern der Insulinsensitivität (z.B. HbA1c, Glucosegehalt, Insulingehalt) entnommen. Des Weiteren wird 1 Tag vor Therapiebeginn und 2 Tage vor Therapieende ein OGTT (oraler Glukosetoleranztest) durchgeführt, ebenfalls als Parameter zur Bestimmung der Insulinsensitivität. Zusätzlich wird der HOMA-IR Index (Nüchtern-Insulinspiegel (mUll) * Nüchtern-Glukosespiegel (mmol/1) / 22,5) berechnet.

### In vivo B-Zell-Lymphom-assoziierte Xenotransplantationsmodelle

Die Anti-Tumoraktivität der erfindungsgemäßen Verbindungen der allgemeinen Formel (I) wird in murinen Xenotransplantationsmodellen untersucht. Hierzu wird weiblichen C.B-17 SCID Mäusen subkutan Tumorzelllinien von humanen B-Zell-Lymphomen, z. B. TMD-8, implantiert. Bei einer mittleren Tumorgröße von 20-30 mm² beginnt die orale monotherapeutische Behandlung mit einer erfindungsgemäßen Verbindung oder die Behandlung durch Verabreichung einer erfindungsgemäßen Verbindung in Kombination mit einer Standardtherapie, welche jeweils oral verabreicht werden. Zuvor erfolgt jedoch eine Randomisierung der Tiere. Die Behandlung wird beendet, sobald die unbehandelte Kontrollgruppe große Tumore aufweist. Die Tumorgröße als auch das Körpergewicht werden dreimal wöchentlich bestimmt. Abnahmen im Körpergewicht stellen ein Maß für eine behandlungsbedingte Toxizität dar (>10% = kritisch, Behandlungsstopp bis zur Genesung, >20% toxisch, Beendigung). Die Tumorfläche wird mittels elektronischer Messschieber erfasst [Länge (mm) × Breite (mm)]. Am Ende der Studie wird außerdem das Tumorgewicht bestimmt. Die Anti-Tumor-Wirksamkeit definiert das Verhältnis des Tumorgewichts von Behandlung vs. Kontrolle (T/C) [Tumorgewicht der Behandlungsgruppe am Tag x/Tumorgewicht der Kontrollgruppe am Tag x] oder das Verhältnis der Tumorfläche von Behandlung vs. Kontrolle [Tumorfläche der Behandlungsgruppe am Tag x/Tumorfläche der Kontrollgruppe am Tag x]. Eine Verbindung mit einem T/C größer gleich 0.5 wird als aktiv (wirksam) definiert. Die statistische Analyse erfolgt unter Verwendung der einfaktoriellen ANOVA und dem Vergleich zur Kontrollgruppe mittels paarweiser Vergleichsanalyse (Dunnett-Test).

**Abbildung 1****:** Hemmung von IL-23 in humanen Monozyten-generierten Des für die Beispielverbindung 12. Daten sind als Mittelwerte mit Standardabweichungen dargestellt.

**Abbildung 2****:** Inhibition von INF-α in (A) Imiquimod (R837)- bzw. (B) CpG-A-stimulierten humanen plasmazytoiden Des für die Beispielverbindung 12. Daten sind als Mittelwerte mit Standardabweichungen dargestellt.

**Abbildung 3****:** Die Behandlung von LPS-induzierter Entzündung mit Beispielverbindung 11 führt zu einer verringerten Menge an sekretiertem TNF-α. Daten sind als Mittelwerte mit Standardabweichungen dargestellt.

**Abbildung 4****:** Die Behandlung einer IL-1β-induzierten Entzündung mit den Beispielverbindungen 11 (links) und 12 (rechts) führt zu einer dosisabhängigen Verringerung der Menge an sekretiertem TNF-α. Daten sind als Mittelwerte mit Standardabweichungen dargestellt.

**Abbildung 5****:** Anti-entzündliche Effekte der Beispielverbindung 11 im Tiermodell der Rheumatoiden Arthritis (Adjuvanz-induziertes Rattenmodell). Signifikante und dosis-abhängige Hemmung der rheumatischen Gelenkentzündung gemessen anhand des Disease Activity Scores. Die Daten entsprechen den Mittelwerten + Standardabweichungen. Einfaktorielle Varianzanalyse ANOVA mit nachgeschalteter multipler Vergleichsanalyse zur CFA-Kontrollgruppe mittels Dunnet*'*s Test; ^{∗}p <0.05; ^{∗∗}p <0.01;^{∗∗∗}p <0.001; ^{∗∗∗∗}p <0.0001.

**Abbildung 6****:** Anti-entzündliche Effekte der Beispielverbindung 12 im Tiermodell der Rheumatoiden Arthritis (Collagen-Antikörper-induziertes Mausmodell). Signifikante und dosis-abhängige Hemmung der rheumatischen Gelenkentzündung gemessen anhand des Disease Activity Scores. Die Daten entsprechen den Mittelwerten + Standardabweichungen. Die statistischen Signifikanzen zwischen Collagen-Antikörper(AK)-Kontrolle und den Behandlungsgruppen wurde mittels einfaktorieller Varianzanalyse ANOVA mit nachgeschalteter multipler Vergleichsanalyse (Dunnet*'*s Test) berechnet (^{∗}p <0.05; ^{∗∗}p <0.01;^{∗∗∗}p <0.001; ^{∗∗∗∗}p <0.0001).

## Patentansprüche

1. Verbindungen der allgemeinen Formel (I) 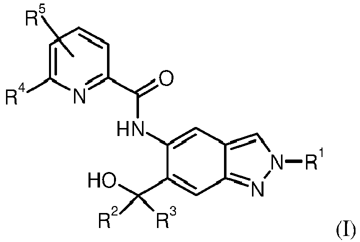 in der:
R¹ für C₁-C₆-Alkyl steht, wobei der C₁-C₆-Alkylrest unsubstituiert oder ein- oder mehrfach, gleich oder verschieden substituiert ist mit
Halogen, Hydroxy, einem unsubstituierten oder ein- oder mehrfach mit Halogen substituierten C₃-C₆-Cycloalkyl, einem Rest R⁶, R⁷SO₂, R⁷SO oder R⁸O
oder für eine Gruppe steht, ausgewählt aus: 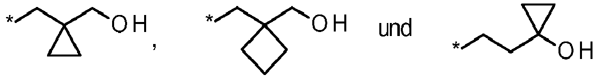
wobei * für die Verknüpfungsstelle der Gruppe mit dem Rest des Moleküls steht;
R² und R³ immer dieselbe Bedeutung haben und gleichzeitig entweder für Wasserstoff oder C₁-C₆-Alkyl stehen;
R⁴ für Halogen, Cyano, ein unsubstituiertes oder ein- oder mehrfach, gleich oder verschieden voneinander substituiertes C₁-C₆-Alkyl oder ein unsubstituiertes oder ein- oder mehrfach, gleich oder verschieden voneinander substituiertes C₃-C₆-Cycloalkyl steht, und die Substituenten ausgewählt sind aus der Gruppe Halogen und Hydroxy;
R⁵ für Wasserstoff, Halogen oder ein unsubstituiertes oder ein- oder mehrfach mit Halogen substituiertes C₁-C₆-Alkyl steht;
R⁶ für einen unsubstituierten oder ein- oder zweifach mit Methyl substituierten monocyclischen, gesättigten Heterocyclus mit 4 bis 6 Ringatomen steht, der ein Heteroatom oder eine Heterogruppe aus der Reihe O, S, SO und SO₂ enthält;
R⁷ für C₁-C₆-Alkyl steht, wobei der C₁-C₆-Alkylrest unsubstituiert oder ein- oder mehrfach, gleich oder verschieden mit Halogen, Hydroxy oder C₃-C₆-Cycloalkyl substituiert ist; oder R⁷ steht für C₃-C₆-Cycloalkyl,
R⁸ für C₁-C₆-Alkyl steht, wobei der C₁-C₆-Alkylrest unsubstituiert oder ein- oder mehrfach, gleich oder verschieden mit Halogen substituiert ist;
und ihre Diastereomere, Enantiomere, ihre Metabolite, ihre Salze, ihre Solvate oder die Solvate ihrer Salze.

2. Verbindungen gemäß Anspruch 1, wobei
R¹ für C₁-C₆-Alkyl steht, wobei der C₁-C₆-Alkylrest unsubstituiert oder ein- oder mehrfach, gleich oder verschieden substituiert ist mit Fluor, Hydroxy, einem Rest R⁶, R⁷SO₂, R⁷SO oder R⁸O,
R² und R³ immer dieselbe Bedeutung haben und gleichzeitig entweder für Wasserstoff oder C₁-C₃-Alkyl stehen;
R⁴ für Halogen, Cyano oder C₁-C₃-Alkyl steht, wobei der C₁-C₃-Alkylrest unsubstituiert oder ein- oder mehrfach, gleich oder verschieden substituiert ist mit Halogen oder Hydroxy;
R⁵ für Wasserstoff, Fluor, Chlor oder C₁-C₃ Alkyl steht;
R⁶ für Oxetanyl oder Tetrahydrofuranyl steht;
R⁷ für C₁-C₄-Alkyl steht, wobei der C₁-C₄-Alkylrest unsubstituiert oder einfach mit Hydroxy oder mit Cyclopropyl oder mit drei Fluoratomen substituiert ist;
R⁸ für einen unsubstituierten C₁-C₄-Alkylrest oder einen dreifach mit Fluor substituierten C₁-C₄-Alkylrest steht.

3. Verbindungen gemäß Anspruch 1 oder 2, wobei R⁴ für Difluormethyl, Trifluormethyl oder Methyl steht.

4. Verbindungen gemäß einem Anspruch 1, 2 oder 3, wobei R⁵ Wasserstoff oder Fluor ist.

5. Verbindungen gemäß Anspruch 1, 2, 3 oder 4, wobei R² und R³ gleichzeitig entweder Wasserstoff oder Methyl bedeuten.

6. Verbindungen gemäß Anspruch 2, wobei
R¹ für C₂-C₆-Alkyl steht, wobei der C₂-C₆-Alkylrest unsubstituiert ist, oder der C₂-C₆-Alkylrest ein-, zwei- oder dreifach mit Fluor substituiert ist oder der C₂-C₆-Alkylrest einfach mit Hydroxy, R⁶, R⁷SO₂, oder R⁸O substituiert ist, oder R¹ für einen mit Oxetanyl subsituierter C₁-C₃ Alkylrest steht;
R² und R³ immer dieselbe Bedeutung haben und gleichzeitig entweder für Wasserstoff oder Methyl stehen;
R⁴ für einen unsubstituierten oder einen ein- oder mehrfach mit Halogen substituierten C₁-C₃-Alkylrest oder einen mit einer Hydroxygruppe substituierten C₁-C₃-Alkyl-rest oder einen mit einer Hydroxygruppe und drei Fluoratomen substituierten C₁-C₃-Alkylrest steht
R⁵ für Wasserstoff, Fluor oder C₁-C₃ Alkyl steht;
R⁷ für C₁-C₃-Alkyl steht;
R⁸ für C₁-C₄-Alkyl steht, wobei der C₁-C₄-Alkylrest unsubstituiert oder ein-, zwei- oder dreifach mit Fluor substituiert ist.

7. Verbindungen gemäß Anspruch 6, worin
R¹ für einen C₂-C₅-Alkylrest, der mit Hydroxy oder C₁-C₃-Alkoxy oder Trifluormethoxy oder 2,2,2-Trifluorethoxy oder Trifluormethyl substituiert ist oder für einen mit Methyl-SOz substituierten C₂-C₄-Alkylrest oder für einen mit Oxetan-3-yl substituierter C₁-C₂ Alkylrest steht;
R² und R³ immer dieselbe Bedeutung haben und gleichzeitig für Wasserstoff oder Methyl stehen;
R⁴ für Methyl, Ethyl, Trifluor-C₁-C₃-alkyl, Difluor-C₁-C₃-Alkyl, Hydroxymethyl, 1-Hydroxyethyl, 2-Hydroxypropan-2-yl und 2,2,2-Trifluor-1-hydroxyethyl steht;
R⁵ für Wasserstoff, Fluor oder Methyl steht.

8. Verbindungen gemäß Anspruch 7, worin
R¹ für 4,4,4-Trifluorbutyl, 3-Hydroxy-3-methylbutyl, 3-Hydroxybutyl, 3-Methoxypropyl, 3-Hydroxypropyl, 3-Hydroxy-2-methylpropyl, 3-Hydroxy-2,2-dimethylpropyl, 3-Trifluormethoxypropyl, 2-Methoxyethyl, 2-Hydroxyethyl, 2-(Methylsulfonyl)ethyl oder 3-(Methylsulfonyl)propyl steht;
R² und R³ gleichzeitig für Methyl oder Wasserstoff stehen;
R⁴ für Difluormethyl, Trifluormethyl oder Methyl steht;
R⁵ für Wasserstoff oder Fluor steht.

9. Verbindungen gemäß Anspruch 8, worin
R¹ für 3-Hydroxy-3-methylbutyl, 3-Hydroxybutyl, 3-Hydroxy-2-methylpropyl, 3-Hydroxy-2,2-dimethylpropyl, 3-(Methylsulfonyl)propyl oder 2-(Methylsulfonyl)ethyl steht;
R² und R³ gleichzeitig für Methyl stehen;
R⁴ für Difluormethyl oder Trifluormethyl steht;
R⁵ für Wasserstoff steht.

10. Verbindungen gemäß Anspruch 8, worin
R¹ für 3-Hydroxy-3-methylbutyl, 3-Hydroxybutyl, 3-Hydroxy-2-methylpropyl, 3-Hydroxy-2,2-dimethylpropyl, 3-(Methylsulfonyl)propyl oder 2-(Methylsulfonyl)ethyl steht;
R² und R³ gleichzeitig für Methyl stehen;
R⁴ für Methyl steht;
R⁵ für Fluor steht, wobei sich R⁵ in ortho-Position zu R⁴ befindet.

11. Verbindungen gemäß Anspruch 1-10 nämlich:
1) N-[6-(2-Hydroxypropan-2-yl)-2-(2-methoxyethyl)-2H-indazol-5-yl]-6-(trifluormethyl)pyridin-2-carboxamid
2) N-[6-(Hydroxymethyl)-2-(2-methoxyethyl)-2H-indazol-5-yl]-6-(trifluormethyl)pyridin-2-carboxamid
3) N-[6-(2-Hydroxypropan-2-yl)-2-(3-methoxypropyl)-2H-indazol-5-yl]-6-(trifluormethyl)pyridin-2-carboxamid
4) N-[6-(Hydroxymethyl)-2-(3-methoxypropyl)-2H-indazol-5-yl]-6-(trifluormethyl)pyridin-2-carboxamid
5) N-[2-(2-Hydroxyethyl)-6-(2-hydroxypropan-2-yl)-2H-indazol-5-yl]-6-(trifluormethyl)pyridin-2-carboxamid
6) N-[6-(2-Hydroxypropan-2-yl)-2-(3-hydroxypropyl)-2H-indazol-5-yl]-6-(trifluormethyl)pyridin-2-carboxamid
7) N-[2-(2-Hydroxyethyl)-6-(hydroxymethyl)-2H-indazol-5-yl]-6-(trifluormethyl)pyridin-2-carboxamid
8) N-[6-(2-Hydroxypropan-2-yl)-2-(oxetan-3-ylmethyl)-2H-indazol-5-yl]-6-(trifluormethyl)pyridin-2-carboxamid
9) N-[6-(Hydroxymethyl)-2-(oxetan-3-ylmethyl)-2H-indazol-5-yl]-6-(trifluormethyl)pyridin-2-carboxamid
10) N-{6-(2-Hydroxypropan-2-yl)-2-[3-(methylsulfonyl)propyl]-2H-indazol-5-yl}-6-(trifluormethyl)pyridin-2-carboxamid
11) N-[2-(3-Hydroxy-3-methylbutyl)-6-(2-hydroxypropan-2-yl)-2H-indazol-5-yl]-6-(trifluormethyl)pyridin-2-carboxamid
12) N- {6-(2-Hydroxypropan-2-yl)-2- [2-(methylsulfonyl)ethyl] -2H-indazol-5-yl} -6-(trifluormethyl)pyridin-2-carboxamid
13) 6-(Difluormethyl)-N-[2-(3-hydroxy-3-methylbutyl)-6-(2-hydroxypropan-2-yl)-2H-indazol-5-yl]pyridin-2-carboxamid
14) 6-(Difluormethyl)-N-{6-(2-hydroxypropan-2-yl)-2-[2-(methylsulfonyl)ethyl]-2H-indazol-5-yl}pyridin-2-carboxamid
15) 6-(Difluormethyl)-N-[6-(2-hydroxypropan-2-yl)-2-(3-hydroxypropyl)-2H-indazol-5-yl]pyridin-2-carboxamid
16) N-[6-(2-Hydroxypropan-2-yl)-2-(4,4,4-trifluorbutyl)-2H-indazol-5-yl]-6-(trifluormethyl)pyridin-2-carboxamid
17) N- {6-(2-Hydroxypropan-2-yl)-2-[3-(trifluormethoxy)propyl]-2H-indazol-5-yl} -6-(trifluormethyl)pyridin-2-carboxamid
18) N- {6-(2-Hydroxypropan-2-yl)-2- [3-(2,2,2-trifluorethoxy)propyl]-2H-indazol-5-yl}-6-(trifluormethyl)pyridin-2-carboxamid
19) 5-Fluor-N-[2-(3-hydroxy-3-methylbutyl)-6-(2-hydroxypropan-2-yl)-2H-indazol-5-yl]-6-methylpyridin-2-carboxamid
20) N-[2-(3-Hydroxy-3-methylbutyl)-6-(2-hydroxypropan-2-yl)-2H-indazol-5-yl]-6-methylpyridin-2-carboxamid
21) 6-(2-Hydroxypropan-2-yl)-N-[6-(2-hydroxypropan-2-yl)-2-(4,4,4-trifluorbutyl)-2H-indazol-5-yl]pyridin-2-carboxamid
22) N- {2- [2-(1-Hydroxycyclopropyl)ethyl] -6-(2-hydroxypropan-2-yl)-2H-indazol-5-yl}-6-(trifluormethyl)pyridin-2-carboxamid

12. Verbindung der allgemeinen Formel (I), wie in einem der Ansprüche 1 bis 11 definiert, zur Behandlung und/oder Prophylaxe von Krankheiten.

13. Verbindung der allgemeinen Formel (I), wie in einem der Ansprüche 1 bis 11 definiert, zur Verwendung in einem Verfahren zur Behandlung und/oder Prophylaxe von Tumorerkrankungen, dermatologischen Erkrankungen, gynäkologischen Erkrankungen, kardiovaskulären Erkrankungen, pulmonalen Erkrankungen, ophthalmologischen Erkrankungen, neurologischen Erkrankungen, Stoffwechselerkrankungen, Lebererkrankungen, inflammatorischen Erkrankungen, Autoimmunerkrankungen und Schmerz.

14. Verbindung der allgemeinen Formel (I), wie in einem der Ansprüche 1 bis 11 definiert, zur Verwendung in einem Verfahren zur Behandlung und/oder Prophylaxe von Lymphomen, Makuladegeneration, Psoriasis, Lupus erythematodes, Multipler Sklerosis, COPD, Gicht, NASH, Leberfibrose, Insulinresistenz, des metabolischen Syndroms, von Spondyloarthritiden und rheumatoider Arthritis, Endometriose sowie Endometriose-assoziierten Schmerzen und anderen Endometriose-assoziierten Symptomen wie Dysmenorrhoe, Dyspareunie, Dysurie und Dyschezie.

15. Verbindung der allgemeinen Formel (I), wie in einem der Ansprüche 1 bis 11 definiert, zur Verwendung in einem Verfahren zur Behandlung und/oder Prophylaxe von Schmerz inklusive akutem, chronischem, entzündlichem und neuropathischem Schmerz, vorzugsweise von Hyperalgesie, Allodynie, Schmerz bei Arthritis (wie Osteoarthritis, rheumatoider Arthritis und Spondyloarthritis), prämenstruellem Schmerz, Endometrioseassoziiertem Schmerz, postoperativem Schmerz, Schmerz bei interstitieller Zystitis, CRPS (komplexes regionales Schmerzsyndrom), Trigeminusneuralgie, Schmerz bei Prostatitis, Schmerzen verursacht durch Rückenmarksverletzungen, entzündungsinduziertem Schmerz, Kreuzschmerzen, Krebsschmerzen, Chemotherapie-assoziiertem Schmerz, HIV behandlungsinduzierter Neuropathie, Verbrennungs-induziertem Schmerz und chronischem Schmerz.

16. Verwendung einer Verbindung der allgemeinen Formel (I), wie in einem der Ansprüche 1 bis 11 definiert, zur Herstellung eines Arzneimittels.

17. Verwendung gemäß Anspruch 16, wobei das Arzneimittel zur Behandlung und/oder Prophylaxe von Tumorerkrankungen, dermatologischen Erkrankungen, gynäkologischen Erkrankungen, kardiovaskulären Erkrankungen, pulmonalen Erkrankungen, ophthalmologischen Erkrankungen, neurologischen Erkrankungen, Stoffwechselerkrankungen, Lebererkrankungen, inflammatorischen Erkrankungen, Autoimmunerkrankungen und Schmerz verwendet wird.

18. Verwendung gemäß der Ansprüche 16 und 17 zur Behandlung und/oder Prophylaxe von Lymphomen, Makuladegeneration, Psoriasis, Lupus erythematodes, Multipler Sklerosis, COPD, Gicht, NASH, Leberfibrose, Insulinresistenz, metabolisches Syndrom, Spondyloarthritiden und rheumatoider Arthritis, Endometriose sowie Endometriose-assoziierten Schmerzen und anderen Endometriose-assoziierten Symptomen wie Dysmenorrhoe, Dyspareunie, Dysurie und Dyschezie.

19. Verwendung gemäß der Ansprüche 16 und 17 zur Behandlung und/oder Prophylaxe von Schmerz inklusive akutem, chronischem, entzündlichem und neuropathischem Schmerz, vorzugsweise von Hyperalgesie, Allodynie, Schmerz bei Arthritis (wie Osteoarthritis, rheumatoider Arthritis und Spondyloarthritis), prämenstruellem Schmerz, Endometrioseassoziiertem Schmerz, postoperativem Schmerz, Schmerz bei interstitieller Zystitis, CRPS (komplexes regionales Schmerzsyndrom), Trigeminusneuralgie, Schmerz bei Prostatitis, Schmerzen verursacht durch Rückenmarksverletzungen, entzündungsinduziertem Schmerz, Kreuzschmerzen, Krebsschmerzen, Chemotherapie-assoziiertem Schmerz, HIV behandlungsinduzierter Neuropathie, Verbrennungs-induziertem Schmerz und chronischem Schmerz.

20. Arzneimittel enthaltend eine Verbindung der Formel (I), wie in einem der Ansprüche 1 bis 11 definiert, in Kombination mit einem inerten, nicht-toxischen, pharmazeutisch geeigneten Hilfsstoff.

21. Verfahren zur Herstellung der Verbindungen der allgemeinen Formel (III) aus den Verbindungen der allgemeinen Formel (II) 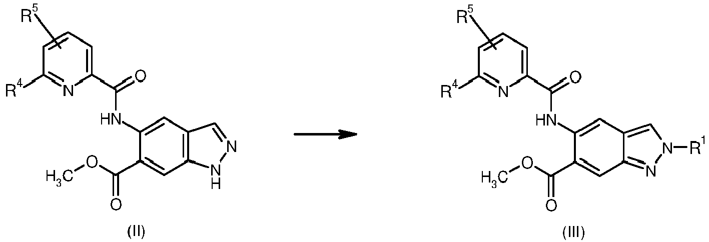 worin
R¹ 4,4,4-Trifluorbutyl, 3-Hydroxy-3-methylbutyl, 3-Methoxypropyl, 3-Hydroxypropyl, 3-Hydroxy-2-methylpropyl, 3-Hydroxy-2,2-dimethylpropyl, 3-Trifluormethoxypropyl, 2-Methoxyethyl, 2-Hydroxyethyl, 2-(Methylsulfonyl)ethyl oder 3-(Methylsulfonyl)propyl ist;
R⁴ für Difluormethyl, Trifluormethyl oder Methyl steht;
R⁵ für Wasserstoff oder Fluor steht;
durch die Reaktion von (II) mit entsprechend substituierten Alkylhalogeniden oder Alkyl-4-methylbenzolsulfonaten in Gegenwart von Kaliumcarbonat.

22. Verbindungen der allgemeinen Formel (III), 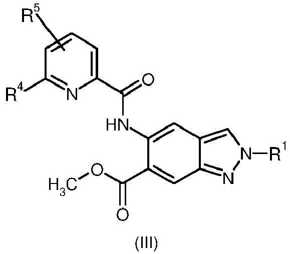 worin
R¹ für 4,4,4-Trifluorbutyl, 3-Hydroxy-3-methylbutyl, 3-Methoxypropyl, 3-Hydroxypropyl, 3-Hydroxybutyl, 3-Hydroxy-2-methylpropyl, 3-Hydroxy-2,2-dimethylpropyl, 3-Trifluormethoxypropyl, 2-Methoxyethyl, 2-Hydroxyethyl, 2-(Methylsulfonyl)ethyl, 3-(Methylsulfonyl)propyl oder 2-(1-Hydroxycyclopropyl)ethyl ist;
R⁴ für Difluormethyl, Trifluormethyl oder Methyl steht; und
R⁵ für Wasserstoff oder Fluor steht,
sowie ihre Diastereomere, Enantiomere, ihre Metabolite, ihre Salze, ihre Solvate oder die Solvate ihrer Salze.

23. Verbindungen der allgemeinen Formel (III), nämlich
Methyl-5-{ [(5-fluor-6-methylpyridin-2-yl)carbonyl]amino}-2-(3-hydroxy-3-methylbutyl)-2H-indazol-6-carboxylat und
Methyl-2-(3-hydroxy-3-methylbutyl)-5-({[6-(trifluormethyl)pyridin-2-yl]carbonyl}amino)-2H-indazol-6-carboxylat.
